(19) Europäisches Patentamt
European Patent Office
Office européen des brevets

(11) Numéro de publication: **0 334 695**
**A1**

(12) **DEMANDE DE BREVET EUROPEEN**

(21) Numéro de dépôt: **89400500.8**

(22) Date de dépôt: **22.02.89**

(51) Int. Cl.[4]: **C 07 D 471/04**
C 07 D 471/14,
C 07 D 401/06,
C 07 D 413/04,
C 07 D 413/14, C 07 F 1/02,
C 07 F 3/02, C 07 F 13/00,
C 07 F 5/00
//(C07D471/04,221:00,209:00),
(C07D471/14,221:00,221:00,
209:00)

(30) Priorité: **23.02.88 FR 8802156**

(43) Date de publication de la demande:
**27.09.89 Bulletin 89/39**

(84) Etats contractants désignés:
**AT BE CH DE ES FR GB GR IT LI LU NL SE**

(71) Demandeur: **SANOFI**
**40, Avenue George V**
**F-75008 Paris (FR)**

(72) Inventeur: **Bouisset, Michel**
**Lotissement Le Thor 73**
**F-04200 Sisteron (FR)**

**Bousquet, André**
**Rue de la Vigne 27**
**F-04200 Sisteron (FR)**

**Dormoy, Jean-Robert**
**Avenue du Thor 9**
**F-04200 Sisteron (FR)**

**Heymes, Alain**
**Rue Frédéric Mistral 5**
**F-04200 Sisteron (FR)**

(74) Mandataire: **Le Guen, Gérard et al**
**CABINET LAVOIX 2, place d'Estienne d'Orves**
**F-75441 Paris Cédex 09 (FR)**

(54) **Procédé de préparation de dérivés d'isoquinoléine.**

(57) La présente invention a pour objet un procédé de préparation de dérivés d'isoquinoléine de formule

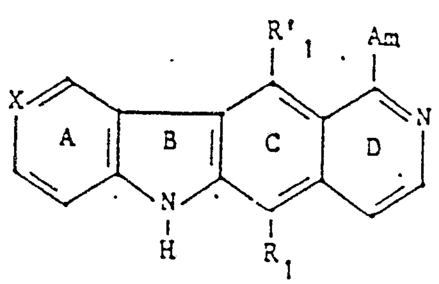

dans laquelle:

- $R_1$ et $R'_1$ représentent chacun l'hydrogène ou un groupement alkyle
- X représente un atome d'azote ou un groupement C-$OR_2$ dans lequel $R_2$ représente alkyle ou benzyle,
- Am représente amino substitué ou non par un goupement de formule:

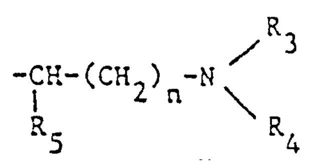

dans laquelle $R_3$ et $R_4$ identiques ou différents, représentent chacun l'hydrogène ou alkyle $R_5$ représente l'hydrogène ou alkyle et n représente une valeur de 1 à 10, en quatre étapes comprenant le passage par des organolithiens ou des composés apparentés.

EP 0 334 695 A1

**Description**

**Procédé de préparation de dérivés d'isoquinoléine**

La présente invention se rapporte, d'une manière générale, à un nouveau procédé de préparation de dérivés d'isoquinoléine.

Plus précisément, l'invention se rapporte à un procédé de préparation de dérivés d'isoquinoléine de formule générale:

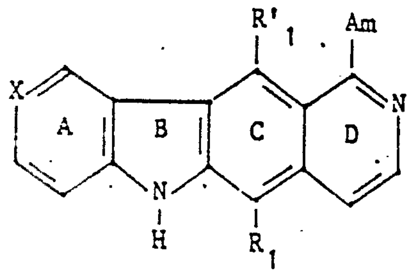

I

dans laquelle:
- $R_1$ et $R'_1$ qui sont identiques ou différents, représentent chacun l'hydrogène ou un groupement alkyle en $C_1$-$C_4$;
- X représente un atome d'azote ou un groupement C-$OR_2$ dans lequel $R_2$ représente un groupement alkyle en $C_1$-$C_4$ ou un groupement benzyle,
- Am représente un groupement amino substitué ou non par un groupement de formule:

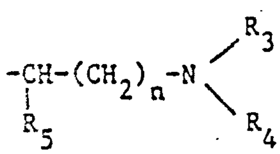

dans laquelle $R_3$ et $R_4$ identiques ou différents, représentent chacun l'hydrogène ou un radical alkyle ayant de 1 à 6 atomes de carbone, $R_5$ représente l'hydrogène ou un radical alkyle ayant de 1 à 6 atomes de carbone et n représente une valeur de 1 à 10.

Dans le présent contexte, l'expression "groupement labile" désigne un groupement protecteur d'amine facilement éliminable en milieu basique tel qu'en particulier un groupement arylsulfonyle, par exemple benzènesulfonyle ou p-toluènesulfonyle, ou un groupement monoalkyl- ou di-alkylaminosulfonyle, par exemple diméthylaminosulfonyle. Les groupements benzènesulfonyle, p-toluènesulfonyle ou diméthylaminosulfonyle constituent des groupements labiles préférés.

Les composés de formule I sont des composés connus ayant été publiés dans le brevet français n° 2.387.229 et ses certificats d'addition n° 2.422.622 et 2.485.537 ainsi que dans le brevet français n° 2.436.786.

Ces composés y sont décrits comme étant des agents antitumoraux et antileucémiques très précieux.

Parmi ces composés de formule I, on peut citer plus particulièrement ceux dans lesquels $R'_1$ représente l'hydrogène ou un groupe méthyle et $R_1$ représente un groupe méthyle.

En outre, parmi l'ensemble de ces composés ceux de formule générale:

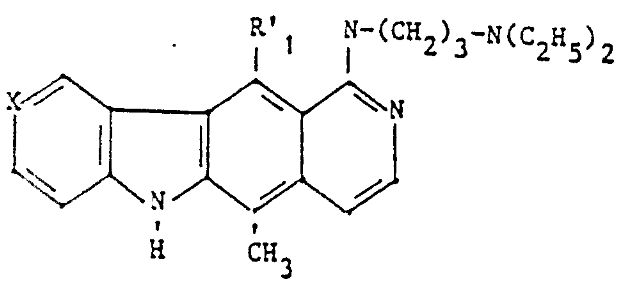

Ia

dans laquelle X représente N et $R'_1$ représente l'hydrogène ou X représente un groupe C-$OCH_3$, et $R'_1$ représente un groupe méthyle, constituent des composés préférés.

La préparation des composés des susdits brevets et certificats d'addition telle qu'elle y est décrite présente de nombreuses difficultés rendant ces procédés antérieurs difficilement exploitables sur le plan industriel.

La recherche d'un procédé permettant une meilleur accessibilité aux composés des brevets et certificats d'addition en question et notamment aux composés de formule Ia reste donc d'un intérêt incontestable.

On a découvert, suivant la présente invention qu'il est possible de préparer les dérivés d'isoquinoléine de formule I, selon un tel procédé industriel.

Ainsi, le procédé de l'invention pour la préparation des dérivés d'isoquinoléine en question implique la succession d'étapes suivantes, lesquelles sont décrites de manière plus détaillée par la suite:

I. Première étape

(a) on transforme un composé de formule

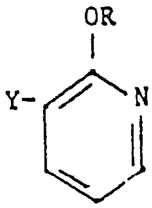

IIIa

dans laquelle:
- R représente un groupement alkyle en $C_1$-$C_4$
- Y représente un groupement diméthyl-4,4 oxazolinyl-2 ou un groupement de formule:

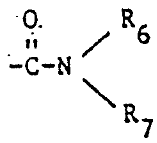

dans lequel $R_6$ et $R_7$ qui sont identiques ou différents, représentent chacun un groupement alkyle en $C_1$-$C_4$, en un dérivé métallique de formule

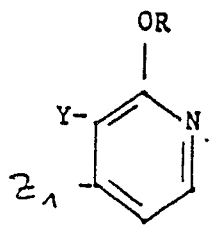

IVa

dans laquelle $Z_1$ représente un atome de lithium ou un radical de formule -Mg Hal, -Mn Hal ou -Ce(Hal)$_2$, Hal représentant un atome d'halogène, puis on condense le dérivé métallique de formule IVa avec un composé hétérocyclique de formule générale:

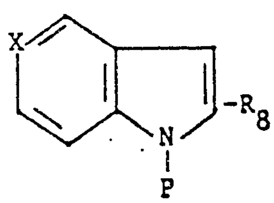

Va

dans laquelle
X a la même signification que précédemment,
P représente un groupe protecteur labile, et
$R_8$ représente:
- un radical de formule
$-\overset{\overset{O}{\|}}{C}-R_1$ dans laquelle $R_1$ a la même signification que précédemment, ou
- un radical de formule

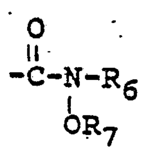

dans laquelle $R_6$ et $R_7$ qui sont identiques ou différents, ont la même signification que précédemment, ou bien

(b) on transforme un composé de formule:

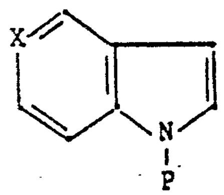

IIIb

dans laquelle X et P ont la même signification que précédemment, en un dérivé métallique de formule

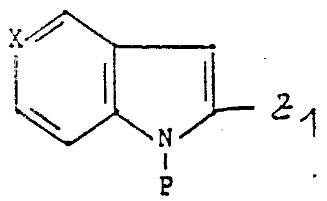

IVb

dans laquelle X, P et $Z_1$ ont la même signification que précédemment, puis on condense le dérivé métallique de formule IVb avec un dérivé d'alkoxy-2 pyridine de formule générale:

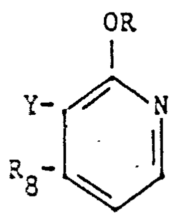

Vb

dans laquelle Y, R et $R_8$ ont la même signification que précédemment de façon à obtenir

- dans le cas où $R_8$ est un radical $-\overset{\overset{O}{\|}}{C}-R_1$, un composé de formule:

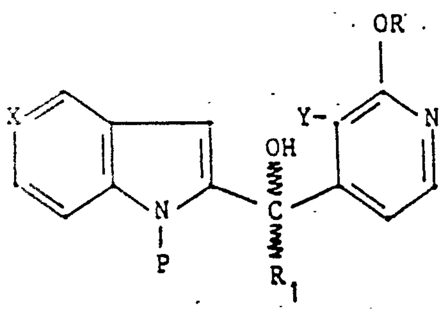

II

dans laquelle X, P, Y, R et $R_1$ ont les mêmes significations que précédemment, et

- dans le cas où $R_8$ est un radical de formule

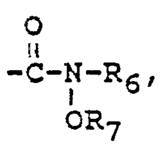

un composé de formule

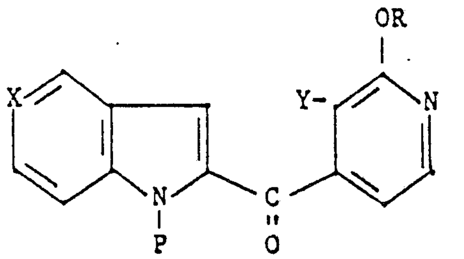

VI

dans laquelle P, R, X et Y ont la même signification que précédemment, cétone que l'on traite par la suite au moyen d'un agent réducteur choisi parmi un borohydrure de métal alcalin, l'hydrure de lithiumaluminium ou un alkyl métal de formule générale:

$R_9M$ VII

dans laquelle $R_9$ représente un groupement alkyle en $C_1$-$C_4$ et M représente un atome de lithium ou un radical -Mg Hal dans lequel Hal représente un atome d'halogène, pour obtenir un complexe que l'on hydrolyse, ce qui fournit un composé de formule II et éventuellement on oxyde un composé de formule II, dans laquelle $R_1$ représente l'hydrogène, en cétone de formule VI, que l'on traite par la suite avec un alkyl métal de formule VII et que l'on hydrolyse, pour obtenir un composé de formule II dans laquelle $R_1$ représente un radical alkyle en $C_1$-$C_4$,

le composé de formule II obtenu dans cette première étape étant par la suite éventuellement déprotégé par traitement au moyen d'un agent basique de façon à obtenir un composé N-déprotégé.

II. Deuxième étape

On cyclise le composé de formule II ou le composé N-déprotégé en une lactone de formule:

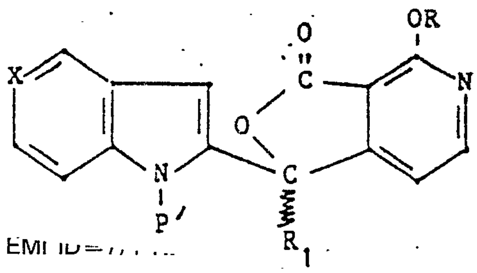

VIII

dans laquelle X, R, $R_1$ ont la même signification que précédemment et P' désigne un groupe protecteur labile P ou un atome d'hydrogène, puis on réduit la lactone ainsi obtenue soit au moyen d'un hydrure métallique pour obtenir un hémiacétal cyclique de formule

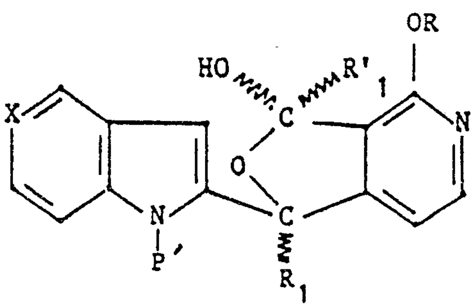

IX

dans laquelle P', R, $R_1$, et X ont la même signification que précédemment, et $R'_1$ représente un atome d'hydrogène, soit au moyen d'un halogénure d'alkylmagnésium de formule VII, avec hydrolyse du complexe formé, pour obtenir un hémiacétal de formule IX dans laquelle $R'_1$ est un radical alkyle en $C_1$-$C_4$ et éventuellement on effectue une déprotection. Ces opérations de cyclisation et de réduction s'effectuent de préférence en traitant le composé de formule II au moyen d'un acide pour obtenir une lactone de formule VIII, lactone que l'on soumet:

- à un traitement au moyen d'un hydrure métallique pour obtenir l'hémiacétal cyclique désiré dans lesquels $R'_1$ représente l'hydrogène,
- à un traitement au moyen d'un halogénure d'alkylmagnésium de formule VII puis à une hydrolyse du complexe formé, pour obtenir l'hémiacétal cyclique désiré dans lesquels $R'_1$ représente un radical alkyle en $C_1$-$C_4$.

III. Troisième étape

On convertit un composé de formule IX en un composé de formule:

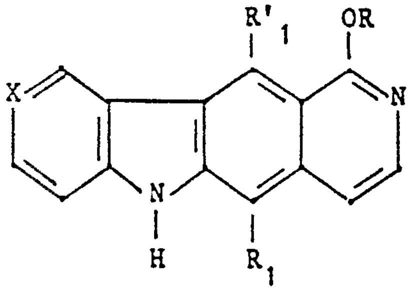

X

dans laquelle R, $R_1$, $R'_1$ et X ont la même signification que précédemment.

Cette opération s'effectue de préférence en traitant l'hémiacétal en question avec au moins 10 équivalents d'un hydroxyde de métal alcalin, généralement de 10 à 15 équivalents, dans un alcoolen $C_1$-$C_3$ au reflux par exemple le méthanol, l'éthanol ou l'isopropanol.

IV. Quatrième étape

On traite un composé de formule X

(a) ou bien avec un composé de formule générale:

H-Am XI

dans laquelle Am a la même signification que précédemment et en présence d'un catalyseur acide, pour obtenir un composé désiré de formule I sous forme de base libre,

(b) ou bien avec de l'oxyde de dichlorophénylphosphine pour obtenir un composé chloro de formule générale:

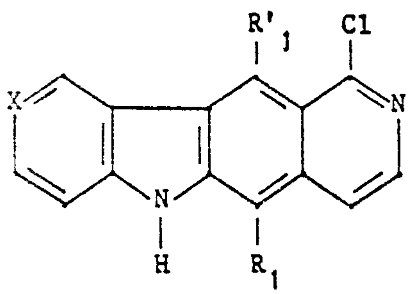

XII

dans laquelle $R_1$, $R'_1$ et X ont la même signification que précédemment, que l'on fait réagir avec un composé de formule XI pour former le composé désiré de formule I sous forme de base libre,

(c) ou bien avec le trichlorure de phosphore en présence d'un co-solvant polaire, d'un chlorure d'ammonium et d'une aniline, pour former un composé chloro de formule XII, que l'on fait réagir avec un composé de formule XI pour obtenir un composé désiré de formule I sous forme de base libre,

(d) ou bien avec un halogénure de triméthylsilyle et en présence d'un halogénure de métal alcalin, pour obtenir un dérivé triméthylsilyloxy que l'on fait réagir avec un composé de formule XI et en présence d'hexaméthyldisilazane et d'acide p-toluènesulfonique, pour former un composé désiré de formule I sous forme de base libre, base libre que l'on peut traiter, si nécessaire, avec un acide organique ou inorganique approprié pour obtenir un sel pharmaceutiquement acceptable.

Les étapes I à IV dont il est question ci-dessus, seront décrites et commentées plus en détail par la suite:

I. Première étape

De manière à disposer d'un procédé permettant une meilleure accessibilité aux composés de formule I et notamment aux composés de formule Ia, on a cherché à préparer ces composés selon un nouveau procédé faisant intervenir pour l'élaboration de l'enchaînement ABCD, la création du cycle C à partir d'un part de l'entité AB porteuse de groupements fonctionnels ou protecteurs adéquats et d'autre part du cycle D également doté de substituants fonctionnels appropriés.

Les composés des susdits brevets et certificats d'addition, en particulier les composés de formule Ia, sont notamment caractérisés par la présence d'une chaîne dialkylaminoalkylamino en $\alpha$ de l'azote isoquinoléinique.

En vue de réaliser la synthèse la plus directe possible, on a cherché à utiliser des composés pyridiniques N-protégés porteurs de la chaîne dialkylaminoalkylamino, en particulier des composés pyridiniques de formule générale:

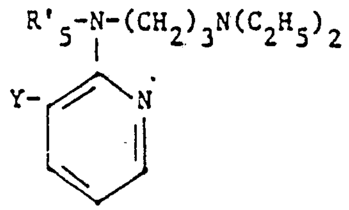

XIII

dans laquelle Y a la même signification que précédemment, et R'5 représente l'hydrogène ou un groupement protecteur, plus particulièrement un groupement benzyle.

Cependant, certains de ces composés de formule XIII se sont avérés particulièrement difficiles à préparer et à purifier, en particulier les composés dans lesquels R'5 est différent d'hydrogène. Ces difficultés ont été enregistrées aussi bien lors de la réaction d'un composé chloropyridinique de formule:

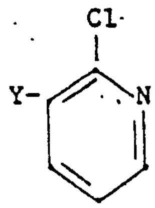

XIV

dans laquelle Y a la même signification que précédemment, avec une amine secondaire telle que la N-benzyl N-diéthylaminopropylamine, que lors de la réaction de ce composé de formule XIV avec une amine primaire telle que la benzylamine suivie de l'alkylation éventuelle avec un halogénure de diéthylaminopropyle ou inversément.

Ensuite, on a entrepris des essais en vue de fixer un atome de lithium en position 4 des dérivés amino-2 pyridine N-monosubstitués ou N,N-disubstitués ainsi préparés en vue de substituer ultérieurement cet atome métallique par un groupement fonctionnel.

A cet effet, on a utilisé soit la méthode décrite dans J. Org. Chem. 47, pp. 2633-2637 (1982) selon laquelle une lithiation est effectuée dans le tétrahydrofuranne à 0°C et en utilisant le tétraméthylpipéridure de lithium comme agent de lithiation, soit une méthode analogue conduite à -78°C moyennant la présence supplémentaire de tétraméthyléthylènediamine comme agent de stabilisation du dérivé lithien.

Cependant, la lithiation en question suivant l'une ou l'autre de ces méthodes et en conséquence la fixation d'un groupement fonctionnel au niveau de tels composés de formule XIII s'est avéré impossible dans la plupart des cas notamment dans le cas des [(N-diéthylamino-3 propyl N-benzyl)-amino]-2 diéthylaminocarbonyl-3 pyridine, N-benzylamino-2 diisopropylaminocarbonyl-3 pyridine et [(N-diéthylamino-3 propyl)-amino]-2 diisopropylaminocarbonyl-3 pyridine.

L'échec de la métallation enregistrée en position 4 des dérivés monosubstitués ou N,N-disubstitués amino-2 pyridine précédents peut être imputable à une désactivation de cette position par chélation parasite de l'agent métallant.

Si la réaction entre le composé chloropyridinique de formule XIV dans laquelle Y représente un groupe diméthyl-4,4 oxazolinyl-2 et une amine primaire telle que la benzylamine ou la diéthylaminopropylamine ou secondaire telle que la N-benzyl N-diéthylaminopropylamine s'est avérée beaucoup plus facile que dans le cas où Y représente un groupement carboxamide, par contre la lithiation en position 4 des composés obtenus à savoir la benzylamino-2(diméthyl-4,4 oxazolinyl-2)-3 pyridine, la [(N-diéthylamino-3 propyl)amino]-2 (diméthyl-4,4 oxazolinyl-2)-3 pyridine ou la [(N-diéthylamino-3 propyl-N-benzyl)amino]-2 (diméthyl-4,4 oxazolinyl-2)-3 pyridine n'a pu être réalisée de façon satisfaisante.

Des observations analogues de résistance à la métallation au niveau de dérivés (diméthyl-4,4 oxazolinyl-2) pyridine ont d'ailleurs été signalées dans Tetrahedron 39, 12 pp. 1991-1999 (1983).

De même, il n'a pas été non plus possible de fixer un atome de lithium en position 4 des chloro-2 diisopropylamino-3 pyridine et chloro-2 (diméthyl-4,4 oxazolinyl-2)-3 pyridine.

En conséquence, la lithiation en position 4 de pyridines substituées en position 3 par un groupement diméthyl-4,4 oxazolinyl-2 ou -CO $NR_6R_7$ tel que décrit précédemment, en vue de la fixation ultérieure d'un substituant fonctionnel, apparaît particulièrement difficile lorsque la position 2 est déjà elle-même substituée par un groupement amine secondaire ou tertiaire.

On a maintenant trouvé, de manière surprenante, que la lithiation en position 4 de pyridine comportant en position 3 un substituant tel que décrit précédemment peut être réalisée de façon satisfaisante à partir de composés pyridiniques dotés d'un groupement alkoxy en position 2, c'est-à-dire à partir de composés de formule IIIa ci-dessus.

Ce groupement alkoxy en position 2 présente en outre l'avantage de pouvoir donner naissance au groupement final souhaité des composés de formule I c'est-à-dire au groupement dialkylaminoalkylamino après aminolyse effectuée de manière directe ou après passage transitoire par un dérivé chloro.

Toutefois, lorsque Y représente un groupement diméthyl-4,4 oxazolinyl-2, R représente préférentiellement un groupement alkyle ramifié tel qu'isopropyle ou tertiobutyle.

Ainsi, on peut réaliser la première étape du procédé de l'invention :

A- Pour préparer un composé protégé de formule II

(1) en faisant réagir dans un solvant un dérivé d'alkoxy-2 pyridine de formule IIIa avec un agent de lithiation lequel est soit un alkyl lithium ramifié soit un amidure de lithium à une température comprise entre -80° C et -20° C et en présence d'un agent de stabilisation tel que la tétraméthyléthylènediamine ou une tris(dioxa-alkyl)amine, telle que par exemple la tris(dioxa-3,6 heptyl) amine pour obtenir les dérivés lithio-4 de formule générale:

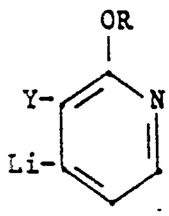

XV

dans laquelle R et Y ont la même signification que précédemment,

(2) puis en condensant le dérivé métallique obtenu de formule XV, dans un solvant et à une température comprise entre -80° C et -20° C avec un composé de formule $V_a$ dans laquelle $R_8$ représente:

- un radical de formule

- $\overset{O}{\overset{\|}{C}}-R_1$ tel que défini précédemment, pour obtenir les composés N-protégés de formule II, ou

- un radical de formule

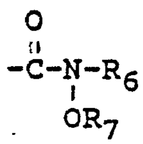

tel que défini précédemment,

pour obtenir les cétones N-protégées de formule VI, cétones que l'on traite par la suite, dans un solvant approprié tel qu'un éther par exemple le tétrahydrofuranne et à une température inférieure ou égale à la température ambiante, avec un agent réducteur approprié tel que le borohydrure de sodium, l'hydrure de lithium-aluminium ou un alkyl métal de formule VII, pour obtenir un complexe que l'on hydrolyse de manière ménagée, par exemple en présence de chlorure d'ammonium, pour obtenir les composés désirés.

Généralement, le solvant utilisé dans les séquences (1) et (2) peut être un éther, par exemple le tétrahydrofuranne, un mélange éther/hydrocarbure par exemple tétrahydrofuranne/cyclohexane ou pentane, un mélange éther/tétraméthyléthylènediamine par exemple tétrahydrofuranne/tétraméthyléthylènediamine ou encore la tétraméthyléthylènediamine seule.

L'alkyl lithium, qui est avantageusement ramifié, est de préférence le tertiobutyllithium. En effet, il apparaît désavantageux d'utiliser directement des alkyl lithium non ramifiés en raison de possibilités de réaction d'addition 1-2 ou 1-4 sur la pyridine.

Quant à l'amidure de lithium, il s'agit généralement du tétraméthyl-2,2,6,6 pipéridure de lithium ou d'un dialkylamidure de lithium tel que par exemple le diéthylamidure de lithium ou le diisopropylamidure de lithium.

Le tétraméthyl-2,2,6,6 pipéridure de lithium étant donné ses fortes propriétés basiques constitue l'amidure de lithium préféré selon l'invention.

Par équivalent de composé alkoxy-2 pyridinique de formule IIIa on utilise de 1 à 2 équivalents d'agent de lithiation. On a remarqué que la métallation n'est optimale, c'est-à-dire supérieure ou égale à 80%, qu'en utilisant un excès d'agent de lithiation par exemple 1,7 équivalent de tétraméthyl-2,2,6,6 pipéridure de lithium ou 1,5 équivalent de tertiobutyllithium (taux de métallation: ≧ 85%)

Ceci implique l'intervention d'un équilibre déplacé vers la formation du dérivé lithio-4 par l'utilisation d'un tel excès d'agent de lithiation. Cette hypothèse est renforcée par l'obtention à stoéchiométrie égale, d'un taux de métallation supérieur avec le tétraméthylpipéridure-2,2,6,6 de lithium par rapport au diisopropylamidure de lithium, moins basique.

La tétraméthyléthylènediamine ou la tris(dioxaalkyl) amine a essentiellement pour but d'augmenter la basicité de l'agent métallant et de stabiliser par ligandage les dérivés lithio-4 de formule XV.

En effet, on a remarqué que l'absence d'un tel agent de stabilisation ou encore l'utilisation de températures plus élevées que celles envisagées dans le procédé de l'invention entraînent une chute des rendements. Généralement, on utilise de 1 à 5 équivalents d'agent de stabilisation de préférence 1 à 2 équivalents par équivalent de composé de formule IIIa.

Ainsi, la métallation des composés de formule IIIa pour l'obtention des dérivés lithiens de formule XV peut

s'effectuer selon différentes mises en oeuvre des réactifs, à savoir:
- préparation extemporanée de l'amidure de lithium par réaction de n-butyllithium avec l'amine correspondante par exemple la diéthylamine, la diisopropylamine ou la tétraméthyl-2,2,6,6 pipéridine et addition de cet amidure lithié à une solution du composé de formule IIIa et d'agent de stabilisation;
- préparation extemporanée de l'amidure de lithium par réaction de n-butyllithium avec l'amine correspondante en présence d'agent de stabilisation et addition de cette solution d'amidure lithié/agent de stabilisation à une solution du composé IIIa;
- addition de tertiobutyllithium, par exemple 1,5 équivalent, à une solution d'agent de stabilisation et de composé de formule IIIa par exemple 1 équivalent de composé de formule IIIa.

Cette dernière méthode présente l'avantage d'utiliser un seul réactif commercial, le tertiobutyllithium. On évite en conséquence la préparation préalable de l'agent de lithiation, contrairement à la mise en oeuvre précédente, ce qui permet de n'utiliser qu'un seul réacteur pour la métallation:
- addition lente de n-butyllithium à un mélange d'amine, d'agent de stabilisation et composé de formule IIIa telle que par exemple 1,5 équivalent de n-butyllithium à un mélange de 0,5 équivalent d'amine, 1,5 équivalent d'agent de stabilisation et 1 équivalent de composé de formule IIIa de manière à former in situ l'amidure de lithium. On obtient ainsi, lorsque l'agent de stabilisation est la tétraméthyléthylènediamine (TMEDA), le schéma réactionnel suivant:

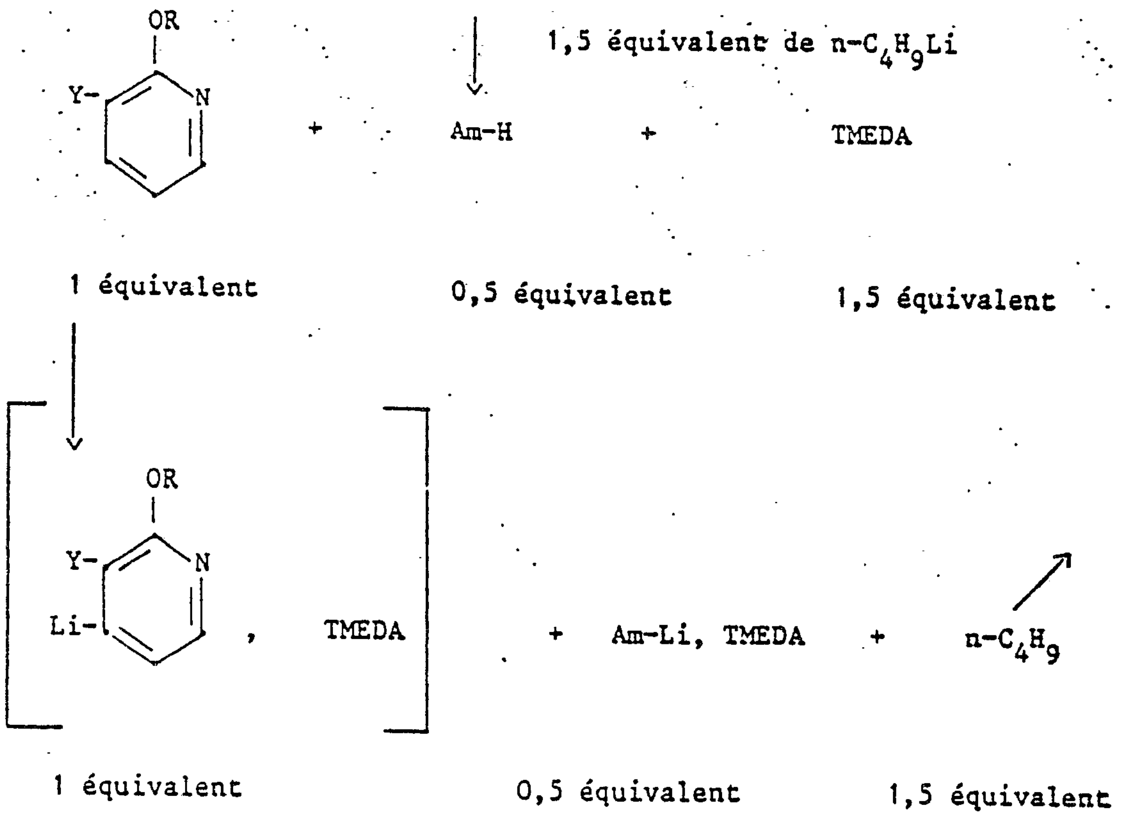


Am = diéthylamino, diisopropylamino, tétraméthylpipéridino..

Cette méthode présente l'avantage de n'utiliser qu'un seul réacteur réfrigéré et un seul milieu réactionnel.

Elle permet également de n'employer qu'une quantité minimum d'amine par rapport à la quantité de dérivé de formule IIIa, ce qui est particulièrement avantageux lorsque l'amine en question est difficilement accessible sur le plan industriel comme la tétraméthyl-2,2,6,6 pipéridine.

En outre, cette méthode provoque le déplacement de la réaction de métallation vers la formation du composé lithié par suite du dégagement de n-butane et de l'absence de toute amine libre dans le milieu en fin d'addition de n-butyllithium.

Les dérivés lithiés de formule XV ainsi produits peuvent être isolés par élimination du solvant utilisé lors de leur production par exemple par évaporation sous pression réduite. Il convient de respecter, pour cette opération, une température inférieure à -60°C de manière à éviter les risques de dégradation. Les dérivés lithiés en question doivent en outre être conservés à basse température, sinon ils se polymérisent, et dans un environnement anhydre étant donné leur facilité de décomposition par l'eau.

Pour cette raison, les dérivés lithiés de formule XV seront utilisés préférentiellement sans être isolés mais au contraire en les mettant en oeuvre directement dans le milieu où ils sont produits avec un composé de formule Va.

On a pu effectuer aisément l'estimation du taux de métallation en position 4 des composés de formule IIIa, en particulier de la diisopropylaminocarbonyl-3 méthoxy-2 pyridine en utilisant le chlorure de triméthylsilyle

comme réactif à caractère électrophile, ce réactif étant inerte vis-à-vis du tétraméthyl-2,2,6,6 pipéridure de lithium à -70°C.

On a utilisé à cet effet la technique suivante:

On refroidit à -70°C une solution de 0,1 mole de diisopropylamino carbonyl-3 méthoxy-2 pyridine, de 0,17 mole de tétraméthyléthylènediamine et de 0,35 mole de chlorotriméthylsilane dans 20 ml de tétrahydrofuranne. On ajoute alors à la solution en quelques minutes en maintenant la température du milieu inférieure ou égale à -70°C, 0,17 mole de tétraméthyl-2,2,6,6 pipéridure de lithium obtenu par réaction de 0,17 mole de n-butyllithium et 0,17 mole de tétraméthyl-2,2,6,6 pipéridine dans 120 ml de tétrahydrofuranne. On verse le milieu réactionnel dans une solution aqueuse d'acide chlorhydrique (57 ml d'acide 37% - 150 ml d'eau) et on extrait au toluène. Après séchage sur sulfate de sodium et évaporation du solvant, on obtient la diisopropylaminocarbonyl-3 méthoxy-2 triméthylsilyl-4 pyridine, sous forme d'une huile, avec un rendement de 79%.

Spectre RMN ($CDCl_3$):

0,3 ppm (s,9H); 1,05-1,7 ppm (m,12H); 3,2-3,8 ppm (m,2H); 3,9 ppm (s,3H); 7,0 ppm (d,1H); 8,1 ppm (d,1H).

Cette méthode dans laquelle le réactif à caractère électrophile est introduit avec l'agent métallant permet une bonne estimation du taux de métallation. Le dérivé lithio-4 est piégé dès sa formation et les réactions de dégradation de ce lithien ou les réactions parasites éventuelles sont minimisées.

On a pu également estimer le taux de métallation par réaction de la diisopropylaminocarbonyl-3 lithio-4 méthoxy-2 pyridine avec $D_2O$ ou $CH_3OD$ (deutériation) selon le schéma suivant en utilisant la méthode de préparation décrite précédemment:

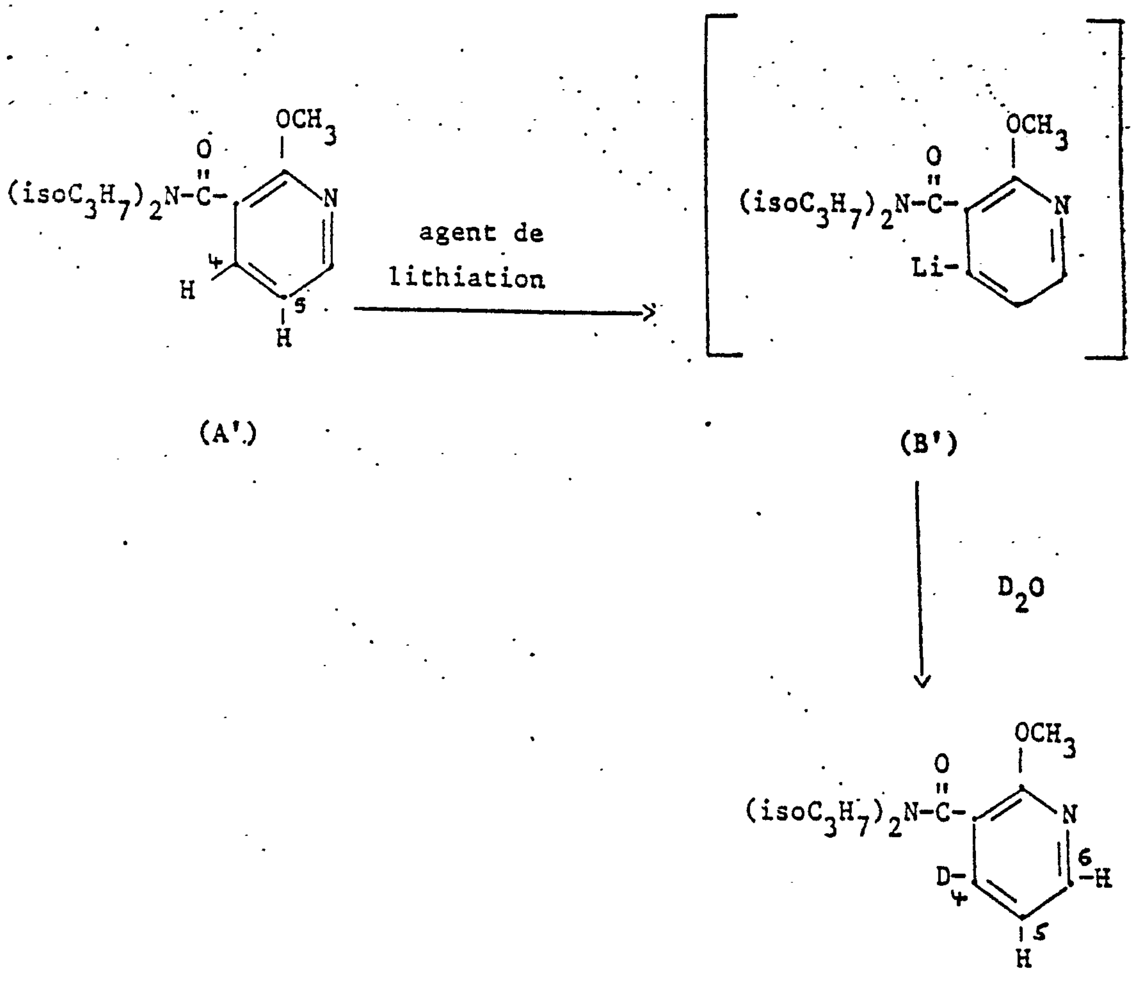


(C')

L'examen du spectre de RMN de $^1H$ des composés obtenus à l'état brut montre la disparition du doublet attribuable au proton $H_4$ et la simplification du proton $H_5$ qui devient simple doublet au lieu de double doublet. Il ne subsiste plus que le couplage $H_5H_6$.

L'intégration des signaux adéquats permet d'estimer avec une bonne précision les quantités relatives des deux espèces et donc le taux de métallation correspondant à

Composé (C')

Composé (A') + Composé (C')

Rendement en dérivé (C'): 95%
Taux de métallation: > 86% déterminé par RMN de $^1H$
Spectre RMN ($CDCl_3$):
0,9-1,7 ppm (m,12H); 3,2-3,8 ppm (2m,2H); 3,95 ppm (s,3H); 6,9 ppm (d,1H); 8,15 ppm (d,1H).

On a également estimé le taux de lithiation en position 4 de la diéthylaminocarbonyl-3 méthoxy-2 pyridine par réaction du dérivé lithié en question avec $D_2O$ selon la méthode décrite précédemment.

On a ainsi obtenu la diéthylaminocarbonyl-3 deutério-4 méthoxy-2 pyridine avec un rendement de 87%.
P.F.: 82°C
Spectre RMN ($CDCl_3$):
1,05 et 1,25 ppm (2t,6H); 3,15 et 3,55 ppm (2s,4H); 3,95 ppm (s,3H); 6,9 ppm (d,1H); 8,15 ppm (d,1H).

Au lieu de dérivés lithiés de formule XV on peut également utiliser, dans le procédé décrit précédemment, d'autres dérivés métalliques tels que des dérivés de formule XV dans lesquels l'atome de lithium a été remplacé par un radical -Mg Hal, -Mn Hal ou -Ce(Hal)$_2$, Hal représentant un atome d'halogène tel que chlore, brome ou iode.

Ces dérivés magnésiens, manganeux ou céreux peuvent être préparés par transmétallation ou réaction d'échange entre des dérivés lithio-4 de formule XV et un halogénure de magnésium tel que le bromure de magnésium, un halogénure de manganèse tel que le chlorure de manganèse ou un trihalogénure de cérium tel que le trichlorure de cérium et ce, à une température comprise entre -70°C et -20°C.

Toutefois, dans cette réaction de transmétallation, on doit parfois éviter la présence d'agent de stabilisation qui perturbe la réaction en question.

Quant à la condensation des composés de formule Va en position 4 des composés métalliques de formule XV, celle-ci peut se réaliser également selon différentes méthodes telles que:
- addition simultanée d'amidure de lithium préparé extemporanément et de composé de formule IVa à une solution du composé de formule IIIa et d'agent de stabilisation,
- addition simultanée d'amidure de lithium, préparé extemporanément en présence d'agent de stabilisation et de composé de formule Va à une solution du composé de formule IIIa,
- métallation du composé de formule IIIa telle qu'explicitée précédemment pour former le dérivé lithié de formule XV puis condensation avec le composé de formule Va.

Cette dernière méthode, selon une procédure classique en deux temps, est parfois préférable, surtout s'il y a lieu de craindre une interaction parasite de l'agent de lithiation et du composé de formule Va.

Lorsque, dans la formule IIIa, Y représente un radical diméthyl-4,4 oxazolinyl-2, R représente, de préférence un groupement alkyle ramifié par exemple isopropyle ou tertiobutyle.

En effet, la facilité de substitution d'un groupement peu encombré comme le groupement méthoxy en $\alpha$ d'une oxazoline peut engendrer des réactions secondaires tant lors de l'utilisation de lithiens carbonés que d'amidures lithiens.

Seuls les groupements alkoxy ramifiés permettent d'obtenir dans ce cas un taux de métallation important ainsi qu'un rendement élevé en dérivé substitué par un groupement électrophile en position 4.

A cet effet, on opère préférentiellement à des températures de l'ordre de -70°C en utilisant par exemple la tétraméthyléthylènediamine ou la tris (dioxa-3,6 heptyl) amine comme agent de stabilisation et la tétraméthyléthylènediamine ou un mélange tétrahydrofuranne/tétraméthylènediamine ou tris (dioxa-3,6 heptyl) amine comme solvant.

En outre, lorsque dans la formule IIIa, Y représente un groupement de formule -CONR$_6$R$_7$, les groupements diéthylaminocarbonyle et diisopropylaminocarbonyle constituent des groupements préférés.

Le procédé ainsi décrit permet la préparation des composés de formule II avec des rendements variables selon les composés de départ mis en oeuvre. Généralement, ces rendements se situent aux environs de 20 à 40% lorsque le groupement protecteur labile P du composé de départ de formule Va est un groupe-ment arylsulfonyle, par exemple benzènesulfonyle.

Ces rendements peuvent être considérablement améliorés pour avoisiner, dans certains cas, 70% lorsqu'on utilise, un dérivé de formule Va dans laquelle le groupement protecteur labile P est un groupement monoamino- ou dialkylaminosulfonyle tel que diméthylaminosulfonyle.

Les composés de formule Va sont soit des produits connus ayant été publiés dans FR-A-2 574 406, soit des composés pouvant être préparés par le procédé décrit dans ce document.

Par exemple, les composés de formule Va dans laquelle R$_8$ représente un radical de formule

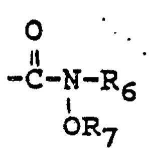

peuvent être préparés à partir d'un dérivé lithio-2 d'aza-5 indole N-protégé ou d'un dérivé lithio-2 d'alkoxy-5

indole N-protégé et d'un halogénure de formule

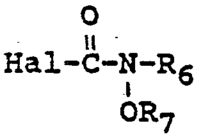

dans laquelle Hal, $R_6$ et $R_7$ ont la même signification que précédemment.

De même, les composés de départ pour l'obtention des composés de formule Va, c'est-à-dire des composés pouvant être représentés par la formule Va dans laquelle $R_8$ est l'hydrogène, peuvent être préparés à partir d'aza-5 indole ou d'un alkoxy-5 indole en faisant réagir, d'abord un hydroxyde de métal alcalin en présence d'un catalyseur de transfert de phase ensuite un halogénure de formule P-Hal dans laquelle P a la même signification que précédemment et Hal représente un atome d'halogène.

Selon la variante (b) du procédé, on peut également obtenir les composés de formule II :

(1′) en faisant réagir, dans un solvant, un dérivé hétérocyclique N-protégé de formule IIIb avec un agent de lithiation lequel est soit un alkyl lithium ramifié, soit un amidure de lithium, à une température comprise entre -80°C et -20°C et en présence d'un agent de stabilisation, pour obtenir les dérivés lithio-2 de formule générale:

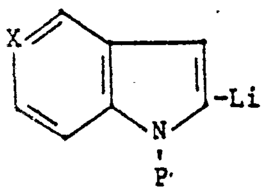

XVI

dans laquelle X et P ont la même signification que précédemment;

(2′) puis en condensant le dérivé métallique obtenu de formule XVI, dans un solvant et à une température comprise entre -80°C et -20°C, avec un dérivé de pyridine de formule IVb dans laquelle $R_8$ représente:

- un radical de formule

- 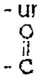 -$R_1$ tel que défini précédemment, pour obtenir les composés N-protégés de formule II,

- un radical de formule

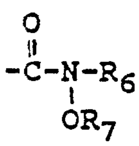

tel que défini précédemment, pour obtenir les cétones N-protégées de formule VII que l'on traite, par la suite, comme décrit précédemment.

B - Pour réaliser la déprotection du composé de formule II

on traite dans un solvant ce composé au moyen d'un agent basique tel qu'un carbonate de métal alcalin, par exemple le carbonate de potassium?

C- Pour préparer un composé protégé de formule II dans laquelle R représente un groupement alkyle,

on peut traiter au reflux et dans un solvant tel que le dichlorométhane, un composé de formule II dans laquelle $R_1$ est l'hydrogène, c'est-à-dire un composé de formule:

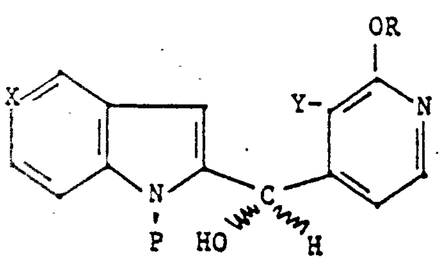

XVII

dans laquelle P, X, R et Y ont la même signification que précédemment, au moyen d'un agent oxydant tel que le bioxyde de manganèse pour obtenir une cétone de formule VI, cétone que l'on traite avec un alkyl métal de formule VII, puis que l'on hydrolyse de manière ménagée, par exemple en présence de chlorure d'ammonium, pour obtenir le composé désiré.

Les dérivés de pyridine de formules II, IIIa, IVa, $V_b$, VI, et XVII sont des composés nouveaux et constituent eux-mêmes des composés intermédiaires particulièrement intéressants pour la préparation notamment des dérivés d'alkoxy-isoquinoléine de formule I.

En conséquence, un autre objet de l'invention se rapporte aux dérivés d'alkoxy-2 pyridine de formule générale:

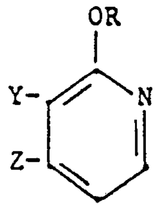

XVIII

dans laquelle R et Y ont la même signification que précédemment et Z représente:
- un atome d'hydrogène,
- un atome de lithium ou un groupement -Mg Hal, -Mn Hal ou $-Ce(Hal)_2$ dans lequel Hal représente un atome d'halogène,
- un groupement de formule:

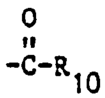

ou

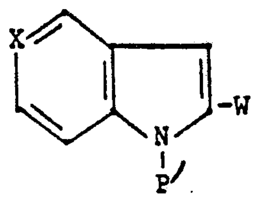

$XIX_a$ $XIX_b$

dans laquelle:
- $R_{10}$ représente l'hydrogène, un groupement alkyle en $C_1$-$C_4$, un groupement hydroxyle ou un groupement de formule

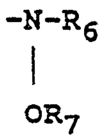

dans laquelle $R_6$ et $R_7$ ont la même signification que précédemment,
- X a la même signification que précédemment,
- P′ représente un groupe protecteur labile P ou un atome d'hydrogène
- W représente un groupement de formule

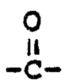

ou

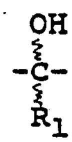

dans lequel $R_1$ a la même signification que précédemment.

L'invention se rapporte également aux deux isomères des composés de formule XVIII dans laquelle W représente un groupement

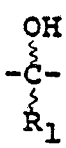

ainsi qu'aux mélanges de ces isoméres

Les composés de formule XVIII dans laquelle Z représente l'hydrogène ou un groupement de formule XIXa peuvent être préparés comme suit:

(1) Lorsque Z représente l'hydrogène en faisant réagir au reflux un dérivé chloro-2 pyridinique de

formule générale:

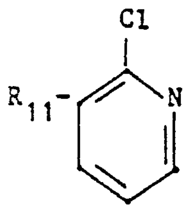

XX

dans laquelle $R_{11}$ représente un groupement $-CONR_6R_7$ tel que défini précédemment, le radical

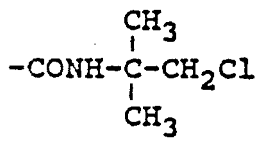

ou le radical diméthyl-4,4 oxazolinyl-2, dans un milieu alcoolique ayant de 1 à 4 atomes de carbone par exemple le méthanol et en présence d'un excès de l'alcoolate de métal alcalin correspondant tel que le méthylate de sodium, ce qui fournit le composé désiré de formule XVIII dans laquelle Z représente l'hydrogène.

En conséquence, les composés de formule XVIII dans laquelle Y représente le radical diméthyl-4,4 oxazolinyl-2 peuvent être obtenus selon deux voies:

- une voie directe consistant à effectuer en milieu alcool/alcoolate de métal alcalin, la cyclisation du radical

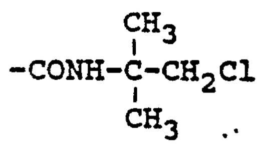

fixé en position 3 et à substituer simultanément l'atome de chlore en position 2 par un groupement alkoxy de préférence un groupement alkoxy ramifié,

- ou une voie indirecte consistant à traiter au reflux et si nécessaire dans un solvant approprié tel qu'un hydrocarbure aromatique par exemple le toluène, le composé de formule XX ci-dessus dans laquelle $R_{11}$ représente le radical

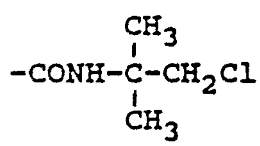

avec une base non nucléophile telle qu'un carbonate de métal alcalin ou l'hexaméthyldisilazane pour obtenir la chloro-2 (diméthyl-4,4 oxazolinyl-2)-3 pyridine dont la réaction avec un mélange alcool/alcoolate de métal alcalin fournit le dérivé désiré de préférence le dérivé dans lequel le groupement alkoxy-2 est un groupement alkoxy ramifié.

Les dérivés de pyridine de formule XX eux-mêmes, peuvent être obtenus:

- lorsque $R_{11}$ représente un groupement $-CONR_6R_7$ tel que décrit précédemment, par réaction au reflux entre l'acide chloro-2 nicotinique et le chlorure de thionyle pour former un chlorure de cet acide que l'on fait alors réagir, dans un solvant approprié par exemple le dichlorométhane, avec une amine de formule générale:

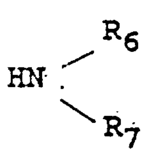

XXI

dans laquelle $R_6$ et $R_7$ ont la même signification que précédemment et ce, à une température inférieure à 30° C, ce qui fournit le composé désiré,

- lorsque $R_{11}$ représente le groupement

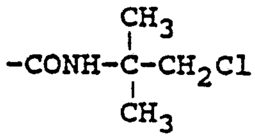

en condensant le chlorure de l'acide chloro-2 nicotinique avec l'amino-2 méthyl-2 propanol à une température inférieure ou égale à 0°C dans un milieu approprié par exemple un mélange dichlorométhane/tétrahydrofuranne pour former la chloro-2[(diméthyl-1,1 hydroxy-2 éthyl)amino-carbonyl]-3 pyridine que l'on fait ensuite réagir avec le chlorure de thionyle à une température inférieure à la température ambiante, ce qui fournit le composé désiré.

(2) Lorsque Z représente un groupement de formule XIXa en utilisant un procédé analogue à celui décrit précédemment pour la préparation des composés de formule II à partir de composés de formules XV et Va. Toutefois, dans le cas présent, le composé à condenser sera choisi parmi le groupe formé par:

- un ester de formule générale:

$R_1-\overset{\overset{O}{\|}}{C}-OR_6$

dans laquelle $R_1$ et $R_6$ ont la même signification que précédemment,

- un anhydride de formule générale:

$(R_1-\overset{\overset{O}{\|}}{C})_2O$

dans laquelle $R_1$ a la même signification que précédemment,

- un amide de formule générale:

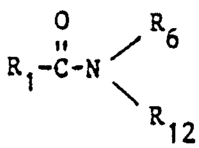

dans laquelle $R_1$ et $R_6$ ont la même signification que précédemment et $R_{12}$ représente un radical alkyle en $C_1$-$C_4$ ou alkoxy en $C_1$-$C_4$,

- un halogénure de formule générale:

$Hal-\overset{\overset{O}{\|}}{C}-R_1$

dans laquelle Hal représente un atome d'halogène et $R_1$ a la même signification que précédemment,

- un nitrile de formule générale:

$R_1$-CN

dans laquelle $R_1$ a la même signification que précédemment,

- l'anhydride carbonique,

- un halogénure de formule générale:

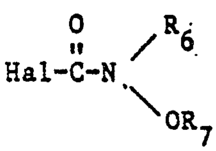

dans laquelle $R_6$ et $R_7$ ont la même signification que précédemment et Hal représente un atome d'halogène, par exemple chlore, pour obtenir le composé désiré de formule XVIII dans laquelle Z représente un groupement de formule XIXa.

Les conditions opératoires utilisées dans les séquences (1) et (2) du procédé ci-dessus, à savoir solvants, agents de stabilisation de même que les différentes mises en oeuvre de ces composés sont identiques à celles décrites dans le procédé de l'invention nécessitant l'utilisation des composés de formules IIIa, XV et Va précédentes.

Suivant une variante, on peut obtenir les composés de formule XVIII dans laquelle Z représente un groupement de formule

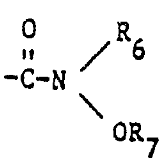

en faisant réagir, à température ambiante, un dérivé pyridyl-carboxylique de formule générale:

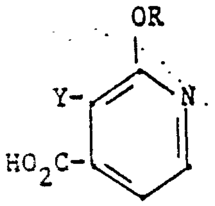

XXII

dans laquelle R et Y ont la même valeur que précédemment, avec un dérivé d'hydroxylamine de formule générale:

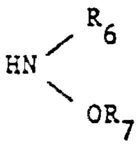

XXIII

dans laquelle $R_6$ et $R_7$ ont la même signification que précédemment, en présence d'un réactif d'amidification tel que le benzotriazolyl-1 tris-diméthylaminophosphonium pour obtenir le composé désiré de formule XVIII.

Lorsque le dérivé de formule XXIII est sous forme de sel par exemple le chlorhydrate, il est préférable d'effectuer la réaction en présence d'un accepteur d'acide par exemple la triéthylamine.

Selon l'invention, les composés de formule II présentent notamment les avantages suivants:

- le substituant OR peut être transformé en groupement Am présent dans les composés de formule I,
- le substituant Y couplé avec le substituant OR permet la fixation d'un atome de lithium en position 4,
- le substituant Y est soit un carboxamide suffisamment encombré au niveau du groupement carbonyle pour éviter la condensation du lithien en position 4 sur lui-même dans les conditions de métallation, soit un groupement diméthyl-4,4 oxazolinyl-2 stable dans de tels milieux basiques,
- le substituant Y peut être réduit du degré d'oxydation 3 en substituants de degré 2 seuls aptes à conduire à la fermeture du cycle par passage du composé VIII aux composés X ci-dessus.

II. Deuxième étape

On a trouvé qu'il est possible, à partir de dérivés de formule II, d'obtenir un composé de formule X de la troisième étape en passant par un composé lactonique de formule VIII puis un dérivé hémiacétal cyclique de formule IX.

Les dérivés hémiacétals cycliques de formule IX peuvent être préparés à partir des lactones en question selon des rendements particulièrement importants de l'ordre de 85 à 95% selon les cas.

Le composé de formule IX peut être obtenu en préparant tout d'abord une lactone de formule VIII par chauffage au reflux d'un alcool de formule II, dans un solvant approprié tel que l'éthanol et en présence d'un acide tel que l'acide acétique .

On soumet ensuite la lactone de formule VIII à une réduction:

- au moyen d'un hydrure métallique, de préférence l'hydrure de diisobutylaluminium dans un solvant tel que le dichlorométhane ou l'hydrure de lithium-aluminium dans un solvant tel que par exemple le tétrahydrofuranne, le traitement ayant lieu à une température comprise entre -20° C et -70° C, pour obtenir un hémiacétal cyclique de formule IX dans laquelle $R'_1$ représente l'hydrogène.

Généralement, on utilise de 1,5 à 2 équivalents d'hydrure métallique par équivalent de lactone de formule VIII,

- au moyen d'un halogénure d'alkyl magnésium de formule VII dans laquelle M représente un radical -Mg Hal, dans un solvant par exemple un éther tel que le tétrahydrofuranne et à une température comprise entre -80° C et la température ambiante et l'on hydrolyse le complexe formé par exemple en présence d'acide acétique, pour obtenir un hémiacétal cyclique de formule IX dans laquelle $R'_1$ représente un radical alkyle en $C_1$-$C_4$.

Comme hydrure métallique, on préfère l'hydrure de diisobutylaluminium lequel fournit une sélectivité importante de la réduction évitant ainsi la formation d'une quantité significative de produits de réduction plus poussée comme c'est partiellement le cas avec l'hydrure de lithium-aluminium.

Le procédé ainsi décrit permet l'obtention des hémiacétals cycliques de formule IX avec des rendements importants. En effet, on a pu enregistrer selon les cas, des rendements globaux de 75 à 90% à partir du composé de formule II.

D'une autre manière, les dérivés d'aza-6 alkoxy-7 phtalide de formule IX dans laquelle P' représente l'hydrogène peuvent être préparés:

(a) en traitant au moyen d'éthérate de trifluorure de bore et dans un alcool de formule générale:

$R_{13}OH$ XXIV

dans laquelle $R_{13}$ représente un groupement alkyle en $C_1$-$C_4$ par exemple dans le méthanol, un dérivé N-protégé de formule IX, à savoir un dérivé phtalide de formule IX dans laquelle P' représente un groupement labile, pour former un cétal de formule générale:

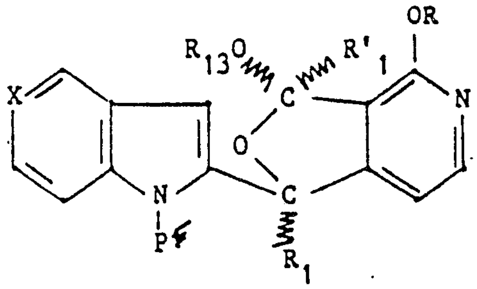

XXV

dans laquelle P, R, $R_1$, $R'_1$, $R_{13}$ et X ont la même signification que précédemment,

(b) en faisant réagir le cétal de formule XXV dans un solvant par exemple un solvant aqueux tel que le méthanol aqueux avec un agent basique tel qu'un carbonate ou bicarbonate de métal alcalin, par exemple le carbonate ou le bicarbonate de potassium, pour obtenir un composé déprotégé de formule générale:

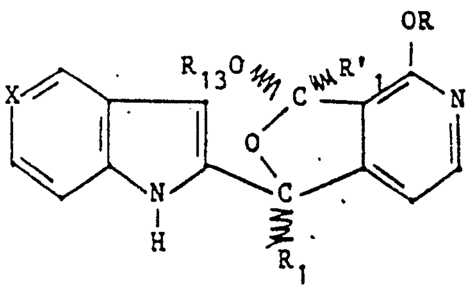

XXVI

dans laquelle R, $R_1$, $R'_1$, $R_{13}$ et X ont la même signification que précédemment,

(c) en hydrolysant le composé déprotégé de formule XXVI en présence d'un agent acide tel que l'acide chlorhydrique ou l'éthérate de trifluorure de bore, pour obtenir le dérivé phtalide désiré de formule IX.

III. Troisième étape

Les mises en oeuvre suivantes des réactions de la troisième étape se sont révélées particulièrement intéressantes.

Ainsi, à titre d'exemple, on peut traiter un équivalent d'un composé de formule IX:

(a) dans laquelle X représente un atome d'azote, P′ représente un radical P et $R'_1$ représentent l'hydrogène, avec 10 à 15 équivalents d'un hydroxyde de métal alcalin, de préférence l'hydroxyde de potassium, durant 15 à 20 heures dans le méthanol au reflux ou durant 1 à 2 heures dans l'éthanol au reflux, pour former les dérivés d'alkoxy-isoquinoléine de formule X dans laquelle X représente un atome d'azote, R représente un radical alkyle en $C_1$-$C_4$ et $R'_1$ représente l'hydrogène,

(b) dans laquelle X représente un groupement C-$OR_2$, P′ a la signification indiquée et $R'_1$ représente un radical alkyle en $C_1$-$C_4$ avec 10 à 15 équivalents d'un hydroxyde de métal alcalin, de préférence l'hydroxyde de potassium, durant 12 à 15 heures, dans le méthanol au reflux, pour former les dérivés d'alkoxy-isoquinoléine de formule X dans laquelle X représente un groupement C-$OR_2$ et R et $R'_1$ représentent chacun un radical alkyle en $C_1$-$C_4$;

(c) dans laquelle X représente un groupement C-$OR_2$, P′ représente un groupement P et $R'_1$ représente l'hydrogène, avec 10 à 15 équivalents d'un hydroxyde de métal alcalin, de préférence l'hydroxyde de potassium, durant 1 à 2 heures dans le méthanol au reflux, pour former les dérivés d'alkoxy-isoquinoléine de formule X dans laquelle X représente un groupement C-$OR_2$, $R'_1$ représente l'hydrogène et R et $R_1$ ont la spécification indiquée.

L'obtention de composés de formule X selon ce procédé résulte de l'intervention de trois étapes chimiques dans le même milieu réactionnel: déprotection de l'azote, cyclisation et aromatisation réductrice en présence d'un seul et même réactif, un hydroxyde de métal alcalin en milieu alcoolique.

Lorsqu'on utilise des conditions de réaction plus douces que celles préconisées ci-dessus, la cyclisation/aromatisation ne s'effectue pas.

En effet, on a remarqué qu'en faisant réagir un dérivé phtalide de formule IX dans laquelle X représente un atome d'azote, avec un hydroxyde de métal alcalin par exemple l'hydroxyde de sodium, dans un alcool ayant de 1 à 3 atomes de carbone, par exemple le méthanol, l'éthanol ou l'isopropanol, à une température allant de -5°C à la température ambiante, on forme non pas le composé de formule X attendu mais un dérivé d'isoquinoléine de formule générale:

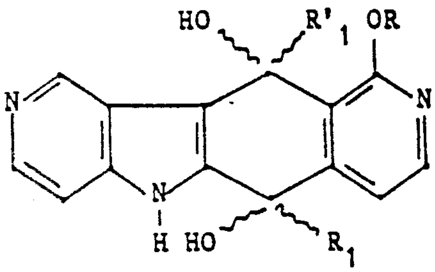

XXVII

dans laquelle R, $R_1$ et R′ ont la même signification que précédemment.

Généralement, cette dernière réaction s'effectue en présence de 1 à 5 équivalents d'hydroxyde de métal alcalin et durant plusieurs heures selon les cas.

Par exemple, on peut faire réagir un équivalent de dérivé phtalide de formule IX dans laquelle X représente un atome d'azote, $R'_1$ représente l'hydrogène et P′ représente l'hydrogène avec un équivalent d'hydroxyde de sodium durant 3 à 4 h ou un équivalent de dérivé phtalide de formule IX dans laquelle X représente un atome d'azote, $R_1$ représente un groupement alkyle en $C_1$-$C_4$ et P′ représente un groupement P avec 4 à 5 équivalents d'hydroxyde de sodium durant 2 à 8 heures pour obtenir un composé de formule XXVII.

De même, on a observé qu'en traitant un dérivé phtalide de formule IX dans laquelle X représente un groupement C-$OR_2$ et $R'_1$ représente un radical alkyle en $C_1$-$C_4$ avec un hydroxyde de métal alcalin, de préférence l'hydroxyde de potassium, dans un alcool en $C_1$-$C_3$ par exemple le méthanol, l'éthanol ou l'isopropanol, à une température allant de 40 à 50° C, on forme un dérivé alkényle de formule générale:

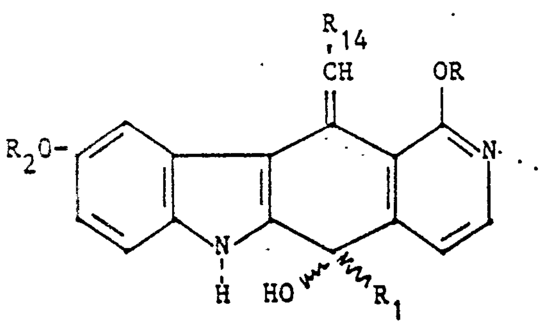

XXVIII

dans laquelle R, $R_1$ et $R_2$ ont la même signification que précédemment et $R_{14}$ représente un atome d'hydrogène ou un radical alkyle en $C_1$-$C_3$.

Généralement, la réaction a lieu en présence de 10 à 15 équivalents d'hydroxyde de métal alcalin et durant plusieurs heures selon les cas.

Par exemple, on peut faire réagir un équivalent de dérivé phtalide de formule IX dans laquelle X représente un groupement C-$OR_2$, $R'_1$ représente un groupement alkyle en $C_1$-$C_4$ et P représente l'hydrogène, avec 10 à 15 équivalents d'hydroxyde de potassium durant 3 à 4 heures pour obtenir un composé de formule XXVIII.

Par contre, l'utilisation de conditions encore plus douces, c'est-à-dire de 1 à 5 équivalents d'hydroxyde de sodium à une température de -5°C à la température ambiante, s'est avérée incapable de provoquer la cyclisation des dérivés phtalides de formule IX dans laquelle X représente un groupement C-$OR_2$.

Les dérivés d'isoquinoléine de formule XXVII et les dérivés alkényles de formule XXVIII peuvent être traités, ensuite, par exemple dans un éther de glycol, de préférence le diméthyléther du diéthylène glycol et à une température comprise entre 100°C et la température de reflux, par exemple de 120°C à 140°C, avec un borohydrure de métal alcalin tel que le borohydrure de sodium, pour fournir les composés désirés de formule X.

Une réaction analogue à celle décrite ci-dessus à partir des composés de formules XXVII et XXVIII figure dans J. Org. Chem. 48, pp. 2690-2695 (1983) dans laquelle on fait réagir un équivalent de dihydroxy-5,11 diméthyl-5,11 dihydro-10,11 5H-pyrido[3,4-b]carbazole avec environ 40 équivalents de borohydrure de sodium pendant 20 heures et au reflux de l'éthanol pour obtenir le diméthyl-5,11 10H-pyrido[3,4-b] carbazole.

L'utilisation dans ce procédé, d'un très large excès de borohydrure de sodium est dû à la destruction parasite de celui-ci par réaction avec l'éthanol à reflux.

L'emploi d'un tel excès conduit cependant à un traitement difficile du mélange réactionnel en fin de réaction dû à l'abondance des sels de bore. Pour cette raison, les composés obtenus doivent être purifiés par chromatographie. Or, on a trouvé, dans le cadre de l'invention, que le remplacement de l'éthanol par un éther de glycol par exemple l'éther diméthylique du diéthylèneglycol permet de réduire considérablement l'excès de borohydrure de sodium nécessaire en passant de 40 équivalents à par exemple 2 à 3 équivalents et de conduire la réaction à plus haute température en un temps très court, par exemple à 120-140°C durant 0,5 heure. Cette mise en oeuvre permet d'obtenir les composés de formule X avec des rendements supérieurs à 90%.

IV. Quatrième étape

On a décrit dans les brevets français cités précédemment, un procédé permettant notamment la préparation des dérivés de formule I ci-dessus à partir de pyridones de formule générale:

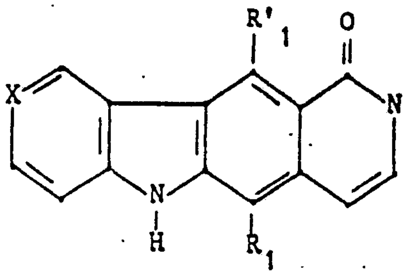

XXIX

dans laquelle X, $R_1$ et $R'_1$ ont la même signification que dans la formule I, par traitement à reflux avec un excès de trichlorure de phosphoryle pour obtenir un dérivé chloro de formule XII et amination du dérivé chloro en question au reflux.

Un des problèmes majeurs rencontrés dans ce mode de synthèse se situe au niveau de la faible solubilité des composés de formule XXIX dans les solvants organiques. Cependant l'utilisation d'un très large excès de trichlorure de phosphoryle en tant que réactif et solvant peut être envisagé pour l'obtention des composés de formule XII. En dépit d'une meilleure solubilité ainsi obtenue, la chloration est cependant difficile à mettre en oeuvre en raison de l'hétérogénéité du milieu, ce qui requiert une agitation spéciale à l'échelle industrielle par exemple sur lit fluidisé.

Au contraire, les composés de formule I peuvent être obtenus facilement avec un rendement élevé à partir d'une alkoxy-isoquinoléine de formule X, par exemple un composé à groupe méthoxy.

Les dérivés alkoxy en question présentent dans les solvants usuels notamment dans l'acétonitrile, un degré de solubilité beaucoup plus important que les pyridones de formule XXIX. Ces dérivés alkoxy de formule X pourront, par conséquent, donner facilement accès aux dérivés chloro de formule XII sans nécessiter l'utilisation d'un aussi large excès de trichlorure de phosphoryle que dans le procédé antérieur.

En outre, les dérivés alkoxy de formule X peuvent fournir les dérivés chloro de formule XII, sans transiter par les pyridones de formule XXIX, ce qui évite l'étape de passage des composés de formule XXIX aux composés de formule XII.

Au surplus, les composés alkoxy de formule X peuvent donner naissance aux composés de formule I, par exemple aux composés de formule Ia, par substitution directe du groupement alkoxy au moyen d'une amine par exemple la diéthylaminopropylamine, dans laquelle ils sont solubles.

Il s'agit là d'un avantage important car cette mise en oeuvre évite la préparation intermédiaire des pyridones de formule XXIX mais également des dérivés chloro de formule XII.

En conséquence, on obtient les composés de formule I en utilisant un procédé selon lequel:

(a) ou bien on traite au reflux, un composé de formule X avec un composé de formule XI et en présence d'un catalyseur acide, par exemple 2 à 4 équivalents de chlorure d'hydrogène ou d'acide trifluorométhanesulfonique, pour obtenir les dérivés aminés d'isoquinoléine de formule I sous forme de base libre.

Cette méthode, d'une mise en oeuvre simple, prévaut sur les trois autres variantes (b), (c) et (d) explicitées ci-dessous;

(b) ou bien, on traite au reflux un composé de formule X au moyen d'oxyde de dichlorophénylphosphine pour obtenir un composé chloro de formule XII que l'on fait alors réagir au reflux avec un composé de formule XI pour former le composé désiré de formule I sous forme de base libre.

(c) ou bien, on traite selon la méthode décrite dans Can. J. Chem. 59, 2601 (1981), un composé de formule X au reflux avec le trichlorure de phosphoryle en présence d'un co-solvant polaire, par exemple l'acétonitrile, d'un chlorure d'ammonium par exemple le chlorure de triéthylbenzylammonium et d'une aniline telle que la diéthylaniline, pour former le composé chloro de formule XII que l'on fait réagir au reflux avec un composé de formule XI pour obtenir le composé désiré de formule I sous forme de base libre.

L'utilisation d'un co-solvant polaire permet d'augmenter l'homogénéité du milieu réactionnel, le chlorure d'ammonium a pour but d'augmenter la concentration du milieu en ion chlorure et d'accélérer ainsi la réaction, tandis que le dérivé aniline a pour effet de solubiliser partiellement le composé de formule X tout en piégeant le chlorure d'alkyle intermédiairement formé pour éviter ainsi l'alkylation du composé de formule X en question. En l'absence de dérivé d'aniline, un dérivé N-alkylpyridone de structure analogue aux composés de formule XXIX a pu être isolé comme produit secondaire;

(d) ou bien, on traite, selon la méthode décrite dans Chem. Ber. 117, 1523-1541 (1984), un composé de formule X au moyen d'un halogénure de triméthylsilyle par exemple le chlorure et en présence d'un halogénure de métal alcalin, de préférence l'iodure, pour obtenir un dérivé triméthylsilyloxy que l'on fait réagir au reflux avec un composé de formule XI et en présence d'hexaméthyldisilazane et d'acide p-toluènesulfonique comme catalyseur pour former le composé désiré de formule I sous forme de base libre. Les exemples non limitatifs suivants illustrent l'invention:

EXEMPLE 1

Préparation de la diéthylaminocarbonyl-3 méthoxy-2 pyridine

(a) Chloro-2 diéthylaminocarbonyl-3 pyridine

Dans un ballon, on introduit 35,5 g (0,2 mole) d'acide chloro-2-nicotinique et 120 ml (8,3 équivalents) de chlorure de thionyle et on maintient au reflux de ce dernier pendant 3 heures. On distille l'excès de chlorure de thionyle sous pression normale et on entraîne les résidus de chlorure de thionyle par distillation azéotropique en présence de toluène.

Le résidu brunâtre de chlorure d'acide chloro-2 nicotinique cristallise au refroidissement et on l'utilise tel quel pour l'étape suivante.

On place le chlorure d'acide obtenu précédemment en solution dans 100 ml de dichlorométhane sec et on refroidit le réacteur au-dessous de 10° C. On ajoute alors, en 1 h à 1,5 h, 62 ml (0,6 mole) de diéthylamine en solution dans 20 ml de dichlorométhane en maintenant la température en-dessus de 30° C. On lave la phase organique avec deux fois 20 ml d'eau, on sèche sur sulfate de sodium, on décolore sur charbon actif puis on filtre.

Après évaporation du solvant, on obtient 41 g d'une huile brune qui après purification par distillation fournit 36,2 g de chloro-2 diéthylaminocarbonyl-3 pyridine.

Rendement: 85%

P.E.: 116° C (0,1 mm Hg)

Analyse centésimale %:

| | C | H | N | Cl |
|---|---|---|---|---|
| Calculé | 56,47 | 6,16 | 13,17 | 16,67 |
| Trouvé | 55,21 | 6,11 | 13,83 | 16,20 |

Spectre I.R. (film):

| | |
|---|---|
| C-H aromatique | 3050 $cm^{-1}$(f) |
| C=O | 1630 $cm^{-1}$(F) |
| C-C aromatique | 1575 $cm^{-1}$(F) |

Spectre RMN ($CDCl_3$):1-1,5 ppm (2t,6H); 3-4 ppm (2q,4H); 7-8.5 ppm (m,3H).

De la même manière que précédemment mais en utilisant la diisopropylamine au lieu de la diéthylamine, on obtient la chloro-2 diisopropylaminocarbonyl-3 pyridine.

Rendement: 99%

P.F.: 117° C

Analyse centésimale %:

| | C | H | N | Cl |
|---|---|---|---|---|
| Calculé | 59,87 | 7,12 | 11,64 | 14,75 |
| Trouvé | 59,68 | 7,26 | 11,30 | 15,19 |

Spectre I.R. (KBr):

| | |
|---|---|
| C-H aromatique | 3060 $cm^{-1}$(f) |
| C=O | 1630 $cm^{-1}$(F) |
| C-C aromatique | 1570 $cm^{-1}$(F) |

Spectre RMN ($CDCl_3$):1,1; 1,2 et 1,3 ppm (3d, 12H); 3-4 ppm (m, 2H); 7,1-7,7 ppm (m,2H); 8,4 ppm (m,1H).

(b) Diéthylaminocarbonyl-3 méthoxy-2 pyridine

On prépare une solution de méthylate de sodium par addition progressive de 22 g(0,96 mole) de sodium en morceaux dans 280 ml de méthanol au reflux jusqu'à disparition totale du solide. On ajoute alors rapidement 40,9 g (0,192 mole) de chloro-2 diéthylaminocarbonyl-3 pyridine en solution dans 160 ml de méthanol absolu et on porte au reflux pendant une douzaine d'heures.

On neutralise l'excès de méthyle de sodium avec une solution d'acide chlorhydrique 3M dans le méthanol et

on filtre le chlorure de sodium formé. On élimine le méthanol sous pression réduite et on reprend le résidu dans du dichlorométhane. Après filtration du chlorure de sodium résiduel, on évapore le solvant pour obtenir 38,9 g de diéthylaminocarbonyl-3 méthoxy-2 pyridine sous forme de cristaux d'un blanc jaunâtre.
Rendement: 97%
P.F.: 83°C

Analyse centésimale %:

| | C | H | N |
|---|---|---|---|
| Calculé | 63,44 | 7,74 | 13,45 |
| Trouvé | 63,63 | 7,89 | 13,44 |

**Spectre I.R. (KBr):**

| | |
|---|---|
| C=O | 1625 $cm^{-1}$ |
| C-N } C-C aromatique | 1580 $cm^{-1}$ |

Spectre RMN ($CDCl_3$):1,0-1,5 ppm (2t, 6H); 3,25 et 3,65 ppm (2q,4H); 4,0 ppm (s,3H); 6,8-8,3 ppm (m,3H).

De la même manière que précédemment mais en portant la chloro-2 diisopropylaminocarbonyl-3 pyridine et le méthylate de sodium au reflux pendant 45 heures, on obtient la diisopropylaminocarbonyl-3 méthoxy-2 pyridine.
Rendement: 98%
P.F.: 109°C

Analyse centésimale %:

| | C | H | N |
|---|---|---|---|
| Calculé | 66,07 | 8,53 | 11,86 |
| Trouvé | 66,55 | 8,85 | 11,56 |

Spectre I.R. (KBr):

| | |
|---|---|
| C-H aromatique | 3100-3000 $cm^{-1}$ (f) |
| C=O | 1620 $cm^{-1}$ (F) |
| C-C aromatique | 1580 $cm^{-1}$ (m) |

Spectre RMN ($CDCl_3$):1,15 et 1,55 ppm (t et d, 12H); 3,5 ppm (m,2H); 4,0 ppm (s,3H); 6,9-8,15 ppm (m,3H).

EXEMPLE 2

Préparation de la tertiobutoxy-2 (diméthyl-4,4 oxazolinyl-2)-3 pyridine

(a) Chloro-2[(diméthyl-1,1 hydroxy-2 éthyl)aminocarbonyl]-3 pyridine

On place 134 ml (1,4 mole) d'amino-2 méthyl-2 propanol en solution dans 600 ml d'un mélange dichloroéthane/tétrahydrofuranne (85/15 v/v) et on refroidit la solution dans un bain de glace/chlorure de sodium. On ajoute 70,4 g (0,4 mole) de chlorure d'acide chloro-2-nicotinique, préparé comme décrit à l'Exemple 1, en solution dans 770 ml de mélange dichlorométhane/tétrahydrofuranne (85/15 v/v) de manière à maintenir une température inférieure ou égale à 0°C.

En fin d'addition, on agite pendant 0,5 h à 0°C puis à température ambiante pendant 1 h. On filtre le chlorhydrate d'amine précipité et on évapore les solvants sous pression réduite. On dissout l'huile résultante dans 2,5 l de chloroforme et on lave avec de l'acide chlorhydrique 12N jusqu'à un pH= 5-6 afin d'éliminer l'aminoalcool en excès. Après décoloration sur charbon actif, séchage sur sulfate de sodium et évaporation du solvant, on obtient 88,9 g de chloro-2[(diméthyl-1,1 hydroxy-2 éthyl)aminocarbonyl]-3 pyridine sous forme d'une poudre blanchâtre.
Rendement: 97%
P.F.: 104°C

Analyse centésimale %:

| | C | H | N | Cl |
|---|---|---|---|---|
| Calculé | 52,52 | 5,73 | 12,25 | 15,50 |
| Trouvé | 52,27 | 6,02 | 12,17 | 15,18 |

Spectre I.R. (KBr):

| | |
|---|---|
| NH, OH | 3250 $cm^{-1}$ (m,l) |
| C=O | 1670 $cm^{-1}$ (F) |
| C-C aromatique | 1570 $cm^{-1}$ (m) |

Spectre RMN ($CDCl_3$):1,4 ppm (s,6H); 3,6 ppm (s,2H); 3,9 ppm (s,1H); 6,6 ppm (s,1H); 7-8,5 ppm (m,3H).

(b) Chloro-2[(diméthyl-1,1 chloro-2 éthyl-1)aminocarbonyl]-3 pyridine

On place 76,8 g (0,336 mole) de chloro-2[(diméthyl-1,1 hydroxy-2 éthyl)aminocarbonyl]-3 pyridine dans un réacteur immergé dans un bain froid (glace + eau). On ajoute alors 244 ml (3,36 moles) de chlorure de thionyle de façon à maintenir une température inférieure à 20°C.

On poursuit l'agitation pendant 2,5 h à 20°C et on évapore le chlorure de thionyle sous pression réduite (température < 50°C). Après élimination du résidu de chlorure de thionyle par distillation azéotropique avec du toluène, on obtient 76 g de chloro-2[(diméthyl-1,1 chloro-2 éthyl-1) aminocarbonyl]-3 pyridine sous forme d'une poudre beige.

Rendement: 91,5%

P.F.: 120°C

Analyse centésimale %:

| | C | H | N | Cl |
|---|---|---|---|---|
| Calculé | 48,60 | 4,89 | 11,34 | 28,69 |
| Trouvé | 48,42 | 4,97 | 11,37 | 27,72 |

Spectre I.R. (KBr):

| | |
|---|---|
| NH | 3350 $cm^{-1}$ (m) |
| C-H aromatique | 3070 $cm^{-1}$ (m) |
| C=O | 1635 $cm^{-1}$ (F) |

Spectre RMN ($CDCl_3$):1,45 ppm (s,6H); 3,85 ppm (s,2H); 6,55 ppm (s,1H); 7-8,5 ppm (m,3H).

(c) Chloro-2(diméthyl-4,4 oxazolinyl-2)-3 pyridine

On place dans 2 l de toluène 75,9 g (0,307 mole) de chloro-2[(diméthyl-1,1 chloro-2 éthyl-1) aminocarbonyl]-3 pyridine et 155 g (1,84 mole) de bicarbonate de sodium et on porte le mélange à reflux sous bonne agitation jusqu'à disparition totale du produit de départ (10 à 15 heures).

On collecte dans un séparateur l'eau formée par la réaction de l'acide chlorhydrique libéré avec le bicarbonate de sodium, on filtre et on lave au toluène le chlorure de sodium formé. On concentre alors le filtrat sous pression réduite pour obtenir 63,3 g de chloro-2(diméthyl-4,4 oxazolinyl-2)-3 pyridine sous forme d'une huile jaune que l'on peut purifier par distillation sous pression réduite.

Rendement: 63,3%

P.E.: 110°C (0,2 mmHg)

$n^{20}$: 1,5397

Analyse centésimale %:

| | C | H | N | Cl |
|---|---|---|---|---|
| Calculé | 57,02 | 5,26 | 13,30 | 16,83 |
| Trouvé | 55,55-59,81 | 5,66-5,28 | 13,65-12,99 | 16,74-16,52 |

Spectre I.R. (film):

| | |
|---|---|
| C-H aromatique | 3100-3000 $cm^{-1}$ (t,f) |
| C=N | 1650 $cm^{-1}$ (F) |
| C-C aromatique | 1575 $cm^{-1}$ (F) |

Spectre R.M.N. ($CDCl_3$):1,45 ppm (s,6H); 4,15 ppm (s,2H); 7-8,5 ppm (m,3H).

d) Tertiobutoxy-2(diméthyl-4,4 oxazolinyl-2)-3 pyridine

On met en solution dans 90 ml de tétrahydrofuranne sec, 32,2 g (0,153 mole) de chloro-2(diméthyl-4,4 oxazolinyl-2)-3 pyridine. On additionne alors 51,4 g (0,459 mole) de tertiobutylate de potassium en solution dans 150 ml de tétrahydrofuranne sec de manière que le milieu soit maintenu au reflux par l'exothermicité de la réaction.

Après refroidissement, on évapore le solvent sous pression réduite. On reprend le résidu dans 800 ml d'éther diisopropylique et on lave avec 2 fois 80 ml d'eau. Après sèchage sur sulfate de sodium, on évapore le solvant sous pression réduite, ce qui fournit 36 g de tertiobutoxy-2 (diméthyl-4,4 oxazolinyl-2)-3 pyridine sous forme d'une huile jaune.

Rendement: 94,7%

$n^{20}$: 1,5047

Analyse centésimale %:

| | C | H | N |
|---|---|---|---|
| Calculé | 67,72 | 8,12 | 11,78 |
| Trouvé | 67,31 | 7,94 | 10.95 |

Spectre I.R. ($CHCl_3$):

| | |
|---|---|
| C-H aromatique | 3100-3000 $cm^{-1}$ (f) |
| C=N | 1650 $cm^{-1}$ (F) |
| C-C aromatique | 1590 $cm^{-1}$ (F) |

Spectre R.M.N. ($CDCl_3$):1,3 ppm (s,6H); 1,55 ppm (s,9H); 4,10 ppm (s,2H); 6,5-8,5 ppm (m,3H).

EXEMPLE 3

Préparation de la (diméthyl-4,4 oxazolinyl-2)-3 méthoxy-2 pyridine.

a) Chloro-2(diméthyl-4,4 oxazolinyl-2)-3 pyridine

On met en suspension dans de l'hexaméthyldisilazane 24,7 g (0,10 mole) de chloro-2[(diméthyl-1,1 chloro-2 éthyl-1)aminocarbonyl]-3 pyridine préparé comme décrit à l'Exemple 2c et on chauffe à reflux pendant 6h (environ 125° C). On évapore, sous pression réduite l'excès d'hexaméthyldisilazane et on distille le résidu sous haut vide pour fournir 16,27 g de chloro-2 (diméthyl-4,4 oxazolinyl-2)-3 pyridine.

P.E.: 110°C (0,2 mm Hg).

Mêmes données spectrales que dans l'Exemple 2c.

b) (Diméthyl-4,4 oxazolinyl-2)-3 méthoxy-2 pyridine

On prépare une solution de méthylate de sodium par addition progressive de 17,4 g (0,76 mole) de sodium en morceaux dans 210 ml de méthanol au reflux jusqu'à disparition totale du solide. On ajoute alors rapidement 34,6 g (0,192 mole) de chloro-2 (diméthyl-4,4 oxazolinyl-2)-3 pyridine en solution dans 160 ml de méthanol absolu et on porte à reflux pendant 6 heures. On neutralise l'excès de méthylate de sodium avec une solution d'acide chlorhydrique 3M dans le méthanol et on filtre le chlorure de sodium formé. On élimine le méthanol sous pression réduite et on reprend le résidu dans du dichlorométhane. Après filtration du chlorure de sodium résiduel, on évapore le solvant pour obtenir la (diméthyl-4,4 oxazolinyl-2)-3 méthoxy-2 pyridine sous forme d'une huile jaune.

Rendement: 95%

Spectre I.R. ($CHCl_3$):

| | |
|---|---|
| C-H aromatique | 3100-3000 $cm^{-1}$ (f) |
| C=N | 1640 $cm^{-1}$ (F) |
| C-C aromatique | 1590 $cm^{-1}$ (F) |

Spectre R.M.N. ($CDCl_3$):1,4 ppm (s,6H); 4,02 ppm (s,3H); 4,07 ppm (s,2H); 6,7-7,0 ppm (m,2H); 7,9-8,4 ppm (m.2H).

EXEMPLE 4

Préparation de la (diméthyl-4,4 oxazolinyl-2)-3 méthoxy-2 pyridine

a) Chloro-2 (diméthyl-4,4 oxazolinyl-2)-3 pyridine

On place 76,8 g (0,336 mole) de chloro-2[(diméthyl-1,1 hydroxy-2 éthyl)aminocarbonyl]-3 pyridine dans un réacteur immergé dans un bain froid (glace + eau). On ajoute alors 244 ml (3,36 moles) de chlorure de thionyle de façon à maintenir une température inférieure à 20° C. On poursuit l'agitation pendant 2,5h à 20° C et on évapore le chlorure de thionyle sous pression réduite (température < 50° C).

A la solution de chloro-2[(diméthyl-1,1 chloro-2 éthyl-1)-aminocarbonyl]-3 pyridine ainsi formée, on ajoute 2l de toluène et 155 g (1,84 mole) de bicarbonate de sodium et on porte le mélange à reflux sous bonne agitation jusqu'à disparition totale du produit de départ (10 à 15h). On collecte dans un séparateur l'eau formée par la réaction de l'acide chlorhydrique libéré avec le bicarbonate de sodium et on filtre et lave au toluène le chlorure de sodium formé. On concentre alors le filtrat sous pression réduite pour obtenir la chloro-2 (diméthyl-4,4 oxazolinyl-2)-3 pyridine sous forme d'une huile jaune que l'on peut purifier par distillation sous pression réduite.

b) (Diméthyl-4,4 oxazolinyl-2)-3 méthoxy-2 pyridine

Ce composé a été obtenu selon l'Exemple 3b à partir de chloro-2 (diméthyl-4,4 oxazolinyl-2)-3 pyridine préparée comme décrit précédemment.
Rendement: 90%

EXEMPLE 5

Préparation de la (diméthyl-4,4 oxazolinyl-2)-3 isopropoxy-2 pyridine

On met en suspension en 10 minutes dans 150 ml de tétrahydrofuranne sec et 40 ml d'isopropanol, 12 g d'hydrure de sodium (50% dans l'huile = 0,25 mole), ce qui provoque l'élévation de la température jusqu'à 50° C. On ajoute alors, en 20 minutes, une solution de 10,52 g (0,05 mole) de chloro-2 (diméthyl-4,4 oxazolinyl-2)-3 pyridine dans 30 ml de tétrahydrofuranne sec et on abandonne le mélange pendant 1,25 heure à température ambiante. On élimine l'isopropanol en excès et le tétrahydrofuranne sous vide partiel et on reprend le résidu dans 50 ml d'eau et 100 ml d'éther diisopropylique. On évapore alors la phase éthérée sous pression réduite pour obtenir 12,5 g de (diméthyl-4,4 oxazolinyl-2)-3 isopropoxy-2 pyridine sous forme d'une huile brun clair.
Rendement: 95%

Spectre I.R. (film):

| | |
|---|---|
| C=N | 1640 $cm^{-1}$ |
| C-C aromatique | 1580 $cm^{-1}$ |

Spectre R.M.N. ($CDCl_3$): 1,35 ppm (s,6H) + (d,6H); 4,1 ppm (s,2H); 5,4 ppm (m,1H); 6,7-7 ppm (m,1H); 7,9-8,4 ppm (m,2H).

EXEMPLE 6

Préparation de la diéthylaminocarbonyl-3 lithio-4 méthoxy-2 pyridine.

On prépare une solution de 0,17 mole de tétraméthyl-2,2,6,6 pipéridure de lithium par addition de 0,17 mole de n-butyllithium (2,3 moles) dans 74 ml de cyclohexane à 0,17 mole (28,7 ml) de tétraméthyl-2,2,6,6 pipéridine dans 120 ml de tétrahydrofuranne à température inférieure à -20° C.

A une solution de 20,8 g (0,1 mole) de diéthylaminocarbonyl-3 méthoxy-2 pyridine et de 25,65 ml (0,17 mole) de tétraméthyléthylènediamine dans 200 ml de tétrahydrofuranne refroidie à -70° C, on ajoute alors cette solution en quelques minutes en maintenant la température du milieu inférieure ou égale à -70° C.

On obtient ainsi une solution homogène orangée de diéthylaminocarbonyl-3 lithio-4 méthoxy-2 pyridine que l'on utilise telle quelle.

De la même manière, on prépare une solution de diisopropylaminocarbonyl-3 lithio-4 méthoxy-2 pyridine.

EXEMPLE 7

Préparation de la diéthylaminocarbonyl-3 lithio-4 méthoxy-2 pyridine

A une solution de 5,2 g (0,025 mole) de diéthylaminocarbonyl-3 méthoxy-2 pyridine, 2,12 ml (0,0125 mole) de tétraméthyl-2,2,6,6 pipéridine et 5,66 ml (0,0375 mole) de tétraméthyléthylènediamine dans 72,5 ml de tétrahydrofuranne refroidie à -80° C, on ajoute en 15 à 30 minutes, 0,0375 mole de n- butyllithium (16,7 ml; 20% dans le cyclohexane) de façon à maintenir une température inférieure à -75° C.

De cette manière, on obtient une solution orangée de diéthylaminocarbonyl-3 lithio-4 méthoxy-2 pyridine que l'on utilise telle quelle.

En utilisant le même procédé que précédemment, on prépare une solution de diisopropylaminocarbonyl-3 lithio-4 méthoxy-2 pyridine.

EXEMPLE 8

Préparation de la diéthylaminocarbonyl-3 lithio-4 méthoxy-2 pyridine.

A une solution de 1,041 g (0,005 mole) de diéthylaminocarbonyl-3 méthoxy-2 pyridine et de 1,13 ml (0,0075 mole) de tétraméthyléthylènediamine dans 15 ml de tétrahydrofuranne refroidie à -80° C, on ajoute en quelques minutes 0,0075 mole de tertiobutyllithium (4,41 ml d'une solution 1,7 molaire dans le pentane) en maintenant la température inférieure à -80° C. Après 20 minutes à cette température, on obtient une solution orangée de diéthylaminocarbonyl-3 lithio-4 méthoxy-2 pyridine que l'on utilise telle quelle.

De la même manière que ci-dessus, on prépare une solution de diisopropylaminocarbonyl-3 méthoxy-2 pyridine.

EXEMPLE 9

Préparation de la (diméthyl-4,4 oxazolinyl-2)-3 isopropoxy-2 lithio-4 pyridine.

A une solution de 15,20 ml (0,09 mole de tétraméthyl-2,2,6,6 pipéridine et de 29 g (0,09 mole) de tris(dioxa-3,6 heptyl)amine, dans 30 ml de tétrahydrofuranne refroidie à température inférieure à -75° C, on ajoute 40,8 ml (0,09 mole; solution 2,2 molaire dans l'hexane) de n-butyllithium de façon à maintenir une température inférieure à -70° C.

On ajoute alors en 5 minutes, 7,02 g (0,03 mole) de (diméthyl-4,4 oxazolinyl-2)-3 isopropoxy-2 pyridine en solution dans 55 ml de tétrahydrofuranne en maintenant la température inférieure à -70° C. Après 20 minutes d'agitation à cette température, on obtient une solution homogène jaune orangé de (diméthyl-4,4 oxazolinyl-2)-3 isopropoxy-2 lithio-4 pyridine que l'on utilise telle quelle.

De la même manière, on prépare une solution de (diméthyl-4,4 oxazolinyl-2)-3 tertiobutoxy-2 lithio-4 pyridine.

EXEMPLE 10

Préparation de la (diméthyl-4,4 oxazolinyl-2)-3 isopropoxy-2 lithio-4 pyridine.

a) On utilise le même procédé que celui décrit à l'Exemple 9 mais en remplaçant le système de solvant tétrahydrofuranne/tris(dioxa-3,6 heptyl) amine par la tétraméthyléthylènediamine seule ou en mélange avec du tétrahydrofuranne.

De la même manière que ci-dessus on prépare une solution de (diméthyl-4,4 oxazolinyl-2)-3 tertiobutoxy-2 lithio-4 pyridine.

b) On utilise le même procédé que celui décrit à l'Exemple 9 mais en remplaçant le système de solvant tétrahydrofuranne/tris(dioxa-3,6 heptyl) amine par un mélange tétraméthyléthylènediamine/tris(dioxa-3,6 heptyl)amine.

De la même manière que ci-dessus, on prépare une solution de (diméthyl-4,4 oxazolinyl-2)-3 tertiobutoxy-2 lithio-4 pyridine.

EXEMPLE 11

Préparation de la diéthylaminocarbonyl-3 formyl-4 méthoxy-2 pyridine.

A la solution de diéthylaminocarbonyl-3 lithio-4 méthoxy-2 pyridine obtenue à l'Exemple 6 et maintenue à une température inférieure à -70° C, on ajoute en 10 à 15 minutes, un réactif à caractère électrophile à savoir 27,8 ml (3,5 équivalents) de diméthylformamide en solution dans 20 ml de tétrahydrofuranne. On verse le milieu réactionnel dans une solution de 57 ml d'acide chlorhydrique à 37% dans 150 ml d'eau et on extrait avec 3 fois 150 ml de toluène. Après séchage sur sulfate de sodium, on évapore le solvant sous pression réduite.

De cette manière, on obtient 23,9 g de diéthylaminocarbonyl-3 formyl-4 méthoxy-2 pyridine sous forme d'une huile jaune titrant 85% en aldéhyde.

Rendement: 85%

Une recristallisation à froid ou une purification chromatographique conduit à un échantillon analytique.

P.F.: < 50° C (solide pâteux).

Analyse centésimale %:

| | C | H | N |
|---|---|---|---|
| Calculé | 61 | 6,83 | 11,86 |
| Trouvé | 60,28 | 6,84 | 11,63 |

Spectre I.R. (film):

| | |
|---|---|
| C=O | 1700 $cm^{-1}$ (F) aldéhyde |
| C=O | 1625 $cm^{-1}$ (F) amide |
| C-C aromatique | 1570 $cm^{-1}$ (F) |

Spectre R.M.N. ($CDCl_3$):1,25 ppm (2t,6H); 3,0-4,0 ppm (2q,4H); 4,0 ppm (s,3H); 7,4 ppm (d,1H); 8,4 ppm (d,1H); 10,1 ppm (s,1H).

En suivant le même procédé que celui décrit ci-dessus, on a préparé les composés suivants:

a) Acétyl-4 diéthylaminocarbonyl-3 méthoxy-2 pyridine.

Réactif à caractère électrophile:

| | |
|---|---|
| acétate d'éthyle | Rendement: 30% |
| acétonitrile | Rendement: 17% |
| N-méthyl N-méthoxy acétamide | Rendement: 45% |

Le produit se présente sous forme huileuse.

Spectre I.R. (film):

| | |
|---|---|
| C=O | 1705 $cm^{-1}$ (F) cétone |
| C=O | 1635 $cm^{-1}$ (F) amide |
| C-C aromatique | 1590 $cm^{-1}$ (F) |

Spectre R.M.N. ($CDCl_3$):1,05 et 1,25 ppm (2t, 6H); 2,50 ppm (s,3H); 3,15 et 3,50 ppm (2q,4H); 3,90 ppm (s,3H); 7,15 ppm (d,1H); 8,25 ppm (d,1H).

b) Acide diéthylaminocarbonyl-3 méthoxy-2 isonicotinique.

Réactif à caractère électrophile:

anhydride carbonique Rendement: 57%

P.F.: 145°C.

Analyse centésimale %:

| | C | H | N |
|---|---|---|---|
| Calculé | 57,13 | 6,39 | 11,10 |
| Trouvé | 56,84 | 6,46 | 10,92 |

Spectre I.R. (KBr):

| | |
|---|---|
| OH | 3500-3400 $cm^{-1}$ (m) |
| C=O | 1725 $cm^{-1}$ (F) acide |
| C=O | 1635 $cm^{-1}$ (F) amide |
| C-C aromatique | 1575 $cm^{-1}$ (F) |

Spectre R.M.N. ($CDCl_3$/$DMSOD_6$):0,8-1,2 ppm (m,6H); 2,8-3,8 ppm (m,4H); 3,9 ppm (s,3H); 7,35 ppm (d,1H); 8,15 ppm (d,1H).

EXEMPLE 12

Préparation de la diisopropylamino-3 formyl-4 méthoxy-2 pyridine.

Ce composé a été préparé selon le procédé de l'Exemple 11.
Réactif à caractère électrophile:
formiate d'éthyle Rendement: 64,5%
P.F.: 103-104°C.

Spectre I.R. (KBr):

| | |
|---|---|
| C=O | 1715 $cm^{-1}$ (F) aldéhyde |
| C=O | 1630 $cm^{-1}$ (F) amide |
| C-C aromatique | 1570 $cm^{-1}$ (F) |

Spectre R.M.N. ($CDCl_3$) (rotamères)1,0-1,8 ppm (m,12H); 3,3-3,9 ppm (m,2H); 4,0 ppm (s,3H); 7,35 ppm (d,1H); 8,30 ppm (d,1H); 10,1 ppm (s,1H).

En utilisant le même procédé que ci-dessus, on a préparé le composé suivant:
Acétyl-4 diisopropylaminocarbonyl-3 méthoxy-2 pyridine.

Réactif à caractère électrophile:

| | |
|---|---|
| anhydride acétique | Rendement: 25-30% |
| acétate d'éthyle | Rendement: 49% |
| diméthylacétamide | Rendement: 15-20% |
| acétonitrile | Rendement: 13% |

Le produit se présente sous forme d'un solide blanc.
P.F.: 124°C

Spectre I.R. (KBr):

| | |
|---|---|
| C=O | 1700 $cm^{-1}$ (F) cétone |
| C=O | 1625 $cm^{-1}$ (F) amide |
| C-C aromatique | 1560 $cm^{-1}$ (F) |

Spectre R.M.N. ($CDCl_3$):1,0-1,7 ppm (m,12H); 2,5 ppm (s,3H); 3,2-3,8 ppm (m,2H); 3,9 ppm (s,3H); 7,1 ppm (d,1H); 8,2 ppm (d,1H).

EXEMPLE 12 BIS

Préparation du benzènesulfonyl-1 aza-5 indolyl-2)-1 (diisopropylaminocarbonyl-3 méthoxy-2 pyridyl-4)-1 éthanol.

On a opéré comme à l'Exemple 12 en utilisant comme réactif à caractère électrophile l'acétyl-2 benzènesulfonyl-1 aza-5 indole. Rendement: 23%.
P.F.: 230-232°C

Spectre I.R. (KBr):

| | |
|---|---|
| OH | 3500-3100 $cm^{-1}$ (m) |
| C=O | 1625 $cm^{-1}$ (F) |
| C-C aromatique | 1565 $cm^{-1}$ (F) |
| $SO_2$-N | (1340 $cm^{-1}$ (F) |
| | (1175 $cm^{-1}$ (F) |

Spectre R.M.N. ($CDCl_3$/Acide trifluoroacétique) (rotamères):1,0-1,8 ppm (m,18H); 2,0-2,2 ppm (m,3H); 3,3-3,9 ppm (m,2H); 3,95 ppm (s,3H); 6,4 ppm (m,1H); 7,0-8,7 ppm (m,10H); 9,15 ppm (s,1H).

En utilisant le même procédé on a préparé le (benzènesulfonyl-1 aza-5 indolyl-2) (diisopropylaminocarbonyl-3 méthoxy-2 pyridyl-4)méthanol.
Réactif à caractère électrophile:
benzènesulfonyl-1 formyl-2 aza-5 indole
Rendement: 40%
P.F.: 234°C

Spectre I.R. (KBr):

| | |
|---|---|
| C=O | 1627 $cm^{-1}$ (F) |
| C-C aromatique | 1570 $cm^{-1}$ (F) |

Spectre R.M.N. ($CDCl_3$/$DMSOD_6$):0,9-1,7 ppm (m,12H); 3,1-3,9 ppm (m,3H); 3,95 ppm (s,3H); 6,5-9,0 ppm (m,11H); 9,25 ppm (s,1H).

EXEMPLE 13

Préparation de la (diméthyl-4,4 oxazolinyl-2)-3 isopropoxy-2 (N-methyl-N-méthoxyaminocarbonyl)-4 pyridine.

A la solution de (diméthyl-4,4 oxazolinyl-2)-3 isopropoxy-2 lithio-4 pyridine préparée selon l'Exemple 9 à l'échelle de 0,005 mole et maintenue à une température inférieure à -65°C, on ajoute en 5 minutes, un réactif à caractère électrophile à savoir du chlorure de N-méthoxy-N-méthylcarbamoyle. La température s'élève à -15°C à la suite de l'exothermicité de la réaction. Après refroidissement à -65°C et addition de 3 ml d'eau, on laisse revenir à 20°C. Après évaporation du solvant, on purifie, par chromatographie sur colonne de gel de silice, le résidu obtenu.

De cette manière, on obtient la (diméthyl-4,4 oxazolinyl-2)-3 isopropoxy-2 (N-méthyl-N-méthoxyaminocarbonyl)-4 pyridine sous forme d'une huile.
Rendement: 41%.

Spectre I.R. (film):

| | |
|---|---|
| C=N | (1660 $cm^{-1}$ (F, large) |
| C=O | ( |
| C-C aromatique | 1575 $cm^{-1}$ (F) |

Spectre R.M.N. ($CDCl_3$):
1,0-1,5 ppm (m,12H); 3,15 ppm (s,3H); 3,50 ppm (s,3H); 5,0-5,5 ppm (m,1H); 6,85 ppm (d, 1H); 8,1 ppm (d,1H).

De la même manière que celle décrite ci-dessus, on a préparé:

a) Acide (diméthyl-4,4 oxazolinyl-2)-3 isopropoxy-2 pyridyl-4 carboxylique
Réactif à caractère électrophile:
anhydride carbonique Rendement: 50%
P.F.: 195°C

Spectre I.R. (KBr):

| | |
|---|---|
| OH | 3600-3300 $cm^{-1}$ (m) |
| C=O | 1700 $cm^{-1}$ (F) |
| C=N | 1660 $cm^{-1}$ (F) |
| C-C aromatique | 1570 $cm^{-1}$ (F) |

Spectre R.M.N. ($CDCl_3$/$DMSOD_6$): 1,2-1,8 ppm (m,12H); 4,05 ppm (s,2H); 5,0-5,5 ppm (m,2H); 7.25 ppm (d.1H); 8.2 ppm (d,1H); 9,5-10,5 ppm (large signal, 1H).

b) [Diméthylaminosulfonyl-1 aza-5 indolyl-2][(diméthyl-4,4 oxazolinyl-2)-3 isopropoxy-2 pyrdyl-4]méthanol.
Réactif à caractère électrophile:
diméthylaminosulfonyl-1 formyl-2 aza-5 indole Rendement: 67%
P.F.: 195°C

Spectre I.R. (KBr):

| | |
|---|---|
| OH | 3300-3300 $cm^{-1}$ |
| C=N | 1655 $cm^{-1}$ |
| C-C aromatique | 1590, 1570 $cm^{-1}$ |

Spectre R.M.N. ($CDCl_3$ + $D_2O$):1,2-1,5 ppm (m,12H); 2,85 ppm (s,6H); 3,9 ppm (s,2H); 5,0-5,5 ppm (m,1H); 6.4-8.8 ppm (m,7H).

EXEMPLE 14

Préparation du (benzènesulfonyl-1 méthoxy-5 indolyl-2) (diéthylaminocarbonyl-3 méthoxy-2 pyridyl-4) méthanol.

a) Benzènesulfonyl-1 méthoxy-5 indole.

On place dans 850 ml de dichlorométhane 29,4 g (0,20 mole) de méthoxy-5 indole, 20 g (0,50 mole) d'hydroxyde de sodium pulvérisé et 0.78 g (0,0020 mole) de sulfate acide de tétrabutylammonium comme catalyseur de transfert de phase puis on agite la solution vigoureusement.

On ajoute ensuite, en une heure, 38,3 ml (0,30 mole) de chlorure de benzènesulfonyle et l'on enregistre une élévation de température de 20° à 40°C. On maintient l'agitation pendant une heure après la fin de l'addition. On essore alors l'excès d'hydroxyde de sodium et le chlorure de sodium formé et on lave le filtrat à l'eau jusqu'à pH=7-8. Après séchage sur sulfate de magnésium et décoloration partielle sur charbon actif, on élimine le solvant sous vide réduit.

On obtient ainsi 51,6 g de benzènesulfonyl-1 méthoxy-5 indole après recristallisation dans l'éthanol.
Rendement: 90%
P.F.: 112°C

Spectre I.R. (KBr):

| | |
|---|---|
| C-H aromatique | 3140-3040 $cm^{-1}$ (M) |
| C-C aromatique | 1580-1605 $cm^{-1}$ (F) |
| $SO_2$ | 1370 $cm^{-1}$ |

Spectre R.M.N. ($CDCl_3$):3,7 ppm (s,3H); 6,55 ppm (d,1H); 6,7-8,0 ppm (m,9H).

b) Benzènesulfonyl-1 lithio-2 méthoxy-5 indole

On refroidit à -70°C une solution de 0,005 mole de benzènesulfonyl-1 méthoxy-5 indole, 0,0075 mole d'agent de stabilisation et 0,0025 mole d'amine dans 7,6 ml de tétrahydrofuranne. On ajoute alors, en 15 minutes, 0,0075 mole de n-butyllithium en maintenant la température en dessous de -60°C et on agite la solution obtenue pendant 30 minutes à cette température.

On obtient ainsi une solution de benzènesulfonyl-1 lithio-2 méthoxy-5 indole en utilisant la tris(dioxa-3,6 heptyl)amine comme agent de stabilisation et la tétraméthyl-2,2,6,6 pipéridine comme amine.

On a estimé le taux de métallation en opérant comme décrit précédemment pour obtenir le benzènesulfonyl-1 deutério-2 méthoxy-5 indole.
Taux de métallation: $\leqq$ 85% (R.M.N. du proton)
spectre R.M.N. ($CDCl_3$)
3,7 ppm (s,3H); 6,55 ppm (s,1H); 6,8-8,0 ppm (m,8H).

c) (Benzènesulfonyl-1 méthoxy-5 indolyl-2) (diéthylaminocarbonyl-3 méthoxy-2 pyridyl-4)méthanol.

A la solution de benzènesulfonyl-1 lithio-2 méthoxy-5 indole obtenue précédemment et maintenue à une température inférieure à -70°C, on ajoute en 10 à 15 minutes 1,5 équivalent de réactif à caractère électrophile dans le tétrahydrofuranne. On verse le milieu réactionnel dans une solution d'acide chlorhydrique (57 ml d'acide à 37%-150 ml d'eau) et on extrait 3 fois avec du toluène. Après séchage sur sulfate de sodium, on évapore le solvant sous pression réduite.

De cette manière, on obtient le (benzènesulfonyl-1 méthoxy-5 indolyl-2) (diéthylaminocarbonyl-3 méthoxy-2 pyridyl-4)méthanol.
Réactif à caractère électrophile:
diéthylaminocarbonyl-3 formyl-4 méthoxy-2 pyridine
Rendement: 63%
P.F.: 110°C

Spectre I.R. (KBr):

| | |
|---|---|
| OH | 3600-3200 $cm^{-1}$ (m) |
| C=O | 1620 $cm^{-1}$ (F) |
| C-C aromatique | 1685-1670 $cm^{-1}$ (F) |

Spectre R.M.N. ($CDCl_3$):0,8-1,4 ppm (m,6H); 2,8-3,8 ppm (m,5H); 3,7 ppm (s,3H); 3,9 ppm (s,3H); 6,1-8,8 ppm (m,12H).

De la même manière que décrite précédemment, on a préparé les composés suivants:

1) (Benzènesulfonyl-1 aza-5 indolyl-2) (diéthylaminocarbonyl-3 méthoxy-2 pyridyl-4)méthanol.

A partir de benzènesulfonyl-1 lithio-2 aza-5 indole et de diéthylaminocarbonyl-3 formyl-4 méthoxy- 2 pyridine comme réactif à caractère électrophile, préparé selon le paragraphe b) [agent de stabilisation: tris(dioxa-3,6 heptyl)amine; amine: diisopropylamine].
Solide blanc.
Rendement: 74%
P.F.: 166°C

Analyse élémentaire %:

| | C | H | N | S |
|---|---|---|---|---|
| Calculé | 60,96 | 5,32 | 11,37 | 6,51 |
| Trouvé | 60,96 | 5,44 | 11,31 | 6,87 |

Spectre I.R. (KBr):

| | |
|---|---|
| OH | 3300-2700 $cm^{-1}$ (m,l) |
| C=O | 1620 $cm^{-1}$ (F) |
| C-C aromatique | 1590-1570 $cm^{-1}$ (M) |

Spectre R.M.N. ($CDCl_3$ + 10% $CF_3CO_2H$):0,8-1,4 ppm (2t,6H); 2,8-3,9 ppm (2q,4H); 3,9 ppm (s,3H); 6,5-9,2 ppm (m,12H).

2) (Tertiobutyloxycarbonyl-1 aza-5 indolyl-2)(diéthylaminocarbonyl-3 méthoxy-2 pyridyl-4)méthanol.

A partir de tertiobutyloxycarbonyl-1 lithio-2 aza-5 indole et de diéthylaminocarbonyl-3 formyl-4 méthoxy-2 pyridine comme réactif à caractère électrophile, préparé selon le paragraphe b (agent de stabilisation: tétraméthyléthylènediamine; amine: tétraméthyl-2,2,6,6 pipéridine).

Purification chromatographique sur silice

Rendement: 57%

P.F.:102°C

Analyse élémentaire %:

| | C | N | H |
|---|---|---|---|
| Calculé | 63,42 | 6,65 | 12,33 |
| Trouvé | 62,70 | 6,69 | 11,89 |

Spectre I.R. (Kbr):

| | |
|---|---|
| OH | 3300-2700 $cm^{-1}$ (m) |
| C=O | 1750 $cm^{-1}$ (F) uréthane |
| C=O | 1625 $cm^{-1}$ (F) amide |
| C-C aromatique | 1590 $cm^{-1}$ (F) |

Spectre R.M.N. ($CDCl_3$):0,9-1,6 ppm (m,15H); 2,8-3,9 ppm (m,5H); 3,95 ppm (s,3H); 6,5-7,4 ppm (m,4H); 8,0-8.3 ppm (m,2H); 8,8 ppm (s,1H).

EXEMPLE 15

Préparation du (diméthylaminosulfonyl-1 aza-5 indolyl-2) (diéthylaminocarbonyl-3 méthoxy-2 pyridyl-4 méthanol.

a) Diméthylaminosulfonyl-1 aza-5 indole

On place dans 100 ml de dichlorométhane, 3,84 g (0,032 mole) d'aza-5 indole, 3,2 g (0,08 mole) d'hydroxyde de sodium pulvérisé et 0,12 g (3,6 x $10^{-4}$ mole) de sulfate acide de tétrabutylammonium comme catalyseur de transfert de phase puis on agite la solution vigoureusement.

On ajoute ensuite 5 ml (0,048 mole) de chlorure de diméthylaminosulfonyle et l'on enregistre une élévation de température de 20° à 40°C. On maintient l'agitation pendant une heure après la fin de l'addition. On essore alors l'excès d'hydroxyde de sodium et le chlorure de sodium formé et on lave le filtrat à l'eau jusqu'à pH = 7-8.

Après sèchage sur sulfate de magnésium et décoloration partielle sur charbon actif, on élimine le solvant sous vide réduit.

On obtient ainsi un résidu brun que l'on purifie par chromatographie sur silice pour donner 6,1 g de diméthylaminosulfonyl-1 aza-5 indole sous forme d'un solide beige.

Rendement: 83%

P.F.: 87°C.

**Analyse élémentaire %:**

| | C | H | N | S |
|---|---|---|---|---|
| Calculé | 47,99 | 4,72 | 18,65 | 14,23 |
| Trouvé | 47,76 | 4,96 | 18,71 | 14,30 |

Spectre I.R. (KBr):

| | |
|---|---|
| C-H aromatique | 3140, 3040 $cm^{-1}$ (f) |
| C-C aromatique | 1593 $cm^{-1}$ (F) |
| $SO_2N$ | 1380 $cm^{-1}$ (F) |

Spectre R.M.N. ($CDCl_3$):2,8 ppm (s,6H); 6,7 ppm (d,1H); 7,45 ppm (d,1H); 7,8 ppm (d,1H); 8,4 ppm (d,1H); 8,9 ppm (s,1H).

b) Diméthylaminosulfonyl-1 lithio-2 aza-5 indole

On refroidit à -60°C une solution de 1,12 g (0,005 mole) de diméthylaminosulfonyl-1 aza-5 indole et de 0,75 ml (0,005 mole) de tétraméthyléthylènediamine dans 10 ml de tétrahydrofuranne. On ajoute alors en quelques minutes, 0,009 mole de diisopropylamidure de lithium (préparé par réaction de 0,009 mole de n-butyllithium avec 0,009 mole de diisopropylamine dans 5 ml de tétrahydrofuranne à température inférieure à 0°C) de façon que la température du milieu n'excède pas -40°C. On agite alors la solution à -60°C pendant 30 minutes.

On obtient ainsi une solution de diméthylaminosulfonyl-1 lithio-2 aza-5 indole que l'on utilise telle quelle.

c) Diméthylaminosulfonyl-1 formyl-2 aza-5 indole

A la solution de diméthylaminosulfonyl-1 lithio-2 aza-5 indole obtenue précédemment, on ajoute 1,11 g (0,015 mole) de formiate d'éthyle et on suit l'avancement de la réaction par chromatographie sur couche mince. On neutralise le milieu réactionnel par l'acide chlorhydrique 1N et on extrait au dichlorométhane. Après sèchage et évaporation de la phase organique, on purifie le résidu par chromatographie sur silice.

De cette manière, on obtient la diméthylaminosulfonyl-1 formyl-2 aza-5 indole avec un rendement de 63%.

Spectre I.R. (film)

| | |
|---|---|
| C=O | 1660 $cm^{-1}$ (F) |
| $N-SO_2$ | 1390 $cm^{-1}$ (F) |

Spectre R.M.N. ($DMSOD_6$):3,9 ppm (s,6H); 7,8 ppm (s,1H); 8 ppm (d,1H); 8,6 ppm (d,1H); 9,2 ppm (s,1H); 10,4 ppm (s,1H).

EXEMPLE 15 BIS

Préparation du (diméthylaminosulfonyl-1 aza-5 indolyl-2) (diéthylaminocarbonyl-3 méthoxy-2 pyridyl-4) méthanol.

Ce composé a été obtenu à partir de diméthylaminosulfonyl-1 lithio-2 aza-5 indole obtenu en b) à l'Exemple 15 et de diéthylaminocarbonyl-3 formyl-4 méthoxy-2 pyridine comme réactif à caractère électrophile.
Purification chromatographie sur silice.
Rendement: 67%
P.F.: 204°C.

Spectre I.R. (KBr):

| | |
|---|---|
| OH | 3500-3000 $cm^{-1}$ (m) |
| C=O | 1630 $cm^{-1}$ (F) |
| C-C aromatique | 1590-1570 $cm^{-1}$ (F) |
| $N-SO_2$ | 1380 $cm^{-1}$ (F) |

Spectre R.M.N. ($CDCl_3$ $DMSOD_6$):0,7-1,4 ppm (m,6H); 2,6-3,7 ppm (m,10H); 3,9 ppm (s,3H); 5,4 ppm (s,1H); 5,6-6,2 ppm (m,1H); 6,4 ppm (d,1H); 7,1 ppm (d,1H); 8,1 ppm (d,1H); 8,3 ppm (d,1H); 8,7 ppm (s,1H).

EXEMPLE 16

Préparatioon du (benzènesulfonyl-1 méthoxy-5 indolyl-2) (diéthylaminocarbonyl-3 méthoxy-2 pyridyl-4)méthanol.

a) Benzènesulfonyl-1 lithio-2 méthoxy-5 indole

On prépare une solution de 0,15 mole de tétraméthyl-2,2,6,6 pipéridure de lithium par addition de 0,15 mole de n-butyllithium à 0,15 mole de tétraméthyl-2,2,6,6 pipéridine dans 120 ml de tétrahydrofuranne à température inférieure à -20°C.

A une solution de 0,1 mole de benzènesulfonyl-1 méthoxy-5 indole et de 0,15 mole d'agent de stabilisation refroidie à -70°C, on ajoute cette solution en quelques minutes en maintenant la température du milieu inférieure on égale à -70°C.

On obtient ainsi une solution de benzènesulfonyl-1 lithio-2 méthoxy-5 indole en utilisant la tris(dioxa-3,6 heptyl)amine comme agent de stabilisation.

b) (Benzènesulfonyl-1 méthoxy-5 indolyl-2) (diéthylaminocarbonyl-3 méthoxy-2 pyridyl-4)méthanol.

Ce composé a été obtenu selon la méthode décrite dans l'Exemple 14c.

Réactif à caractère électrophile:
diéthylaminocarbonyl-3 formyl-4 méthoxy-2 pyridine
Rendement: 68%

En suivant le même procédé que décrit ci-dessus, on a obtenu les composés suivants:

1) (Tertiobutyloxycarbonyl-1 aza-5 indolyl-2) (diéthylaminocarbonyl-3 méthoxy-2 pyridyl-4)méthanol.

A partir de tertiobutyloxycarbonyl-1 lithio-2 aza-5 indole et de diéthylaminocarbonyl-3 formyl-4 méthoxy-2 pyridine comme réactif à caractère électrophile, préparé selon la paragraphe a) (agent de stabilisation: tétraméthyléthylènediamine).
Rendement: 64%

2) (Benzènesulfonyl-1 aza-5 indolyl-2)-1 (diisopropylaminocarbonyl-3 méthoxy-2 pyridyl-4)-1 éthanol.

A partir de benzènesulfonyl-1 lithio-2 aza-5 indole et d'acétyl-4 diisopropylaminocarbonyl-3 méthoxy-2 pyridine comme réactif à caractère électrophile, préparé selon le paragraphe a) (agent de stabilisation: tétraméthyléthylènediamine).
Rendement: 37%

EXEMPLE 17

Préparation du (benzènesulfonyl-1 aza-5 indolyl-2) (diéthylaminocarbonyl-3 méthoxy-2 pyridyl-4)cétone.

Dans une solution de 22,7 g (0,046 mole) de benzènesulfonyl-1 aza-5 indolyl-2) (diéthylamino-carbonyl-3 méthoxy-2 pyridyl-4)méthanol dans 400 ml de dichlorométhane, on met en suspension 40 g (10 équivalents) de bioxyde de manganèse et on chauffe la suspension à reflux pendant 1 à 2 heures. Après refroidissement, on filtre la suspension et on lave le solide avec 4 fois 50 ml de dichlorométhane. On évapore le filtrat à sec sous pression réduite pour obtenir un solide jaune qui, lavé à l'éther éthylique, fournit un solide crème.

De cette manière, on obtient 19,7 g de (benzènesulfonyl-1 aza-5 indolyl-2) (diéthylamino-carbonyl-3 méthoxy-2 pyridyl-4)cétone.
Rendement: 85%
P.F.: 150°C

Analyse centésimale %:

| | C | H | N | S |
|---|---|---|---|---|
| Calculé | 60,96 | 4,91 | 11,37 | 6,51 |
| Trouvé | 60,87 | 4,90 | 11,42 | 6,69 |

Spectre I.R. (KBr):

| | |
|---|---|
| C-H et C-H aromatique | 3100-3000 $cm^{-1}$ (t,f) |
| C=O cétone | 1665 $cm^{-1}$ (F) |
| C=O amide | 1630 $cm^{-1}$ (F) |
| C-C aromatique | 1590-1560 $cm^{-1}$ (m) |

Spectre R.M.N. ($DMSOD_6$): 0,7-1,3 ppm (2t,6H); 2,7-3,5 ppm (2q,4H); 3,9 ppm (s,3H); 7,0-9,0 ppm (m,11H).

De la même manière que précédemment mais au départ de (benzènesulfonyl-1 méthoxy-5 indolyl-2) (diéthylaminocarbonyl-3 méthoxy-2 pyridyl-4)méthanol, on a préparé la (benzènesulfonyl-1 méthoxy-5 indolyl-2) (diéthylaminocarbonyl-3 méthoxy-2 pyridyl-4)cétone. Rendement: 89%

P.F.: 145°C

Analyse élémentaire %:

| | C | H | N | S |
|---|---|---|---|---|
| Calculé | 62,17 | 5,12 | 8,06 | 6,15 |
| Trouvé | 62,32 | 5,33 | 7,82 | 6,20 |

Spectre I.R. (KBr):

| | |
|---|---|
| C=O cétone | 1665 $cm^{-1}$ (F) |
| C=O amide | 1630 $cm^{-1}$ (F) |
| C-C aromatique | 1590-1560 $cm^{-1}$ (m) |

Spectre R.M.N. ($CDCl_3$) (rotamères):0,9-1,3 ppm (2t,6H); 3,0-3,8 ppm (2q,4H); 3,75 ppm (s,3H); 3,95 ppm (s,3H); 6,8-7,6 ppm (m,7H); 7,9-8,4 ppm (m,3H).

EXEMPLE 18

Préparation de (benzènesulfonyl-1 aza-5 indolyl-2)-1(diéthylaminocarbonyl-3 méthoxy-2 pyridyl-4)-1 éthanol.

A une solution de 9,9 g (0,020 mole) de benzènesulfonyl-1 aza-5 indolyl-2) (diéthylamino-carbonyl-3 méthoxy-2 pyridyl-4)cétone dans 100 ml de tétrahydrofuranne anhydre, refroidie à -20°C, on ajoute en 5 minutes 0,020 mole de méthyllithium (12 ml d'une solution 1,65 molaire dans l'éther éthylique).

Après 10 minutes d'agitation à -20°C, on hydrolyse avec une solution aqueuse de chlorure d'ammonium (1 g/150 ml). On filtre le précipité obtenu après addition de 50 ml d'éther éthylique et on dissout le solide visqueux obtenu dans l'éthanol. On élimine alors le solvant sous pression réduite à 50°C.

On obtient ainsi 9,26 g de benzènesulfonyl-1 aza-5 indolyl-2)-1(diéthylaminocarbonyl-3 méthoxy-2 pyridyl-4)-1 éthanol sous forme d'un solide blanc. Rendement: 91%

P.F.: 236°C

Analyse centésimale %:

| | C | H | N | S |
|---|---|---|---|---|
| Calculé | 61,40 | 5,55 | 11,02 | 6,30 |
| Trouvé | 59,56 | 5,49 | 10,55 | 6,33 |

Spectre I.R. ($CHCl_3$):

| | |
|---|---|
| OH | 3500-3100 $cm^{-1}$ (m,l) |
| C=O | 1600-1620 $cm^{-1}$ (F) |
| C-C aromatique | 1570 $cm^{-1}$ (m) |

Spectre R.M.N. (pyridine $D_5$):1,0-1,4 ppm (m,6H); 2,45 ppm (m,3H); 3,0-3,8 ppm (m,4H); 3,9 ppm (s,3H); 6,5-9,2 ppm (m,12H).

Le même composé peut être préparé selon le procédé utilisé précédemment conduit à température ambiante (20°C) avec le chlorure de méthyl-magnésium au lieu de méthyllithium.

EXEMPLE 19

Préparation du (benzènesulfonyl-1 aza-5 indolyl-2)-1(diéthylaminocarbonyl-3 méthoxy-2 pyridyl-4)-1 éthanol.

a) Benzènesulfonyl-1 chloromangano-2 aza-5 indole

On prépare une solution de 0,15 mole de tétraméthyl-2,2,6,6 pipéridure de lithium par addition de 0,15 mole de n-butyllithium à 0,15 mole de tétraméthyl-2,2,6,6 pipéridine dans 120 ml de tétrahydrofuranne à une température inférieure à -20°C. On ajoute alors cette solution, en quelques minutes, à 0,1 mole de

benzènesulfonyl-1 aza-5 indole refroidie à -70°C en maintenant cette température durant l'addition. A la solution de benzènesulfonyl-1 lithio-2 aza-5 indole, on ajoute alors 0,15 mole de chlorure manganeux anhydre. On porte alors la température du milieu de -65°C à -25°C pour compléter la réaction d'échange et obtenir une solution du dérivé manganeux souhaité que l'on utilise telle quelle.

b) (Benzènesulfonyl-1 aza-5 indolyl-2)-1 (diéthylaminocarbonyl-3 méthoxy-2 pyridyl-4)-1 éthanol

Ce composé a été obtenu selon la méthode décrite dans l'Exemple 14c.
Réactif à caractère électrophile:
acétyl-4 diéthylaminocarbonyl-3 méthoxy-2 pyridine
Rendement: 45 à 50%

De la même manière que décrite précédemment mais au départ de benzènesulfonyl-1 acétyl-2 aza-5 indole et de diisopropylaminocarbonyl-3 bromomagnésio-4 méthoxy-2 pyridine, obtenue à partir de diisopropylaminocarbonyl-3 lithio-4 méthoxy-2 pyridine et de bromure de magnésium, on a préparé le (benzènesulfonyl-1 aza-5 indolyl-2)-1 (diisopropyl-aminocarbonyl-3 méthoxy-2 pyridyl-4)-1 éthanol.
Rendement: 30%

EXEMPLE 20

Préparation du (benzènesulfonyl-1 aza-5 indolyl-2) (diéthylaminocarbonyl-3 méthoxy-2 pyridyl-4)cétone.

a) Diéthylaminocarbonyl-3 méthoxy-2 (N-méthoxy N-méthylaminocarbonyl)-4 pyridine.

On met en solution 6,3 g (0,025 mole) d'acide diéthylaminocarbonyl-3 méthoxy-2 pyridyl-4 carboxylique, 17.42 ml (0,125 mole) de triéthylamine, 3,66 g (0,037 mole ) de chlorhydrate de N-O- diméthylhydroxylamine et 16.6 g (0,037 mole) d'hexafluorophosphate de benzotriazolyl-1 tris-diméthylaminophosphonium dans 250 ml de dichloro-méthane et on laisse sous agitation à 20°C pendant 5 heures. On ajoute alors 375 ml d'une solution saturée de chlorure de sodium et on extrait avec 3 fois 250 ml d'acétate d'éthyle. On évapore le solvant sous pression réduite et on purifie le résidu par chromatographie sur silice.

De cette manière, on obtient 4 g de diéthylaminocarbonyl-3 méthoxy-2 (N-méthoxy N-méthylaminocarbonyl)-4 pyridine sous forme d'un solide pâteux.
Rendement: 54%

Spectre I.R. (film):

| | |
|---|---|
| C=O | 1660-1610 $cm^{-1}$ (F,l) |
| C-C aromatique | 1590, 1570 $cm^{-1}$ (F) |

Spectre R.M.N. ($CDCl_3$):0,9-1,4 ppm (2t,6H); 2,7-3,6 ppm (m,4H); 3,2 ppm (s,3H); 3,6 ppm (s,3H); 3,9 ppm (s.3H); 6,8 ppm (d,1H); 8,1 ppm (d,1H).

b) (Benzènesulfonyl-1 aza-5 indolyl-2) (diéthylaminocarbonyl-3 méthoxy-2 pyridyl-4)cétone.

Ce composé a été préparé selon la méthode décrite dans l'Exemple 14c à partir du benzènesulfonyl-1 lithio-2 aza-5 indole et de diéthylaminocarbonyl-3 méthoxy-2 (N-méthoxy N-méthylaminocarbonyl)-4 pyridine.
Rendement: 30%

EXEMPLE 21

Préparation de la (méthoxy-5 indolyl-2) (diéthylaminocarbonyl-3 méthoxy-2 pyridyl-4)cétone.

On met en solution 26,1 g (0,05 mole) de (benzènesulfonyl-1 méthoxy-5 indolyl-2) (diéthylaminocarbonyl-3 méthoxy-2 pyridyl-4)cétone dans 400 ml de méthanol et on ajoute une solution de 27,64 g (0,2 mole) de carbonate de potassium dans 200 ml d'eau. On chauffe le mélange à 65°C pendant une heure. Après refroidissement, on élimine le méthanol sous pression réduite et on extrait la phase aqueuse avec deux fois 300 ml de dichlorométhane. On sèche la phase organique sur sulfate de sodium et on élimine les solvants sous pression réduite pour obtenir 19,6 g de produit brut que l'on purifie par chromatographie.

De cette manière, on obtient la (méthoxy-5 indolyl-2) (diéthylaminocarbonyl-3 méthoxy-2 pyridyl-4)cétone avec un rendement de 95%.
P.F.: 104°C

Analyse élémentaire %:

| | C | H | N |
|---|---|---|---|
| Calculé | 66,13 | 6,08 | 11,02 |
| Trouvé | 66,08 | 6,30 | 10,78 |

**Spectre I.R. (KBr):**

| | |
|---|---|
| NH | 3300 $cm^{-1}$ (large) |
| C=O cétone + amide | 1630 $cm^{-1}$ (F, large) |
| C-C aromatique | 1590-1560 $cm^{-1}$ (m) |

Spectre R.M.N. ($CDCl_3$):1,15 ppm (t,6H); 3,0-3,7 ppm (m,4H); 3,7 ppm (s,3H); 4,0 ppm (s,3H); 6,7-7,4 ppm (m,5H); 8,25 ppm (d,1H); 10 ppm (s,1H).

De la même manière que précédemment, mais au départ de (benzènesulfonyl-1 aza-5 indolyl-2) (diéthyl-aminocarbonyl-3 méthoxy-2 pyridyl-4)cétone, on a préparé l'(aza-5 indolyl-2) (diéthylaminocarbonyl-3 méthoxy-2 pyridyl-4)cétone.

Rendement: 70% après purification par chromatographie.

P.F.: 169°C.

**Analyse élémentaire %:**

| | C | H | N |
|---|---|---|---|
| Calculé | 64,76 | 5,72 | 15,90 |
| Trouvé | 64,36 | 5,87 | 15,58 |

Spectre I.R. (KBr):

| | |
|---|---|
| N-H | 3300-2700 $cm^{-1}$ (large) |
| C=O cétone + amide | 1630 $cm^{-1}$ (F, large) |
| C=C aromatique | 1600-1550 $cm^{-1}$ (m) |

Spectre R.M.N. ($DMSOD_6$):1,0 ppm (2t,6H); 3,0-3,5 ppm (2q,4H); 3,9 ppm (s,3H); 7,0-9,0 ppm (m,6H); 10,5 ppm (s,1H).

EXEMPLE 22

Préparation de l'aza-6 méthoxy-7 méthyl-3 (benzènesulfonyl-1 aza-5 indolyl-2)-3 phtalide (formule VIII: X=N, $R=R_1=CH_3$, P'=benzènesulfonyle)

On met en solution 114 g (0,233 mole) de (benzènesulfonyl-1 aza-5 indolyl-2) (diéthylamino-carbonyl-3 méthoxy-2 pyridyl-4)cétone dans 1,21 de tétrahydrofuranne anhydre et on ajoute, en 15 minutes environ, 19,1 g (0,256 mole) (86,8 ml d'une solution 2,95M dans le tétrahydrofuranne) de chlorure de méthylmagnésium en maintenant la température inférieure ou égale à 25°C.

Après agitation totale pendant 1 heure, on ajoute 146 ml d'acide acétique glacial et on porte le mélange à reflux pendant 1,5 heure. On élimine les solvants sous pression réduite et on obtient une poudre beige que l'on agite vigoureusement avec 460 ml d'éther éthylique.

Après essorage, on obtient 96 g d'aza-6 méthoxy-7 méthyl-3 (benzènesulfonyl-1 aza-5 indolyl-2)-3 phtalide sous forme d'un solide blanchâtre. Rendement:95%

P.F.: 200°C

**Analyse élémentaire %:**

| | C | H | N | S |
|---|---|---|---|---|
| Calculé | 60,68 | 3,93 | 9,65 | 7,36 |
| Trouvé | 60,42 | 3,96 | 9,54 | 7,10 |

Spectre I.R. (KBr):

| | |
|---|---|
| C-H aromatique | 3100 $cm^{-1}$ (t,f) |
| C=O | 1780 $cm^{-1}$ (F) |
| C-C aromatique | 1600 $cm^{-1}$ (F) |

Spectre R.M.N. ($CDCl_3$):2,2 ppm (s,3H); 4,15 ppm (s,3H); 7,0-9,0 ppm (m,11H).

De la même manière que précédemment on a préparé les composés suivants:

a) A partir de (benzènesulfonyl-1 méthoxy-5 indolyl-2) (diéthylaminocarbonyl-3 méthoxy-2 pyridyl-4)cétone, l'aza-6 méthoxy-7 méthyl-3 (benzènesulfonyl-1 méthoxy-5 indolyl-2)-3 phtalide.
Rendement: 95%
P.F.: 145°C

Spectre I.R. (KBr):

| | |
|---|---|
| C=O lactone | 1780 $cm^{-1}$ (F) |
| C-C aromatique | 1620-1590 $cm^{-1}$ (m) |

Spectre R.M.N. ($CDCl_3$):2,1 ppm (s,3H); 3,75 ppm (s,3H); 4,1 ppm (s,3H); 6,6-7,5 ppm (m,7H); 7,7-8,0 ppm (m,2H); 8,15 ppm (d,1H); 8,35 ppm (d,1H).

b) A partir de (méthoxy-5 indolyl-2) (diéthylaminocarbonyl-3 méthoxy-2 pyridyl-4)cétone et de 2,5 équivalents de chlorure de méthyl/magnésium l'aza-6 (méthoxy-5 indolyl-2)-3 méthoxy-7 méthyl-3 phtalide.
Rendement: 93%
P.F.: 203°C

Spectre I.R. (KBr):

| | |
|---|---|
| NH | 3360 $cm^{-1}$ (large) |
| C=O | 1765 $cm^{-1}$ (F) |
| C-C aromatique | 1615-1580 $cm^{-1}$ (m) |

Spectre R.M.N. ($DMSOD_6$): 2,1 ppm (s,3H); 3,7 ppm (s,3H); 4,1 ppm (s,3H); 6,4-7,4 ppm (m,5H); 8,45 ppm (d,1H); 10,5 ppm (s,1H).

EXEMPLE 23

Préparation de l'aza-6 méthoxy-7 (aza-5 benzènesulfonyl-1 indolyl-2)-3 phtalide (formule VIII: $R=CH_3$, $R_1=H$, X=N, P'=benzènesulfonyle)

On met en solution 14,8 g (0,03 mole) de (benzènesulfonyl-1 aza-5 indolyl-2) (diéthylaminocarbonyl-3 méthoxy-2 pyridyl-4)méthanol dans 233 ml d'éthanol et 17,2 ml (0,3 mole) d'acide acétique glacial puis on porte le mélange à reflux pendant 1,5 heure. On élimine les solvants sous pression réduite et on obtient une poudre que l'on agite vigoureusement avec 200 ml d'éther éthylique.

Après essorage, on obtient 11,16 g d'aza-6 méthoxy-7(benzènesulfonyl-1 aza-5 indolyl-2)-3 phtalide sous forme d'un solide.
Rendement: 88%
P.F.: 177°C

Analyse élémentaire %:

| | C | H | N |
|---|---|---|---|
| Calculé | 59,85 | 3,59 | 9,97 |
| Trouvé | 59,47 | 3,56 | 9,70 |

Spectre I.R. (KBr):

| | |
|---|---|
| C=O | 1775 $cm^{-1}$ (F) |
| C-C aromatique | 1600 $cm^{-1}$ (F) |
| $SO_2$-N | 1375 $cm^{-1}$ (F) |
| $SO_2$ | 1325 $cm^{-1}$ (F) |

Spectre R.M.N. ($CDCl_3$):4,1 ppm (s,3H); 6,55 ppm (s,1H); 7,20-8,80 ppm (m,10H).

De la même manière que précédemment à partir de (benzènesulfonyl-1 aza-5 indolyl-2)-1(diéthylaminocarbonyl-3 méthoxy-2 pyridyl-4)-1 éthanol, on a obtenu l'aza-6 méthoxy-7 méthyl-3 (benzènesulfonyl-1 aza-5 indolyl-2)-3 phtalide.

EXEMPLE 24

Préparation de l'aza-6 dihydro-1,1 hydroxy-1 méthoxy-7 méthyl-3 (benzènesulfonyl-1 aza-5 indolyl-2)-3 phtalide (formule IX: X=N, $R=R_1=CH_3$, $R'_1=H$ et P'=benzènesulfonyle)

On met en solution 48 g (0,11 mole) d'aza-6 méthoxy-7 méthyl-3 (benzènesulfonyl-1 aza-5 indolyl-2)-3 phtalide dans 550 ml de dichlorométhane anhydre et on refroidit le mélange à -25°C. On ajoute alors 117,5 ml (0,176 mole) d'hydrure de diisobutylaluminium (solution à 25% dans le toluène) en 10 à 15 minutes de manière à maintenir la température du milieu réactionnel entre -25°C et -20°C. Après 30 minutes d'agitation à cette température, on détruit l'excès d'hydrure de diisobutylaluminium par addition d'acétone en excès et on verse le mélange dans une solution froide (température de la glace) d'acide acétique à 20%. On extrait la phase aqueuse avec 300 ml de dichlorométhane, on sèche sur sulfate de sodium les phases organiques réunies et on élimine le solvant sous pression réduite pour obtenir 48,2 g d'un résidu. On met ce résidu en suspension dans 480 ml d'éther diisopropylique, on agite pendant 30 minutes et on essore.

On obtient ainsi 46,6 g d'aza-6 dihydro-1,1 hydroxy-1 méthoxy-7 méthyl-3 (benzènesulfonyl-1 aza-5 indolyl-2)-3 phtalide sous forme d'une poudre beige.
Rendement: 97%
P.F.: décomposition vers 250°C

Analyse élémentaire %:

| | C | H | N | S |
|---|---|---|---|---|
| Calculé | 60,40 | 4,38 | 9,61 | 7,33 |
| Trouvé | 60,23 | 4,37 | 9,52 | 7,46 |

Spectre I.R. (KBr):

| | |
|---|---|
| OH | 3200-2700 $cm^{-1}$ (m,l) |
| C-C aromatique | 1610 $cm^{-1}$ (F) |
| C-O-C | 1175 $cm^{-1}$ (F) |

Spectre R.M.N. ($DMSOD_6$):2,10 ppm (s,3H); 4,0 ppm (s,3H); 6,4-9,0 ppm (m,13H).

De la même manière que précédemment à partir d'aza-6 méthoxy-7 méthyl-3 (benzènesulfonyl-1 méthoxy-5 indolyl-2)-3 phtalide, on a obtenu l'aza-6 dihydro-1, 1 hydroxy-1 méthoxy-7 méthyl-3 (benzènesulfonyl-1 méthoxy-5 indolyl-2)-3 phtalide.
Rendement: 95%
P.F.: 170°C

Spectre I.R. (KBr):

| | |
|---|---|
| OH | 3600-3300 $cm^{-1}$ (large) |
| C-C aromatique | 1620-1580 $cm^{-1}$ (m) |

Spectre R.M.N. ($CDCl_3$) (rotamères):2,05 et 2,15 ppm (2s,3H); 3,70 et 3,75 ppm (2s,3H); 4,0 et 4,05 ppm (2s,3H); 6,4 et 8,3 ppm (m,13H).

EXEMPLE 25

Préparation de l'aza-6 dihydro-1,1 hydroxy-1 méthoxy-7 (benzènesulfonyl-1 aza-5 indolyl-2)-3 phtalide (formule IX: X=N, $R_1=R'_1=H$, $R=CH_3$, P'=benzènesulfonyle)

On met en solution 46,3 g (0,11 mole) d'aza-6 méthoxy-7 (benzènesulfonyl-1 aza-5 indolyl-2)-3 phtalide dans 550 ml de dichlorométhane anhydre et on redroidit le mélange à -25°C. On ajoute alors 161,5 ml (0,242 mole) d'hydrure de diisobutylaluminium en 10 à 15 minutes de manière à maintenir la température du milieu réactionnel inférieure ou égale à -70°C. Après 30 minutes d'agitation à cette température, on détruit l'excès d'hydrure de diisobutylaluminium par addition d'acétone en excès et on verse le mélange dans une solution glacée d'acide acétique à 20%. On extrait la phase aqueuse avec 300 ml de dichlorométhane, on réunit les phases organiques et on les sèche sur sulfate de sodium. On élimine le solvant sous pression réduite et on met le résidu obtenu en suspension dans 480 ml d'éther diisopropylique. On agite pendant 30 minutes et on essore.

On obtient ainsi l'aza-6 dihydro-1, 1 hydroxy-1 méthoxy-7 (aza-5 benzènesulfonyl-1 indolyl-2)-3 phtalide.

Rendement: 86%
P.F.: 213°C

Spectre I.R. (KBr):

| | |
|---|---|
| OH | 3200-2700 $cm^{-1}$ |
| C=O | 1715 $cm^{-1}$ |
| C-C aromatique | 1595 $cm^{-1}$ |
| C-O-C | 1165 $cm^{-1}$ |

Spectre R.M.N. ($DMSOD_6/CF_3CO_2H$; 9/1):4,0 ppm (s,3H); 6,5-9,5 ppm (m,13H).

EXEMPLE 26

Préparation de l'aza-6 dihydro-1,1 diméthyl-1,3 hydroxy-1 méthoxy-7 (benzènesulfonyl-1 aza-5 indolyl-2)-3 phtalide (formule IX: X=N, $R=R_1=R'_1=CH_3$, P'=benzènesulfonyle)

On met en solution 48 g (0,11 mole) d'aza-6 méthoxy-7 méthyl-3 (benzènesulfonyl-1 aza-5 indolyl-2)-3 phtalide dans 550 ml de tétrahydrofuranne anhydre et on refroidit le mélange à -40°C. On ajoute alors 18,1 g (0.242 mole) de chlorure de méthylmagnésium en 10 à 15 minutes de manière à maintenir la température du milieu réactionnel à -40°C. On agite tout en laissant la température remonter jusqu'à 0°C puis on détruit l'excès de chlorure de méthylmagnésium par addition d'acétone en excès et on verse le mélange dans une solution glacée d'acide acétique à 20%. On extrait la phase aqueuse avec 300 ml de dichlorométhane, on réunit les phases organiques et on les sèche sur sulfate de sodium. On élimine le solvant sous pression réduite et on met le résidu obtenu en suspension dans 480 ml d'éther diisopropylique. On agite pendant 30 minutes et on essore.

On obtient ainsi l'aza-6 dihydro-1,1 diméthyl-1,3 hydroxy-1 méthoxy-7 (benzènesulfonyl-1 aza-5 indolyl-2)-3 phtalide.
Rendement: 85%
P.F.: environ 195°C (décomposition)

Spectre I.R. (KBr):

| | |
|---|---|
| OH | 3600-3000 $cm^{-1}$ (m,l) |
| C-C aromatique | 1610 $cm^{-1}$ (F) |
| C-O-C | 1180 $cm^{-1}$ (F) |
| $SO_2$-NC | (1370 $cm^{-1}$ (F) |
| | (1180 $cm^{-1}$ (F) |

Spectre R.M.N. ($DMSOD_6$):1,35, 1,80, 2,05 et 2,15 ppm (4s,6H); 3,0 et 3,5 ppm (m,1H); 3,95 ppm (s,3H); 6,3-8,8 ppm (m,11H).

De la même manière que précédemment, on a obtenu les composés suivants:

a) A partir d'aza-6 méthoxy-7 méthyl-3 (benzènesulfonyl-1 méthoxy-5 indolyl-2)-3 phtalide et de 2 équivalents de chlorure de méthyl magnésium, la réaction ayant lieu à la température de -30°C à +20°C, l'aza-6 dihydro-1,1 diméthyl-1,3 hydroxy-1 méthoxy-7 (benzènesulfonyl-1 méthoxy-5 indolyl-2)-3 phtalide.
Rendement: 85%
P.F.: 158°C

Spectre I.R. (KBr):

| | |
|---|---|
| OH | 3540-3460 $cm^{-1}$ (large) |
| C-C aromatique | 1620-1580 $cm^{-1}$ (m) |

Spectre R.M.N. ($CDCl_3$):1,9-2,2 ppm (m,6H); 3,2-3,6 ppm (m,1H); 3,65 ppm (s,3H); 4,0 ppm (s,3H); 6,2-8,3 ppm (m,11H).

b) A partir d'aza-6 (méthoxy-5 indolyl-2)-3 méthyl-3 méthoxy-7 phtalide et de 3 équivalents de chlorure de méthyl magnésium, la réaction ayant lieu à la température de -40°C à +20°C, l'aza-6 dihydro-1,1 (méthoxy-5 indolyl-2)-3 méthoxy-7 phtalide.
Rendement: 80%
P.F.: environ 86°C

Spectre I.R. (KBr):

| | |
|---|---|
| N-H | 3500-3300 $cm^{-1}$ (large) |
| C-C aromatique | 1630-1580 $cm^{-1}$ (m) |

Spectre R.M.N. ($DMSOD_6$):1,8-2,0 ppm (m,6H); 3,7 ppm (s,3H); 6,2-7,4 ppm (m,6H); 8,1 ppm (dd,1H); 10,4 et 10,6 ppm (2s,1H).

EXEMPLE 27

Préparation de l'aza-6 dihydro-1,1 hydroxy-1 méthoxy-7 (aza-5 indolyl-2)-3 phtalide (formule IX: X=N, $R_1=R'_1=P'=H$, $R=CH_3$)

a) Aza-6 dihydro-1, 1 diméthoxy-1,7 (benzènesulfonyl-1 aza-5 indolyl-2)-3 phtalide.

On met en suspension 14,24 g (0,036 mole) d'aza-6 dihydro-1,1 hydroxy-1 méthoxy-7 (benzènesulfonyl-1 aza-5 indolyl-2)-3 phtalide dans 276 ml de méthanol anhydre puis on ajoute 7,8 ml (0,063 mole) d'éthérate de trifluorure de bore. On laisse alors la solution homogène obtenue sous agitation pendant 4 heures. On verse lentement le milieu réactionnel dans une solution aqueuse froide de bicarbonate de sodium (32 g/900 ml d'eau glacée). On essore le précipité formé et on le sèche sous pression réduite à 50°C.

On obtient ainsi 12,6 g d'aza-6 dihydro-1,1 diméthoxy-1,7 (benzènesulfonyl-1 aza-5 indolyl-2)-3 phtalide sous forme d'une poudre blanchâtre.

Rendement: 80%

P.F.: 110°C (fusion pâteuse)

Spectre I.R. (KBr):

| | |
|---|---|
| C-C aromatique | 1605, 1595 $cm^{-1}$ (F) |
| $SO_2$-N | (1375 $cm^{-1}$ (F) |
| | (1170 $cm^{-1}$ (F) |

Spectre R.M.N. ($DMSOD_6$):3,3 et 3,5 ppm (2s,3H); 4,0 ppm (s,3H); 6,3-9,0 ppm (m,13H).

De la même manière que précédemment mais au départ d'aza-6 dihydro-1,1 diméthyl-1,3 hydroxy-1 méthoxy-7 (benzènesulfonyl-1 aza-5 indolyl-2)-3 phtalide, on a préparé l'aza-6 dihydro-1,1 diméthoxy-1,7 diméthyl-1,3 (benzènesulfonyl-1 aza-5 indolyl-2)-3 phtalide.

Rendement: 69%

P.F.: 218°C

Spectre I.R.:

| | |
|---|---|
| C-C aromatique | 1605, 1590 $cm^{-1}$ (F) |
| C-O-C | 1180 $cm^{-1}$ (F) |
| $SO_2N$ | 1170 $cm^{-1}$ (F) |

Spectre R.M.N. ($CDCl_3/DMSOD_6$: 2/1) (mélange d'énantiomères):1,8, 2,10, 2,15 et 2,20 ppm (4s,6H); 3,15 et 3,30 ppm (2s,3H); 4,0 ppm (s,3H); 6,4-8,8 ppm (m,11H).

b) Aza-6 dihydro-1,1 diméthoxy-1,7 (aza-5 indolyl-2)-3 phtalide.

A une solution de 10,06 g (0,023 mole) d'aza-6 dihydro-1,1 diméthoxy-1,7 (benzènesulfonyl-1 aza-5 indolyl-2)-3 phtalide dans 207 ml de méthanol, on ajoute, en quelques minutes, une solution aqueuse de 22,25 g (0,161 mole) de bicarbonate de potassium dans 86 ml d'eau. On agite, à 20°C pendant 4 heures, la suspension obtenue pour fournir une solution limpide. On concentre la solution sous pression réduite et on reprend le résidu dans 144 ml de dichlorométhane et 58 ml d'eau. On filtre rapidement la phase organique sur silice, on rince au méthanol et on concentre le filtrat sous pression réduite.

De cette manière, on obtient 5,41 g d'aza-6 dihydro-1,1 diméthoxy-1,7 (aza-5 indolyl-2)-3 phtalide sous forme d'un solide beige.

Rendement: 79%

P.F.: environ 135°C (décomposition)

Spectre I.R. (KBr):

| | |
|---|---|
| N-H | 3400-3300 $cm^{-1}$ (m) |
| C-C aromatique | 1610, 1595 $cm^{-1}$ (F) |
| C-O-C | 1080 $cm^{-1}$ (F) |

Spectre R.M.N. ($CDCl_3$) (mélange d'énantiomères):3,5 et 3,7 ppm (2s,3H); 3,85 et 4,0 ppm (2s,3H); 6,0-7,4 ppm (m,5H); 8,0-8,20 ppm (m,2H); 8,8 ppm (s,1M); 9,8-10,5 ppm (m,1H).

c) Aza-6 dihydro-1,1 hydroxy-1 méthoxy-7 (aza-5 indolyl-2)-3 phtalide

On met en solution 3 g (0,010 mole) d'aza-6 dihydro-1,1 diméthoxy-1,7 (aza-5 indolyl-2)-3 phtalide dans 100 ml d'acétone et 1 ml d'eau puis on ajoute 5 ml (0,041 mole) d'éthérate de trifluorure de bore. Après 48 heures

sous agitation à 20° C, on neutralise le mélange avec une solution aqueuse de bicarbonate de sodium (6,88 g/7 ml d'eau). Après évaporation des solvants sous pression réduite, on reprend le résidu dans un mélange dichlorométhane/eau (1/1). On essore le précipité obtenu et on le sèche sous vide partiel.

De cette manière, on obtient 2,20 g d'aza-6 dihydro-1,1 hydroxy-1 méthoxy-7 (aza-5 indolyl-2)-3 phtalide sous forme d'un solide jaune.
Rendement: 78%
P.F.: > 250° C (décomposition)

Spectre I.R. (KBr):

| | |
|---|---|
| OH | 3600-3100 $cm^{-1}$ (m,l) |
| C-C aromatique | 1605, 1580 $cm^{-1}$ (F) |
| C-O-C | 1080 $cm^{-1}$ (F) |

Spectre R.M.N. ($DMSOD_6$):4,0 ppm (s,3H); 6,0-9,3 ppm (m,10H).

EXEMPLE 28

Préparation de l'aza-6 dihydro-1,1 hydroxy-1 méthoxy-7 méthyl-3 (aza-5 indolyl-2)-3 phtalide (formule IX: X=N; $R=R_1=CH_3$, $P'=R'_1=H$).

a) Aza-6 dihydro-1, 1 diméthoxy-1,7 méthyl-3 (aza-5 benzènesulfonyl-1 indolyl-2)-3 phtalide.

Ce composé a été obtenu selon la procédure de l'Exemple 27 à partir de l'aza-6 dihydro-1,1 hydroxy-1 méthoxy-7 méthyl-3 (benzènesulfonyl-1 aza-5 indolyl-2)-3 phtalide.
Rendement: 78%
P.F.: 167° C (peu net).

Spectre I.R. (KBr):

| | |
|---|---|
| C-C aromatique | 1610, 1595 $cm^{-1}$ (F) |
| $SO_2$-N | 1380 $cm^{-1}$ |
| C-O-C | 1190 $cm^{-1}$ (F) |
| $SO_2$-N | 1175 $cm^{-1}$ (F) |

Spectre R.M.N. ($CDCl_3$):2,1 ppm (s,3H); 3,5 ppm (s,3H); 4,0 ppm (s,3H); 6,0-9,0 ppm (m,12H).

b) Aza-6 dihydro-1,1 diméthoxy-1,7 méthyl-3 (aza-5 indolyl-2)-3 phtalide.

A une solution de 10,3 g (0,023 mole) d'aza-6 dihydro-1,1 diméthoxy-1,7 benzènesulfonyl-1 aza-5 indolyl-2)-3 phtalide dans 207 ml de méthanol, on ajoute, en quelques minutes, une solution aqueuse de 0,092 mole de bicarbonate de potassium dans 86 ml d'eau. On agite la suspension à reflux durant 2 heures pour fournir une solution limpide.

On concentre la solution sous pression réduite et on reprend le résidu dans 144 ml de dichlorométhane et 58 ml d'eau. On filtre rapidement la phase organique sur silice, on rince au méthanol, on concentre le filtrat sous pression réduite et on purifie par chromatographie sur silice.

On obtient ainsi l'aza-6 dihydro-1,1 diméthoxy-1,7 méthyl-3 (aza-5 indolyl-2)-3 phtalide.
Rendement: 80%
P.F.: 121° C (peu net).

Analyse élémentaire :

| | C | H | N |
|---|---|---|---|
| Calculé | 65,58 | 5,50 | 13,50 |
| Trouvé | 63,85 | 5,47 | 12,95 |

Spectre I.R. (KBr):

| | |
|---|---|
| N-H | 3500-3300 $cm^{-1}$ (m,l) |
| C-C aromatique | 1610, 1595 $cm^{-1}$ (F) |
| C-O-C | 1070 $cm^{-1}$ (F) |

Spectre R.M.N. ($CDCl_3$) (mélange d'énantiomères):2,0 et 2,1 ppm (2s,3H); 3,5 et 3,7 ppm (2s,3H); 3,8 et 4,0

ppm (2s,3H); 6,0-9,0 ppm (m,7H); 9,5-10 ppm (m,1H).

c) Aza-6 dihydro-1,1 hydroxy-1 méthoxy-7 méthyl-3 (aza-5 indolyl-2)-3 phtalide.

On agite à 20°C pendant 3 heures, une solution de 3 g (0,009 mole) d'aza-6 dihydro-1, 1 diméthoxy-1,7 méthyl-3 (aza-5 indolyl-2)-3 phtalide dans 46 ml d'acide chlorhydrique 0,5M (0,015 mole) puis on neutralise le milieu avec 1,94 g de bicarbonate de sodium. On réduit la phase aqueuse à 10 ml par évaporation sous pression réduite et on refroidit dans la glace pour compléter la précipitation du produit. On essore le précipité et on le sèche par lavage à l'éther éthylique.

On obtient ainsi 2,6 g d'aza-6 dihydro-1,1 hydroxy-1 méthoxy-7 méthyl-3 (aza-5 indolyl-2)-3 phtalide sous forme d'un solide blanc.
Rendement: 90%
P.F.: environ 210°C (décomposition)

Spectre I.R. (KBr):

| | |
|---|---|
| NH, OH | 3600-3100 $cm^{-1}$ (m,l) |
| C-C aromatique | 1600-1580 $cm^{-1}$ (F) |
| C-O-C | 1080 $cm^{-1}$ (F) |

Spectre R.M.N. (pyridine $D_5$):2,3 ppm (s,3H); 4,1 ppm (s,3H); 5,5-6,5 ppm (m,2H); 7,0-9,5 ppm (m,7H).
Spectre R.M.N. ($DMSOD_6$):
1,9 ppm (s,3H); 4,1 ppm (s,3H); 5,5-6,5 ppm (m,2H); 6,5-9,0 ppm (m,7H).

EXEMPLE 29

Préparation de la dihydro-6,11 dihydroxy-6,11 méthoxy-10 méthyl-6 5H-pyrido[3',4':4,5]pyrrolo[2,3-g]isoquinoléine (formule XXVII: $R=R_1=CH_3$, $R'_1=H$).

A une solution de 41,12 g (0,094 mole) d'aza-6 dihydro-1,1 hydroxy-1 méthoxy-7 méthyl-3 (benzènesulfonyl-1 aza-5 indolyl-2)-3 phtalide dans 200 ml de méthanol refroidie à 0°C, on ajoute en 5 minutes, 47 ml (0,47 mole) d'hydroxyde de sodium 10N puis on agite la suspension pendant 2,5 heures à 20°C environ pour obtenir une solution homogène brune. Après une heure d'agitation supplémentaire, le produit attendu précipite. On complète alors la précipitation par refroidissement dans la glace pendant 1 heure. On essore le précipité et on le sèche sous vide partiel. On obtient ainsi 26,7 g de dihydro-6,11 dihydroxy-6,11 méthoxy-10 méthyl-6 5H pyrido[3',4':4,5]-pyrrolo[2,3-g]isoquinoléine sous forme d'une poudre blanc cassé.
Rendement: 86%
P.F.: environ 190°C

La R.M.N. du proton montre que le produit forme un solvate avec le méthanol dans le rapport constant de 1/1.

Spectre I.R. (KBr):

| | |
|---|---|
| NH ) | 3500-3400 $cm^{-1}$ (m) |
| OH ) | 3300-3100 $cm^{-1}$ (m) |
| C-C aromatique | 1620, 1595, 1570 $cm^{-1}$ (m) |

Spectre R.M.N. (pyridine $D_5$):
2,1 ppm (s,3H); 3,35 ppm (s,3H); 4,1 ppm (s,3H); 4,8-5,5 ppm (1m,4H); 6,2 ppm (s,1H); 7,5-7,9 ppm (2d,3H); 8,3-8,7 ppm (2d,2H); 9,4 ppm (s,1H).

EXEMPLE 30

Préparation de la dihydro-6,11 dihydroxy-6,11 diméthyl-6,11 méthoxy-10 5H pyrido[3',4':4,5]-pyrrolo-[2,3-g]isoquinoléine (formule XXVII: $R=R_1=R'_1=CH_3$).

A une solution de 42,24 g (0,094 mole) d'aza-6 dihydro-1,1 diméthyl-1,3 hydroxy-1 méthoxy-7 (benzènesulfonyl-1 aza-5 indolyl-2)-3 phtalide dans 200 ml de méthanol refroidie à 0°C, on ajoute en 5 minutes, 47 ml (0,47 mole) d'hydroxyde de sodium 10N puis on agite la suspension pendant 8 heures à 20°C ce qui provoque la précipitation du produit attendu. On complète alors la précipitation par refroidissement dans la glace pendant 1 heure. On essore le précipité et on le sèche sous vide partiel. On purifie alors le produit par chromatographie sur silice.

De cette manière, on obtient la dihydro-6,11 dihydroxy-6,11 diméthyl-6,11 méthoxy-10 5H-pyrido[3',4':4,5]pyrrolo[2,3-g]isoquinoléine.
Rendement: 73%
P.F.: > 250°C

Spectre I.R. (KBr):

| | |
|---|---|
| OH, NH | 3700-3000 $cm^{-1}$ (m,l) |
| C-C aromatique | 1600 $cm^{-1}$ (F,l) |

Spectre R.M.N. (pyridine $D_5$): mélange d'énantiomères1,8-2,3 ppm (m,6H); 3,9-4,0 ppm (m,3H); 6,5-9,2 ppm (m,8H).

EXEMPLE 31

Préparation de la dihydro-6,11 dihydroxy-6,11 méthoxy-10 5H-pyrido[3',4':4,5]pyrrolo[2,3-g]iso-quinoléine (formule XXVII: $R=CH_3$, $R_1=R'_1=H$).

A une solution de 26,6 g (0,094 mole) d'aza-6 dihydro-1,1 hydroxy-1 méthoxy-7 (aza-5 indolyl-2)-3 phtalide dans 200 ml de méthanol refroidie à 0°C, on ajoute en 5 minutes, 10 ml (0,10 mole) d'hydroxyde de sodium 10N puis on agite la suspension pendant 3,5 heures à 20°C ce qui provoque la précipitation du produit attendu. On complète alors la précipitation par refroidissement dans la glace pendant 1 heure. On essore le précipité et on le sèche sous vide partiel.

On obtient ainsi la dihydro-6,11 dihydroxy-6,11 méthoxy-10 5H-pyrido[3',4':4,5]pyrrolo[2,3-g]isoquinoléine.
Rendement: 41%
P.F.: > 260°C (décomposition)

Spectre I.R. (KBr):

| | |
|---|---|
| OH, NH | 3600-3000 $cm^{-1}$ (m,l) |
| C-C aromatique | 1665-1580 $cm^{-1}$ (F) |

Spectre R.M.N. ($DMSOD_6$ + pyridine $D_5$: 1/1):4,1 ppm (s,3H); 7,0-9,3 ppm (m,10H).

EXEMPLE 32

Préparation de la dihydro-6,11 dihydroxy-6,11 méthoxy-10 méthyl-6 5H-pyrido[3',4':4,5]pyrrolo[2,3-g]isoquinoléine (formule XXVII: $R=R_1=CH_3$, $R'_1=H$)

On a utilisé la même procédure qu'à l'Exemple 29 à partir de l'aza-6 dihydro-1,1 hydroxy-1 méthoxy-7 méthyl-3 (aza-5 indolyl-2)-3 phtalide et de 1 équivalent d'hydroxyde de sodium 10N.
Rendement: 78,5%

EXEMPLE 33

Préparation de la méthoxy-10 méthyl-6 5H-pyrido[3',4':4,5]pyrrolo[2,3-g] isoquinoléine (formule X: $R=R_1=CH_3$, $R'_1=H$, X=N).

On met en solution 12,02 g (0,036 mole de dihydro-6,11 dihydroxy-6,11 méthoxy-10 méthyl-6 5H-pyrido[3',4':4,5]pyrrolo[2,3-g]isoquinoléine et 3,45 g (0,092 mole) de borohydrure de sodium dans 97 ml de diméthyléther de diéthylèneglycol déperoxydé et on chauffe à 120-130°C pendant 20 à 30 minutes. On refroidit le mélange dans un bain de glace et on additionne 27 ml d'acétone pour détruire l'excès de borohydrure de sodium. On introduit alors 600 ml d'acide chlorhydrique 2N en 15 minutes à température inférieure ou égale à 10°C et on agite pendant 4 heures à température ambiante. On verse le mélange dans 275 ml d'une solution d'hydroxyde de sodium à 35% préalablement refroidie et on agite pendant une heure. On essore le précipité formé puis on le sèche sous vide partiel à 50°C.

De cette manière, on obtient 9,34 g de méthoxy-10 méthyl-6 5H-pyrido[3',4':4,5]pyrrolo[2,3-g]isoquinoléine sous forme d'une poudre blanc-verdâtre.
Rendement: 97%
P.F.: < 263°C

Analyse élémentaire % (correspondant à l'hémihydrate: 1/2 $H_2O$):

| | C | H | N |
|---|---|---|---|
| Calculé | 70,59 | 5,15 | 15,44 |
| Trouvé | 70,65 | 4,91 | 15,28 |

Spectre I.R. (KBr):

| | |
|---|---|
| NH | 3300-2500 $cm^{-1}$ (m,l) |
| C=N, C=C | 1640 $cm^{-1}$ (f) |
| | 1610 $cm^{-1}$ (F) |
| C-C aromatique | 1585 $cm^{-1}$ (F) |

Spectre R.M.N. (acide trifluoroacétique):3,2 ppm (s,1H); 4,90 ppm (s,3H); 8,0-8,5 ppm (m,3H); 8,6-9,2 ppm (m,1H); 9,4-10,1 ppm (m,2H).

De la même manière que précédemment mais au départ de dihydro-6,11 dihydroxy-6,11 5H-pyrido[3',4':4,5]pyrrolo[2,3-g]isoquinoléine, on a préparé la méthoxy-10 5H-pyrido[3',4':4,5]pyrrolo[2,3-g]isoquinoléine.
Rendement: 83%
Spectre R.M.N. (acide trifluoroacétique):
4,5 ppm (s,3H); 6,7-7,0 ppm (m,2H); 7,7-8,6 ppm (m,4H); 8,9 ppm (d,1H).

EXEMPLE 34

Préparation du diméthoxy-1,9 méthyl-5 6H-pyrido[4,3-b]carbazole (formule X: $R=R_1=CH_3$, $R'_1=H$, $X=C\text{-}OCH_3$).

A une suspension de 3,97 g (0,0085 mole) d'aza-6 dihydro-1,1 hydroxy-1 méthoxy-7 méthyl-3 (benzènesulfonyl-1 méthoxy-5 indolyl-2)-3 phtalide dans 20 ml de méthanol, on ajoute en 5 minutes, une solution de 4,77 g (0,085 mole) d'hydroxyde de potassium en pastille dans 10 ml de méthanol puis on chauffe à reflux pendant 1 heure.

On dilue le milieu réactionnel par addition de 70 ml de méthanol et on refroidit dans un bain de glace. On essore le précipité obtenu et on le sèche sous pression réduite pour obtenir 1,59 g de produit. On obtient un second jet de 0,277 g en évaporant à sec le filtrat précédent et en chromatographiant le résidu sur silice (solvant: acétate d'éthyle/hexane).

De cette manière, on isole 1,767 g de diméthoxy-1,9 méthyl-5 6H-pyrido[4,3-b]carbazole.
Rendement: 71%

Spectre I.R. (KBr):

| | |
|---|---|
| NH | 3400-3300 $cm^{-1}$ (large) |
| C-C et C=C | 1640-1590 $cm^{-1}$ (m) |

Spectre R.M.N. ($DMSOD_6$):2,8 ppm (s,3H(; 3,9 ppm (s,3H); 4,10 ppm (s,3H); 7,1 ppm (dd,1H, J=9 Hz, J'=2,5 Hz); 7,45 ppm (d,1H, J=9 Hz); 7,50 ppm (d,1H, J''=6,5 Hz); 7,9 ppm (d,1H, J''=6,5 Hz); 7,95 ppm (d,1H, J'=2,5 Hz); 8,9 ppm (s,1H); 11,15 ppm (s,1H).

EXEMPLE 35

Préparation de la méthoxy-10 méthyl-6 5H-pyrido[3',4';4,5]pyrrolo[2,3-g]isoquinoléine (formule X: $R=R_1=CH_3$, $R'_1=H$, $X=N$).

On place 10,937 g (0,025 mole) d'aza-6(benzènesulfonyl-1 aza-5 indolyl-2)-3 dihydro-1,1 hydroxy-1 méthoxy-7 méthyl-3 phtalide, 14,03 g d'hydroxyde de potassium en pastille dans 80 ml d'éthanol et on chauffe à reflux pendant 2 heures. On évapore le solvant à sec sous vide, on reprend le résidu avec 100 ml d'eau et on filtre la suspension obtenue. On reprend le solide formé avec 50 ml d'eau et on agite la suspension pendant 15 heures.

Après essorage et séchage sous vide, on obtient 5,576 g de méthoxy-10 méthyl-6 5H-pyrido[3',4':4,5]pyrrolo[2,3-g]isoquinoléine sous forme d'une poudre brune.
Rendement: 85%
Mêmes données analytiques qu'à l'Exemple 33.

EXEMPLE 36

Préparation du dihydro-5,11 diméthoxy-1,9 hydroxy-5 méthyl-5 méthylène-11 6H-pyrido[4,3-b]carbazole (formule XXVIII: $R=R_1=R_2=CH_3$, $R_{14}=H$).

On place 2,04 g (0,006 mole) d'aza-6 dihydro-1,1 diméthyl-1,3 hydroxy-1 méthoxy-7 (méthoxy-5 indolyl-2)-3 phtalide dans 9 ml de méthanol et 2,4 ml d'eau. On ajoute 18 ml d'hydroxyde de potassium (solution 5N dans le méthanol) et on chauffe 3 à 4 heures entre 40 et 45°C. Après refroidissement et ajout de 36 ml d'eau, on essore le précipité obtenu et on le sèche sous pression réduite.

On obtient ainsi 1,55 g de dihydro-5,11 diméthoxy-1,9 hydroxy-5 méthyl-5 méthylène-11 6H-pyrido[4,3-b]carbazole.
Rendement: 80%

P.F.: environ 208°C.

Spectre I.R. (KBr):

| | |
|---|---|
| OH, NH | 3460, 3360 $cm^{-1}$ |
| C=C exocyclique | 1615 $cm^{-1}$ |
| C-C aromatique | 1580 $cm^{-1}$ |

Spectre R.M.N. ($CD_3OD/DMSOD_6$ 2/1):1,65 ppm (s,3H); 3,80 ppm (s,3H); 4,0 ppm (s,3H); 6,0 ppm (d,1H); 6,45 ppm (d,1H); 6,8 ppm (dd,1H); 7,2-7,5 ppm (m,3H); 8,1 ppm (d,1H).

EXEMPLE 37

Préparation du diméthoxy-1,9 diméthyl-5,11 6H-pyrido[4,3-b]carbazole (formule X: $R=R_1=R'_1=CH_3$, $X=C\text{-}OCH_3$).

On met en solution 0,322 g (0,001 mole) de dihydro-5,11 diméthoxy-1,9 hydroxy-5 méthyl-5 méthylène-11 6H-pyrido[4,3-b]carbazole et 0,19 g (0,005 mole) de borohydrure de sodium dans 5 ml de diméthyléther de diéthylèneglycol déperoxydé et on chauffe à 120-130°C pendant 20 à 30 minutes. On refroidit le mélange dans un bain de glace et on additionne 1 ml d'acétone pour détruire l'excès de borohydrure de sodium. On introduit alors 15 ml d'acide chlorhydrique 2N en 15 minutes à température inférieure ou égale à 10°C et on agite pendant 4 heures à température ambiante. On verse le mélange dans 8 ml d'une solution d'hydroxyde de sodium à 35% préalablement refroidie et on agite pendant une heure. On essore le précipité formé puis on le sèche sous vide partiel à 50°C.

De cette manière, on obtient 0,254 g de diméthoxy-1,9 diméthyl-5,11 6H-pyrido[4,3-b]carbazole.
Rendement: 83%
P.F.: environ 190°C

Spectre I.R. (KBr):

| | |
|---|---|
| NH | 3380 $cm^{-1}$ |
| C-C et C=C | 1595 $cm^{-1}$ |

Spectre R.M.N. ($DMSOD_6$):2,7 ppm (s,3H); 3,3 ppm (s,3H); 3,9 ppm (s,3H); 4,1 ppm (s,3H); 6,5-8 ppm (m,5H); 11 ppm (m,1H).

EXEMPLE 38

Préparation de la (diéthylamino-3 propyl-1 amino)-10 méthyl-6 5H-pyrido[3′,4′:4,5]pyrrolo[2,3-g]isoquinoléine (formule I: Am=diéthylamino-3 propylamino: $R_1=CH_3$, $R'_1=H$, $X=N$).

On place 10,53 g (0,040 mole) de méthoxy-10 méthyl-6 5H-pyrido[3′,4′:4,5]pyrrolo[2,3-g]iso-quinoléine dans un mélange de 130 ml (0,824 mole) de diéthylaminopropylamine et de 12,2 ml d'une solution méthanolique de chlorure d'hydrogène (6,54N; 0,080 mole). On chauffe progressivement jusqu'à reflux de l'amine, période durant laquelle on distille le méthanol. On maintient alors le mélange réactionnel entre 150 et 160°C pendant 5 à 6 heures. On distille l'amine en excès sous pression réduite, on reprend le résidu dans 250 ml d'eau et on neutralise par addition de 8 ml d'hydroxyde de sodium 10N. Après une heure d'agitation, on essore la suspension et on sèche le produit sous vide partiel à 50°C.

De cette manière, on obtient 11,02 g de (diéthylamino-3 propyl-1 amino)-10 méthyl-6 5H-pyrido[3′,4′:4,5]pyrrolo[2,3-g]isoquinoléine sous forme d'une poudre verdâtre.
Rendement: 76%
P.F.: 222°C

Analyse élémentaire %: (correspondant à l'hémihydrate: 1/2 $H_2O$):

| | C | H | N |
|---|---|---|---|
| Calculé | 71,35 | 7,57 | 18,92 |
| Trouvé | 71,23 | 7,47 | 19,02 |

Spectre I.R. (KBr):

| | |
|---|---|
| NH | 3500-3000 $cm^{-1}$ (m,l) |
| C=N, C=C | 1635 $cm^{-1}$ (m) |
| aromatique | |
| | 1610 $cm^{-1}$ (F) |
| | 1575 $cm^{-1}$ (m) |

Spectre R.M.N. (méthanol $D_4$):1,0 ppm (t,6H); 1,6-2,1 ppm (m,2H); 2,2-2,8 ppm (m,9H); 3,2-3,7 ppm (m,2H); 4,9 ppm (m,2H); 6,85 ppm (d,1H); 7,25 ppm (d,1H); 7,65 ppm (d,1H); 8,25 ppm (d,1H); 8,40 ppm (s,1H); 9,0 ppm (s,1H).

EXEMPLE 39

Préparation de la (diéthylamino-3 propyl-1 amino)-10 méthyl-6 5H-pyrido[3',4':4,5]pyrrolo[2,3-g]isoquinoléine

On met en suspension 0,87 g (0,0033 mole) de méthoxy-10 méthyl-6 5H-pyrido[3',4':4,5]pyrrolo[2,3-g]isoquinoléine et 0,495 g (0,003 mole) d'iodure de sodium dans 25 ml d'acétonitrile et 4,2 ml (0,033 mole) de chlorure de triméthylsilyle puis on chauffe à 40°C pendant 17 heures. On introduit alors 10,5 ml (0,050 mole) d'hexaméthyldisilazane, 0,113 g ($6x10^{-4}$ mole) d'acide p-toluènesulfonique anhydre et 22,4 ml (0,14 mole) de diéthylaminopropylamine. Après distillation de l'acétonitrile, on chauffe au reflux pendant 20 heures. On évapore le mélange à sec sous vide partiel et on agite le résidu avec 20 ml d'eau et 5 ml d'éther diisopropylique pendant une heure. On filtre le précipité obtenu et on le sèche sous vide à 50°C.

On obtient ainsi 1,040 g d'un solide brun que l'on purifie par chromatographie sur silice.

De cette manière, on obtient 0,43 g de (diéthylamino-3 propyl-1 amino)-10 méthyl-6 5H-pyrido[3',4':4,5]pyrrolo[2,3-g]isoquinoléine.
Rendement: 36%

EXEMPLE 40

Préparation de la (diéthylamino-3 propyl-1 amino)-10 méthyl-6 5H-pyrido[3',4':4,5]pyrrolo[2,3-g]isoquinoléine.

a) Chloro-10 méthyl-6 5H-pyrido[3',4':4,5]pyrrolo[2,3-g]isoquinoléine.

On met en solution 20 g (0,076 mole) de méthoxy-10 méthyl-6 5H-pyrido[3',4':4,5]pyrrolo[2,3-g]isoquinoléine, 48,6 ml (0,304 mole) de diéthylaniline et 69,24 g (0,304 mole) de chlorure de benzyltriéthylammonium dans 200 ml d'acétonitrile sec. On ajoute alors en quelques minutes 349 ml (3,8 moles) de trichlorure de phosphoryle et on porte la suspension obtenue au reflux pendant 6 heures. On élimine l'acétonitrile et le chlorure de phosphoryle en excès par distillation sous pression réduite.

On hydrolyse progressivement le résidu huileux par addition de 930 ml d'eau et on amène alors le pH du milieu à 8 par addition d'hydroxyde de sodium 10N. On rince le précipité avec 100 ml d'eau et on essore le second précipité formé dans le filtrat. On rassemble les deux précipités et on agite le solide obtenu après l'avoir mis en suspension dans 300 ml d'acétone. Après essorage et sèchage sous pression réduite à 50°C, on obtient 17 g de chloro-10 méthyl-6 5H-pyrido[3',4':4,5]pyrrolo[2,3-g]isoquinoléine sous forme d'un solide beige marron.
Rendement: 83%
P.F.: > 263°C.
Teneur en chlore, 13,25% (théorie: 13,24%)

**Spectre I.R. (KBr):**

| | |
|---|---|
| C=N, C=C | ( 1615, 1600 $cm^{-1}$ |
| C-C aromatique | ( |

Spectre R.M.N. (acide trifluoroacétique):
3,15 ppm (S,3H ; 8,0-9,7 ppm (m,6H).

b) (Diéthylamino-3 propyl-1 amino)-10 méthyl-6 5H-pyrido[3',4':4,5]pyrrolo[2,3-g]isoquinoléine.

On met en suspension 5,9 g (0,022 mole) de chloro-10 méthyl-6 5H-pyrido[3',4':4,5]pyrrolo[2,3-g]isoquinoléine dans 149 ml (0,946 mole) de diéthylaminopropylamine et on chauffe à 150°C pendant 5 heures. On distille l'excès d'amine sous pression réduite et en obtient un solide jaune que l'on reprend dans 106 ml d'eau et 22 ml d'hydroxyde de sodium 1N.

Après une heure d'agitation, on filtre le solide en suspension, essore sur verre fritté, lave à l'éther diisopropylique et sèche sous vide partiel à 50°C.

On obtient ainsi 6,75 g de (diéthylamino-3 propyl-1 amino)-10 méthyl-6 5H-pyrido[3',4':4,5]pyrrolo[2,3-g]isoquinoléine sous forme d'un solide jaune.
Rendement: 85%

P.F.: 223°C

EXEMPLE 41

Préparation du trimaléate de (diéthylamino-3 propyl-1 amino)-10 méthyl-6 5H-pyrido[3',4':4,5]pyrrolo[2,3-g]isoquinoléine.

A) Chloro-10 méthyl-6 5H-pyrido[3',4':4,5]pyrrolo[2,3-g]isoquinoléine.

On met 1,053 g (0,004 mole) de méthoxy-10 méthyl-6 5H-pyrido[3',4':4,5]pyrrolo[2,3-g]isoquinoléine en suspension dans 5,6 ml (0,040 mole) d'oxyde de dichlorophénylphosphine et on chauffe à 160°C pendant 2 heures. On verse le milieu réactionnel sur de la glace et on amène à pH = 7 par addition d'hydroxyde de sodium 10N. Après essorage et sèchage du précipité formé on obtient 0,580 g de chloro-10 méthyl-6 5H-pyrido[3',4':4,5]pyrrolo[2,3-g]isoquinoléine.
Rendement: 54%.

B) (Diéthylamino-3 propyl-1 amino)-10 méthyl-6 5H-pyrido[3',4':4,,5]pyrrolo[2,3-g]isoquinoléine.

Ce composé a été obtenu selon la méthode décrite à l'Exemple 40b.

c) Trimaléate de (diéthylamino-3 propyl-1 amino)-10 méthyl-6 5H-pyrido[3',4':4,5]pyrrolo[2,3-g]isoquinoléine.

On dissout 5,06 g (0,014 mole) de (diéthylamino-3 propyl-1 amino)-10 méthyl-6 5H-pyrido[3',4':4,5]pyrrolo[2,3-g]isoquinoléine dans 47 ml de méthanol et on ajoute, en 5 minutes, 4,87 g (0,042 mole) d'acide maléique en solution dans 47 ml de méthanol. Après une heure d'agitation à 20°C, on essore l'épais précipité sur verre fritté et on le rince avec 5 ml de méthanol. On sèche alors sous vide partiel à 50°C.

De cette manière, on obtient 9,15 g de trimaléate de (diéthylamino-3 propyl-1 amino)-10 méthyl-6 5H-pyrido[3',4':4,5]pyrrolo[2,3-g] isoquinoléine sous forme d'une poudre beige.
Rendement: 92%
P.F.: environ 225°C (peu net)

Analyse élémentaire %:

| | C | H | N | O |
|---|---|---|---|---|
| Calculé | 57,53 | 5,55 | 9,87 | 27,05 |
| Trouvé | 57,33 | 5,57 | 9,61 | 26,92 |

Spectre I.R. (KBr):

| | |
|---|---|
| OH, $NH^+$, $NH_2^+$ | 3200-2300 $cm^{-1}$ (m,l) |
| C=O, C=C | 1625, 1585 $cm^{-1}$ (m) |
| $COO^-$ | 1470 $cm^{-1}$ (m,l) |

Spectre RMN (acide trifluoroacétique):1,5 ppm (t,6H); 2,5 ppm (m,2H); 3,0 ppm (s,3H); 3,0-4,0 ppm (m,8H); 6.65 ppm (s,6H); 7,7-9,6 ppm (m,6H).

Exemple 42

Préparation du (diéthylamino-3 propyl-1 amino)-1 diméthyl-5,11 6H-pyrido[4,3-b]carbazole (formule I:Am = diéthylamino-3 propylamino, X = $OCH_3$, $R_1 = R'_1 = CH_3$).

On place 0,245 g (8 x $10^{-4}$ mole) de diméthoxy-1,9 diméthyl-5,11 6H-pyrido[4,3-b] carbazole dans un mélange de 26 ml (0,0165 mole) de diéthylaminopropylamine et de 0,28 ml (0,0032 mole) d'acide trifluorométhane sulfonique dans le méthanol.

On chauffe progressivement jusqu'à reflux de l'amine, période durant laquelle on distille le méthanol. On maintient alors le mélange réactionnel entre 150 et 160°C pendant environ 13 heures. On distille l'amine en excès sous pression réduite, on reprend le résidu dans 5 ml d'eau et on neutralise par addition de 0,15 ml d'hydroxyde de sodium 10N. Après une heure d'agitation, on essore la suspension et on sèche le produit sous vide partiel à 50°C.

De cette manière, on obtient 0,112 g de (diéthylamino-3 propyl-1 amino)-1 diméthyl-5,11 6H-pyrido[4,3-b] carbazole après chromatographie sur silice.
Rendement: 35%
P.F.: 156°C
Spectre RMN ($DMSOD_6$):
0.7-1.7 ppm (m,12H); 2,6 ppm (m,5H); 3,2 ppm (m,2H); 3,5 ppm (s,3H); 3,9 ppm (s,3H); 4,0-4,2 ppm (m,2H); 6.5-8 ppm (m,5H).

## Revendications

1. Procédé de préparation de dérivés d'isoquinoléine de formule générale:

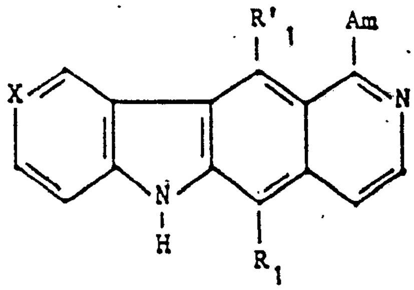

I

et de leurs sels d'addition pharmaceutiquement acceptables, dans laquelle:
-$R_1$ et $R'_1$ qui sont identiques ou différents, représentent chacun l'hydrogène ou un groupement alkyle en $C_1$-$C_4$,
- X représente un atome d'azote ou un groupement C-$OR_2$ dans lequel $R_2$ représente un groupement alkyle en $C_1$-$C_4$ ou un groupement benzyle,
- Am représente un groupement amino substitué ou non par un goupement de formule:

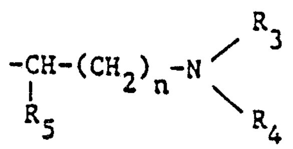

dans laquelle $R_3$ et $R_4$ identiques ou différents, représentent chacun l'hydrogène ou un radical alkyle ayant de 1 à 6 atomes de carbone, $R_5$ représente l'hydrogène ou un radical alkyle ayant de 1 à 6 atomes de carbone et n représente une valeur de 1 à 10, caractérisé en ce que:

I. dans une première étape

(a) on transforme un composé de formule

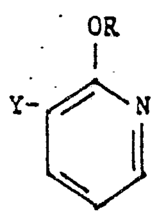

IIIa

dans laquelle:
- R représente un groupement alkyle en $C_1$-$C_4$
- Y représente un groupement diméthyl-4,4 oxazolinyl-2 ou un groupement de formule:

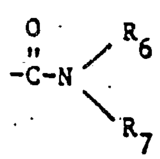

dans lequel $R_6$ et $R_7$ qui sont identiques ou différents, représentent chacun un groupement alkyle en $C_1$-$C_4$, en un dérivé métallique de formule

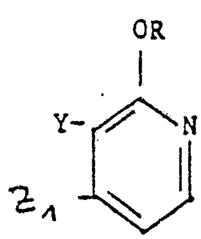

IVa

dans laquelle $Z_1$ représente un atome de lithium ou un radical de formule -Mg Hal, -Mn Hal ou $-Ce(Hal)_2$, Hal représentant un atome d'halogène, puis on condense le dérivé métallique de formule IVa avec un composé hétérocyclique de formule générale:

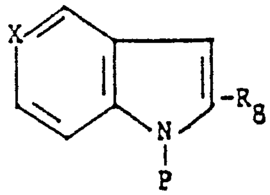

Va

dans laquelle
X a la même signification que précédemment,
P représente un groupe protecteur labile, et
$R_8$ représente:
- un radical de formule
- $\overset{O}{\overset{\|}{C}}-R_1$ dans laquelle $R_1$ a la même signification que précédemment, ou
- un radical de formule

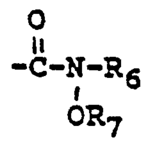

dans laquelle $R_6$ et $R_7$ qui sont identiques ou différents, ont la même signification que précédemment,
ou bien
(b) on transforme un composé de formule:

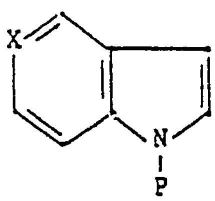

IIIb

dans laquelle X et P ont la même signification que précédemment,
en un dérivé métallique de formule

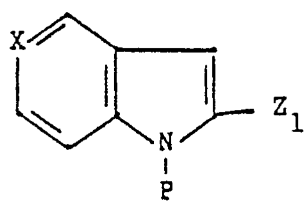

IVb

dans laquelle X, P et $Z_1$ ont la même signification que précédemment,
puis on condense le dérivé métallique de formule IVb avec un dérivé d'alkoxy-2 pyridine de formule générale:

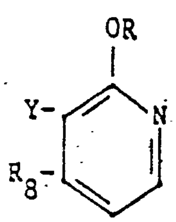

Vb

dans laquelle Y, R et $R_8$ ont la même signification que précédemment de façon à obtenir
- dans le cas où $R_8$ est un radical

$-\overset{O}{\overset{\|}{C}}-R_1$, un composé de formule:

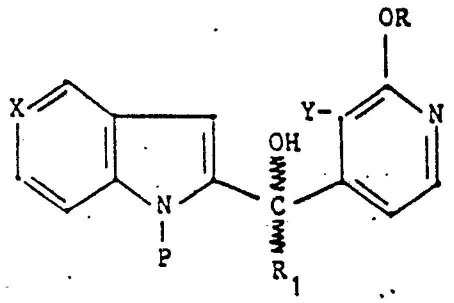

II

dans laquelle X, P, Y, R et $R_1$ ont les mêmes significations que précédemment, et
- dans le cas où $R_8$ est un radical de formule

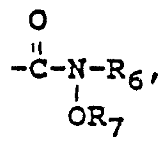

un composé de formule

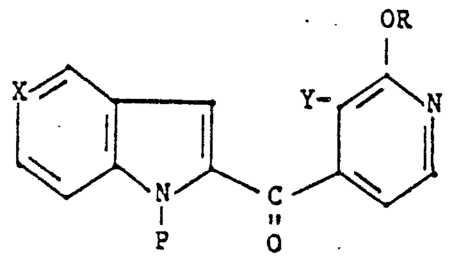

VI

dans laquelle P, R, X et Y ont la même signification que précédemment, cétone que l'on traite par la suite au moyen d'un agent réducteur choisi parmi un borohydrure de métal alcalin, l'hydrure de lithium-aluminium ou un alkyl métal de formule générale:

$R_9M$ VII

dans laquelle $R_9$ représente un groupement alkyle en $C_1$-$C_4$ et M représente un atome de lithium ou un radical -Mg Hal dans lequel Hal représente un atome d'halogène, pour obtenir un complexe que l'on hydrolyse, ce qui fournit un composé de formule II et éventuellement on oxyde un composé de formule II, dans laquelle $R_1$ représente l'hydrogène, en cétone de formule VI, que l'on traite par la suite avec un alkyle métal de formule VII et que l'on hydrolyse, pour obtenir un composé de formule II dans laquelle $R_1$ représente un radical alkyle en $C_1$-$C_4$,

le composé de formule II obtenu dans cette première étape étant par la suite éventuellement déprotégé par traitement au moyen d'un agent basique de façon à obtenir un composé N-déprotégé;

II. dans une deuxième étape

on cyclise le composé de formule II ou le composé N-déprotégé en une lactone de formule:

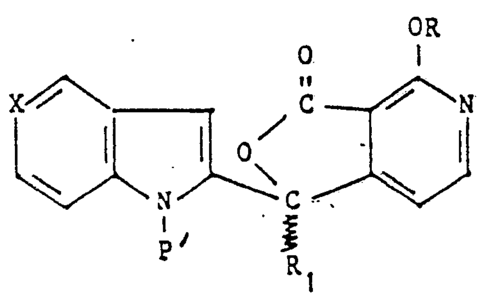

VIII

dans laquelle X, R, $R_1$ ont la même signification que précédemment et P' désigne un groupe protecteur labile P ou un atome d'hydrogène, puis on réduit la lactone ainsi obtenue soit au moyen d'un hydrure métallique pour obtenir un hemiacétal cyclique de formule

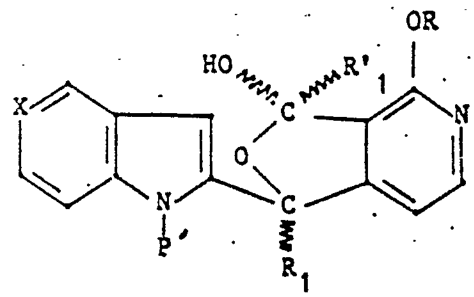

IX

dans laquelle P′, R, $R_1$, et X ont la même signification que précédemment, et $R'_1$ représente un atome d'hydrogène, soit au moyen d'un halogénure d'alkylmagnésium de formule VII, avec hydrolyse du complexe formé, pour obtenir un hémiacétal de formule IX dans laquelle $R'_1$ est un radical alkyle en $C_1$-$C_4$ et éventuellement on effectue une déprotection;

III. dans une troisième étape

on convertit un composé de formule IX en un composé de formule:

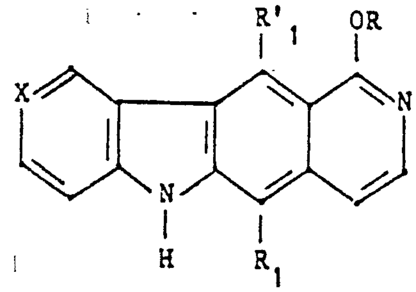

X

dans laquelle R, $R_1$, $R'_1$ et X ont la même signification que précédemment;

IV. dans une quatrième étape

on traite un composé de formule X

(a) ou bien avec un composé de formule générale:

H-Am XI

dans laquelle Am a la même signification que précédemment et en présence d'un catalyseur acide, pour obtenir un composé désiré de formule I sous forme de base libre,

(b) ou bien avec de l'oxyde de dichlorophénylphosphine pour obtenir un composé chloro de formule générale:

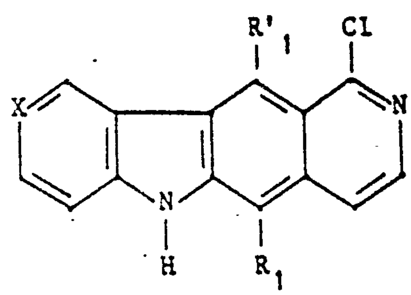

XII

dans laquelle $R_1$, $R'_1$ et X ont la même signification que précédemment, que l'on fait réagir avec un composé de formule XI pour former le composé désiré de formule I sous forme de base libre,

(c) ou bien avec le trichlorure de phosphore en présence d'un co-solvant polaire, d'un chlorure d'ammonium et d'une aniline, pour former un composé chloro de formule XII, que l'on fait réagir avec un composé de formule XI pour obtenir un composé désiré de formule I sous forme de base libre,

(d) ou bien avec un halogénure de triméthylsilyle et en présence d'un halogénure de métal alcalin, pour obtenir un dérivé triméthylsilyloxy que l'on fait réagir avec un composé de formule XI et en présence d'hexaméthyldisilazane et d'acide p-toluènesulfonique, pour former un composé désiré de formule I sous forme de base libre,

base libre que l'on peut traiter, si nécessaire, avec un acide organique ou inorganique approprié pour obtenir un sel pharmaceutiquement acceptable.

2. Procédé de préparation de dérivés d'alkoxyisoquinoléine de formule générale:

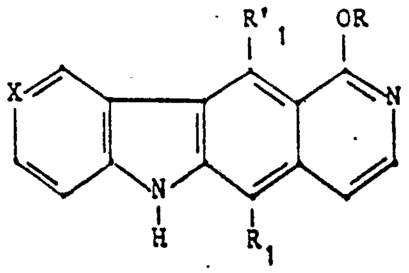

X

dans laquelle:
- $R_1$ et $R'_1$ qui sont identiques ou différents, représentent chacun l'hydrogène ou un groupement alkyle en $C_1$-$C_4$,
- R représente un groupement alkyle en $C_1$-$C_4$,
- X représente un atome d'azote ou un groupement C-$OR_2$ dans lequel $R_2$ représente un groupement alkyle en $C_1$-$C_4$ ou un groupement benzyle, caractérisé en ce que:

I. dans une première étape

(a) on transforme un composé de formule

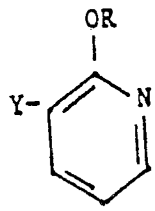

IIIa

dans laquelle:
R a la même signification que précédemment et
Y représente un groupement diméthyl-4,4 oxazolinyl-2 ou un groupement de formule:

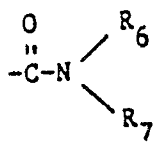

dans lequel $R_6$ et $R_7$ qui sont identiques ou différents, représentent chacun un groupement alkyle en $C_1$-$C_4$, en un dérivé métallique de formule

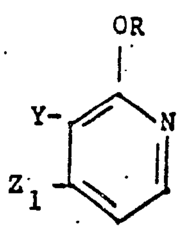

IVa

dans laquelle $Z_1$ représente un atome de lithium ou un radical de formule -Mg Hal, -Mn Hal ou -Ce$(Hal)_2$, Hal représentant un atome d'halogène, puis on condense le dérivé métallique de formule IVa avec un composé hétérocyclique de formule générale:

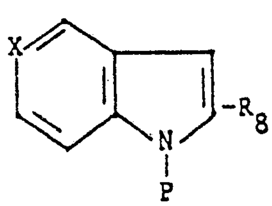

Va

dans laquelle
X a la même signification que précédemment,
P représente un groupe protecteur labile, et
$R_8$ représente:

- un radical de formule
$-\overset{\overset{O}{\|}}{C}-R_1$ dans laquelle $R_1$ a la même signification que précédemment, ou
- un radical de formule

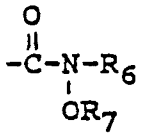

dans laquelle $R_6$ et $R_7$ qui sont identiques ou différents, ont la même signification que précédemment,
ou bien
(b) on transforme un composé de formule:

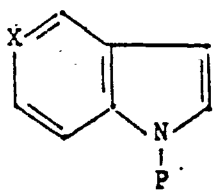

IIIb

dans laquelle X et P ont la même signification que précédemment,
en un dérivé métallique de formule

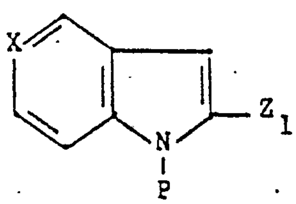

IVb

dans laquelle X, P et $Z_1$ ont la même signification que précédemment,
puis on condense le dérivé métallique de formule IVb avec un dérivé d'alkoxy-2 pyridine de formule générale:

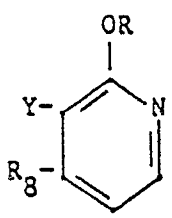

Vb

dans laquelle Y, R et $R_8$ ont la même signification que précédemment de façon à obtenir
- dans le cas où $R_8$ est un radical
$-\overset{\overset{O}{\|}}{C}-R_1$, un composé de formule:

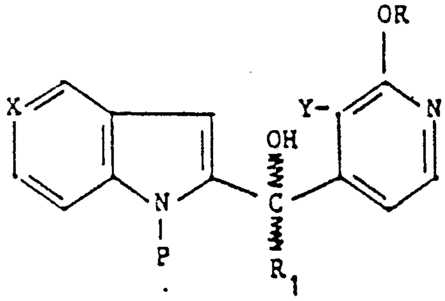

II

dans laquelle X, P, Y, R et $R_1$ ont les mêmes significations que précédemment, et
- dans le cas où $R_8$ est un radical de formule

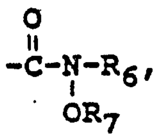

un composé de formule

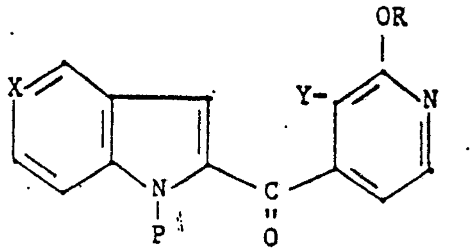

VI

dans laquelle P, R, X et Y ont la même signification que précédemment, cétone que l'on traite par la suite au moyen d'un agent réducteur choisi parmi un borohydrure de métal alcalin, l'hydrure de lithium-aluminium ou un alkyl métal de formule générale:

$R_9M$ VII

dans laquelle $R_9$ représente un groupement alkyle en $C_1$-$C_4$ et M représente un atome de lithium ou un radical -Mg Hal dans lequel Hal représente un atome d'halogène, pour obtenir un complexe que l'on hydrolyse, ce qui fournit un composé de formule II et éventuellement on oxyde un composé de formule II, dans laquelle $R_1$ représente l'hydrogène, en cétone de formule V, que l'on traite par la suite avec un alkyle métal de formule VII et que l'on hydrolyse, pour obtenir un composé de formule II dans laquelle $R_1$ représente un radical alkyle en $C_1$-$C_4$ ,

le composé de formule II obtenue dans cette première étape étant par la suite éventuellement déprotégé par traitement au moyen d'un agent basique de façon à obtenir un composé N-déprotégé.

II. dans une deuxième étape

on cyclise le composé de formule II ou le composé N-déprotégé en une lactone de formule:

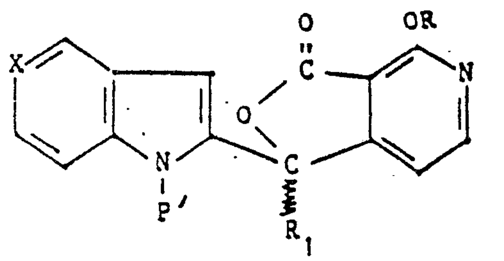

VIII

dans laquelle X, R, $R_1$ ont la même signification que précédemment et P′ désigne un groupe protecteur labile P ou un atome d'hydrogène, puis on réduit la lactone ainsi obtenue soit au moyen d'un hydrure métallique pour obtenir un hemiacétal cyclique de formule

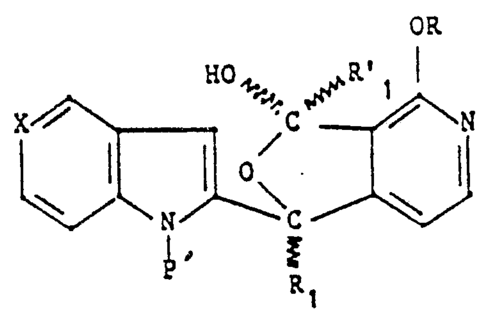

IX

dans laquelle P′, R, $R_1$, et X ont la même signification que précédemment, et $R'_1$ représente une atome d'hydrogène, soit au moyen d'un halogénure d'alkylmagnésium de formule VII, avec hydrolyse du complexe formé, pour obtenir un hemiacétal de formule IX dans laquelle $R'_1$ est un radical alkyle en $C_1$-$C_4$ et éventuellement on effectue une déprotection;

III. dans une troisième étape

on convertit un composé de formule IX en un composé de formule X.

3. Procédé selon la revendication 1 ou 2, caractérisé en ce que, dans la première étape, on transforme un composé de formule IIIa en un dérivé métallique de formule IVa dans laquelle $Z_1$ représente un atome

de lithium ou un composé de formule IIIb en un dérivé métallique de formule IVb dans laquelle $Z_1$ représente un atome de lithium, avec un agent de lithiation qui est soit un alkyl lithium ramifié, soit un amidure de lithium, à une température comprise entre -80° C et -20° C et en présence d'un agent de stabilisation, puis on condense le dérivé métallique obtenu à une température comprise entre -80° C et -20° C.

4. Procédé selon l'une quelconque des revendications 1 à 3, caractérisé en ce que, dans la première étape, on transforme un composé de formule IIIa en un dérivé métallique de formule IVa dans laquelle $Z_1$ représente un radical de formule -Mg Hal, -Mn Hal ou -Ce(Hal)$_2$ ou un composé de formule IIIb en un dérivé métallique de formule IVb dans laquelle $Z_1$ représente un radical de formule -Mg Hal, -Mn Hal ou -Ce(Hal)$_2$ par transmétallation d'un dérivé lithié de formule générale:

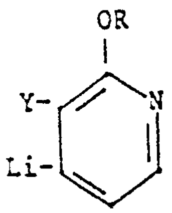

ou

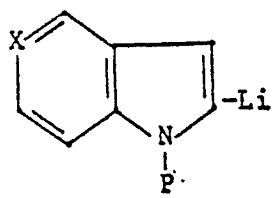

dans lequel R, X, Y et P ont la même signification que précédemment.

5. Procédé selon une des revendications 1 à 4, caractérisé en ce que l'agent de stabilisation est la tétraméthyléthylènediamine ou une tris(dioxaalkyl) amine.

6. Procédé selon l'une quelconque des revendications 3 à 5, caractérisé en ce que l'alkyl lithium ramifié est le tertiobutyllithium et l'amidure de lithium est le diéthylamidure de lithium, le diisopropylamidure de lithium ou le tétraméthyl-2,2,6,6 pipéridure de lithium.

7. Procédé selon l'une quelconque des revendications 1 à 6, caractérisé en ce que dans la deuxième étape, la cyclisation s'effectue en traitant un composé de formule II au moyen d'un acide pour obtenir une lactone de formule VIII.

8. Procédé selon la revendication 1, caractérisé en ce que dans la deuxième étape l'hydrure métallique est l'hydrure de diisobutylaluminium.

9. Procédé selon l'une des revendications 1 à 8, caractérisé en ce que, dans la deuxième étape, la déprotection s'effectue:

(a) en traitant un hémiacétal cyclique de formule IX dans laquelle P′ représente un groupement labile, au moyen d'éthérate de trifluorure de bore et dans un alcool de formule générale:

$R_{13}OH$ XXIV

dans laquelle $R_{13}$ représente un groupement alkyle en $C_1$-$C_4$, pour former un cétal de formule générale:

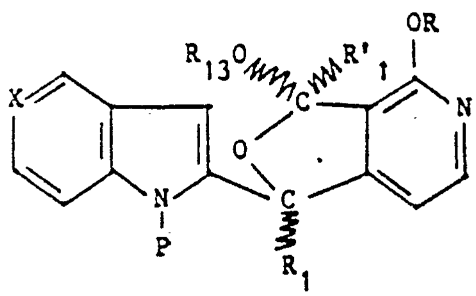

XXV

dans laquelle P, R, $R_1$, $R'_1$, $R_{13}$ et X ont la même signification que dans la revendication 1,

(b) en faisant réagir le cétal de formule XXV dans un solvant avec un agent basique pour obtenir un composé déprotégé de formule générale:

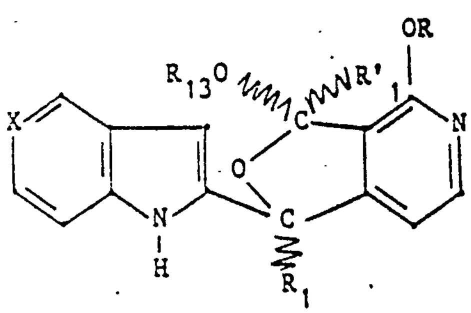

XXVI

dans laquelle R, $R_1$, $R'_1$, $R_{13}$ et X ont la même signification que précédemment,

(c) en hydrolysant le composé déprotégé de formule XXV en présence d'un agent acide pour obtenir un hémiacétal cyclique de formule IX dans laquelle P′ représente l'hydrogène.

10. Procédé selon l'une quelconque des revendications 1 à 9, caractérisé en ce que, dans la troisième étape, la conversion s'effectue en traitant l'hémiacétal de formule IX avec de 10 à 15 équivalents d'un hydroxyde de métal alcalin dans un alcool inférieur au reflux.

11. Procédé selon l'une quelconque des revendications 1 à 10, caractérisé en ce que l'on traite un équivalent d'hémiacétal de formule IX dans laquelle X représente un atome d'azote, P′ représente un groupement labile et $R'_1$ représente l'hydrogène, avec 10 à 15 équivalents d'un hydroxyde de métal alcalin, durant 15 à 20 heures dans le méthanol au reflux ou durant 1 à 2 heures dans l'éthanol au reflux, pour former les dérivés d'alkoxyisoquinoléine de formule X dans laquelle X représente un atome d'azote, R représente un radical alkyle en $C_1$-$C_4$ et $R'_1$ représente l'hydrogène.

12. Procédé selon l'une quelconque des revendications 1 à 10, caractérisé en ce qu'on traite un équivalent d'hémiacétal de formule IX dans laquelle X représente un groupement C-$OR_2$ et $R'_1$ représente un radical alkyle en $C_1$-$C_4$, avec 10 à 15 équivalents d'un hdyroxyde de métal alcalin durant 12 à 15 heures, dans le méthanol au reflux pour former les dérivés d'alkoxy-isoquinoléine de formule X dans laquelle X représente un groupement C-$OR_2$ et R et $R'_1$ représentent chacun un radical alkyle en $C_1$-$C_4$.

13. Procédé selon l'une quelconque des revendications 1 à 10, caractérisé en ce que l'on traite un équivalent d'hémiacétal de formule IX dans laquelle X représente un groupement C-$OR_2$, P′ représente un groupement labile et $R'_1$ représente l'hydrogène, avec 10 à 15 équivalents d'un hydroxyde de métal alcalin durant 1 à 2 heures dans le méthanol au reflux, pour former les dérivés d'alkoxyisoquinoléine de formule X dans laquelle X représente un groupement C-$OR_2$, $R'_1$ représente l'hydrogène et R et $R_1$ ont la signification indiquée.

14. Procédé selon l'une quelconque des revendications 1 à 9, caractérisé en ce que dans la troisième étape, la conversion s'effectue en faisant réagir un composé de formule IX dans laquelle X représente un atome d'azote, avec un hydroxyde de métal alcalin dans un alcool en $C_1$-$C_3$ à une température allant de -5° C à la température ambiante, pour former un dérivé d'isoquinoléine de formule générale:

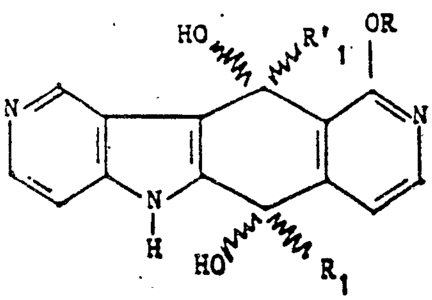

XXVII

dans laquelle R, $R_1$ et $R'_1$ ont la même signification que dans la revendication 1, dérivés d'isoquinoléine que l'on traite avec un borohydrure de métal alcalin pour former un composé de formule X dans laquelle X représente un atome d'azote.

15. Procédé selon la revendication 14 caractérisé en ce que l'on fait réagir un équivalent de dérivé phtalide de formule IX dans laquelle X représente un atome d'azote et P′ représente l'hydrogène, avec un équivalent d'hydroxyde de sodium, la réaction ayant lieu durant 3 à 4 heures.

16. Procédé selon la revendication 14, caractérisé en ce que l'on fait réagir un équivalent de dérivé phtalide de formule IX dans laquelle X représente un atome d'azote P′ représente un groupement labile et $R_1$ représente un groupement alkyle en $C_1$-$C_4$ avec 4 à 5 équivalents d'hydroxyde de sodium durant 2 à 8 heures.

17. Procédé selon l'une quelconque des revendications 1 à 9, caractérisé en ce que, dans la troisième étape, la conversion s'effectue en faisant réagir un dérivé phtalide de formule IX dans laquelle X représente un groupement C-$OR_2$ et $R'_1$ représente un radical alkyle en $C_1$-$C_4$ avec un hydroxyde de métal alcalin dans un alcool en $C_1$-$C_3$, à une température allant de 40 à 50°C, pour former un dérivé alkényle de formule générale:

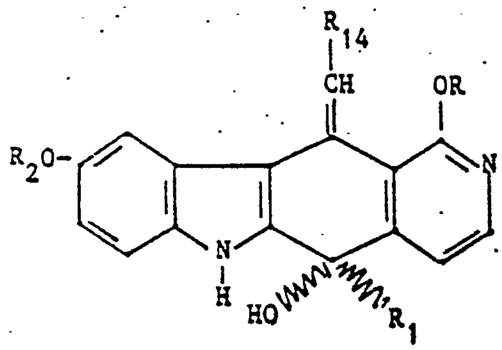

XXVIII

dans laquelle R, $R_1$ et $R_2$ ont la même signification que dans la revendication 1 et $R_{14}$ représente l'hydrogène ou un radical alkyle en $C_1$-$C_3$, dérivé alkényle que l'on traite avec un borohydrure de métal alcalin pour former un dérivé désiré de formule X dans laquelle X représente un groupement C-$OR_2$.

18. Procédé selon la revendication 17 caractérisé en ce que l'on fait réagir un équivalent de dérivé phtalide de formule IX dans laquelle X représente un groupement C-$OR_2$, $R'_1$ représente un groupement alkyle en $C_1$-$C_4$ et P' représente l'hydrogène, avec 10 à 15 équivalents d'hydroxyde de potassium durant 3 à 4 heures.

19. Procédé suivant la revendication 14 ou la revendication 18, caractérisé en ce que le traitement avec le borohydrure de métal alcalin s'effectue dans un éther de glycol comme solvant à une température comprise entre 100° C et la température de reflux.

20. Dérivés d'alkoxy-2 pyridine de formule générale:

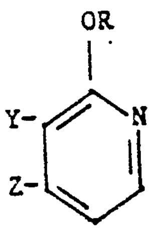

dans laquelle:

- R représente un groupement alkyle en $C_1$-$C_4$
- Y représente un groupement diméthyl-4,4 oxazolinyl-2 ou un groupement de formule:

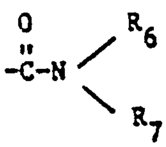

dans lequel $R_6$ et $R_7$ qui sont identiques ou différents, représentent chacun un groupement alkyle en $C_1$-$C_4$;

Z représente

- un atome de lithium ou un groupement -Mg Hal, -Mn Hal ou -Ce$(Hal)_2$ dans lequel Hal représente un atome d'halogène,
- un groupement à caractère électrophile de formule:

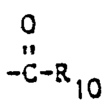

ou

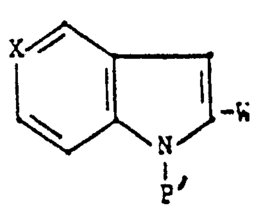

dans laquelle:

- $R_{10}$ représente l'hydrogène, un groupement alkyle en $C_1$-$C_4$, un groupement hydroxyle ou un groupement de formule:

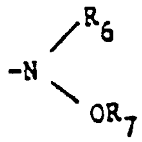

dans laquelle $R_6$ et $R_7$ ont la même signification que précédemment,

- X représente un atome d'azote ou un groupement C-$OR_2$ dans lequel $R_2$ représente un groupement alkyle en $C_1$-$C_4$ ou un groupement benzyle,
- P'représente un groupement protecteur labile, ou un atome d'hydrogène
- W représente un groupement de formule

$-\overset{O}{\overset{\|}{C}}-$ ou

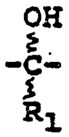

3 WI = 15 TI = CHE

dans lequel $R_1$ représente l'hydrogène ou un groupement alkyle en $C_1$-$C_4$.

21. Dérivés d'alkoxy-2 pyridine selon la revendication 20 dans laquelle Y représente un radical diéthylaminocarbonyle ou diisopropylaminocarbonyle.

22. Dérivés d'alkoxy-2 pyridine selon la revendication 20 ou 21 dans laquelle R représente un radical isopropyle ou tertiobutyle et Y représente un radical diméthyl-4,4 oxazolinyl-2.

23. Dérivés d'alkoxy-2 pyridine selon l'une quelconque des revendications 20 à 22 dans laquelle P' représente un radical benzènesulfonyle, p-toluènesulfonyle ou diméthylaminosulfonyle.

24. Dérivés selon la revendication 20 qui sont les composés suivants:

(aza-5 benzènesulfonyl- indolyl-2)-1 (diisopropylaminocarbonyl-3 méthoxy-2 pyridyl-4)-1 éthanol,
(aza-5 benzènesulfonyl-1 indolyl-2) (diisopropylaminocarbonyl-3 méthoxy-2 pyridyl-4) méthanol,
[aza-5 diméthylaminosulfonyl-1 indolyl-2][(diméthyl-4,4 oxazolinyl-2)-3 isopropoxy-2 pyridyl-4]méthanol,
(benzènesulfonyl-1 méthoxy-5 indolyl-2)(diéthylaminocarbonyl-3 méthoxy-2 pyridyl-4) méthanol,
(aza-5 benzènesulfonyl-1 indolyl-2)(diéthylaminocarbonyl-3 méthoxy-2 pyridyl-4) méthanol,
(aza-5 tertiobutyloxycarbonyl-1 indolyl-2) (diéthylaminocarbonyl-3 méthoxy-2 pyridyl-4) méthanol,
(aza-5 diméthylaminosulfonyl-1 indolyl-2)(diéthylaminocarbonyl-3 méthoxy-2 pyridyl-4) méthanol,
(aza-5 benzènesulfonyl-1 indolyl-2) (diéthylaminocarbonyl-3 méthoxy-2 pyridyl-4)cétone,
(aza-5 benzènesulfonyl-1 indolyl-2)-1 (diéthylaminocarbonyl-3 méthoxy-2 pyridyl-4)-1 éthanol,
(méthoxy-5 indolyl-2) (diéthylaminocarbonyl-3 méthoxy-2 pyridyl-4) cétone,
(aza-5 indolyl-2) (diéthylaminocarbonyl-3 méthoxy-2 pyridyl-4) cétone.

Office européen des brevets

# RAPPORT DE RECHERCHE EUROPEENNE

Numero de la demande

EP 89 40 0500

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | EP-A-0 010 029 (ANVAR)<br>* Revendication 8 * & FR-A-2 436 786<br>----- | 1 | C 07 D 471/04<br>C 07 D 471/14<br>C 07 D 401/06<br>C 07 D 413/04<br>C 07 D 413/14<br>C 07 F 1/02<br>C 07 F 3/02<br>C 07 F 13/00<br>C 07 F 5/00 //<br>(C 07 D 471/04<br>C 07 D 221:00<br>C 07 D 209:00 )<br>(C 07 D 471/14<br>C 07 D 221:00<br>C 07 D 221:00<br>C 07 D 209:00 ) |
| | | | **DOMAINES TECHNIQUES RECHERCHES (Int. Cl.4)** |
| | | | C 07 D 471/00<br>C 07 D 401/00<br>C 07 D 413/00 |

Le présent rapport a été établi pour toutes les revendications

| Lieu de la recherche | Date d'achèvement de la recherche | Examinateur |
|---|---|---|
| LA HAYE | 08-05-1989 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)